# EUROPEAN PATENT SPECIFICATION

(11) **EP 3 919 488 B1**
(45) Date of publication and mention of the grant of the patent: **27.08.2025**
(21) Application number: 20748337.1
(22) Date of filing: 21.01.2020
(51) Int. Cl.: C07D 417/14, C07D 403/14, C07D 405/14, C07D 491/048, C07D 491/107, A61K 31/427, A61P 37/00, A61P 29/00, A61P 31/00, A61P 35/00

(54) **IMMUNOMODULATOR**
IMMUNMODULATOR
IMMUNOMODULATEUR

(30) Priority: 31.01.2019 CN 201910083149; 04.09.2019 CN 201910819220
(43) Date of publication of application: 08.12.2021
(73) Proprietor: Hitgen Inc., Chengdu, Sichuan 610200 (CN)
(72) Inventor: LI, Jin, Chengdu, Sichuan 610200 (CN); ZHANG, Dengyou, Chengdu, Sichuan 610200 (CN); PAN, Fei, Chengdu, Sichuan 610200 (CN); MA, Rong, Chengdu, Sichuan 610200 (CN); ZHU, Wenji, Chengdu, Sichuan 610200 (CN); XIN, Yanfei, Chengdu, Sichuan 610200 (CN); LI, Si, Chengdu, Sichuan 610200 (CN); LIU, Weiping, Chengdu, Sichuan 610200 (CN); LIN, Yanping, Chengdu, Sichuan 610200 (CN)
(74) Representative: Wilson, Justin Scott
(86) International application number: PCT/CN2020/073405
(87) International publication number: WO 2020/156363

(56) References cited:
- WO-A1-2017/175147
- WO-A1-2019/069269
- WO-A1-2019/069270
- WO-A1-2019/134705
- WO-A1-2021/026009
- CN-A- 109 071 514
- JOSHI, M. R. ET AL.: "Design of Amidobenzimidazole STING Receptor Agonists with Systemic Activity", NATURE, vol. 564, no. 7736, 27 November 2018 (2018-11-27), pages 439 - 443, XP036660354, DOI: 20200409215137X

## Description

### FIELD

The invention relates to an immunomodulator, in particular to a class of compounds that activate STING and its use as an immunomodulator in the manufacture of a medicament.

### BACKGROUND

The human body's immune system can generally be divided into "natural immunity" system and "adaptive immunity" system. The natural immune system plays an important role in resisting infections, inhibiting tumor growth and the pathogenesis of autoimmune diseases. It mainly recognizes pathogenic microorganisms and cancer cell components through pattern recognition receptors, initiates downstream signal pathways, and finally induces cytokine expression, kill pathogenic microorganisms and cancer cell components, and adapt to the immune system to promote the production of antibodies and specific T lymphocytes.

STING (interferon gene stimulating factor, TMEM173, MITA, etc.) is a key node molecule for intracellular response to DNA invasion. Under the stimulation of cytoplasmic DNA, it recognizes the signal of cytoplasmic DNA receptor and plays a key role in the process of inducing interferon production. After the host cell's DNA recognition receptor recognizes exogenous or endogenous "non-self" DNA, it transmits the signal to the node molecule STING, and then STING rapidly dimerizes and transfers from the endoplasmic reticulum to the perinuclear body. The activation of STING leads to the up-regulation of IRF3 and NKκB pathways, which leads to the induction of interferon-β and other cytokines.

Compounds that induce human interferon can be used to treat various diseases (including allergic diseases and other inflammatory diseases, allergic rhinitis and asthma, infectious diseases, neurodegenerative diseases, precancerous syndrome and cancer), and can also be used as immunization composition or vaccine adjuvant. Therefore, the development of new compounds that are capable of activating STING is an effective method for the treatment of type 1 IFN pathway diseases (including inflammatory diseases, allergic and autoimmune diseases, infectious diseases, cancer, and precancerous syndromes).

WO2017175147 discloses heterocyclic amides that are useful as modulators of STING.

### SUMMARY

In order to solve the above-mentioned problems, the present disclosure provides an immunomodulator.

The present invention provides a compound, or the stereoisomer, or the pharmaceutically acceptable salt thereof, wherein the compound is represented by formula II as defined by claim 1

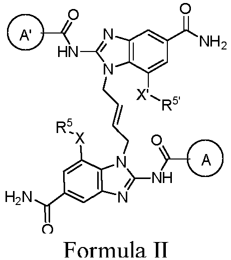

The present invention further provides, a compound, or the stereoisomer, or the pharmaceutically acceptable salt thereof, wherein the compound is:

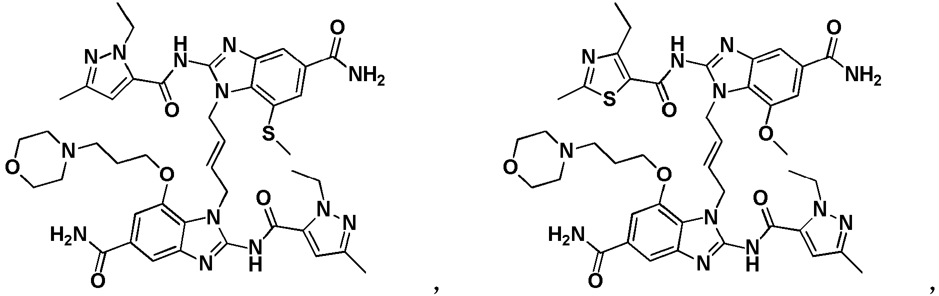

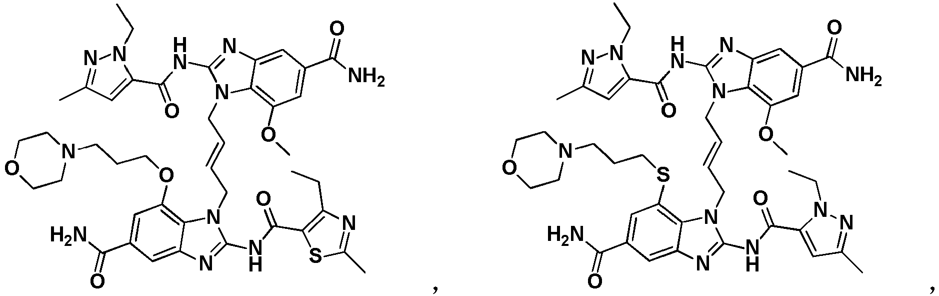

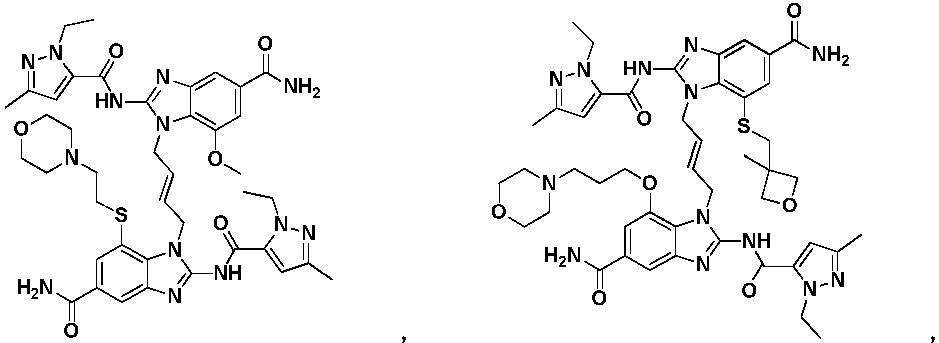

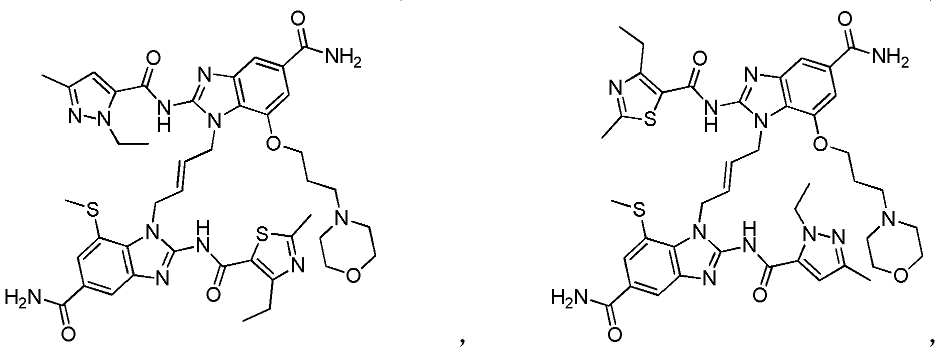

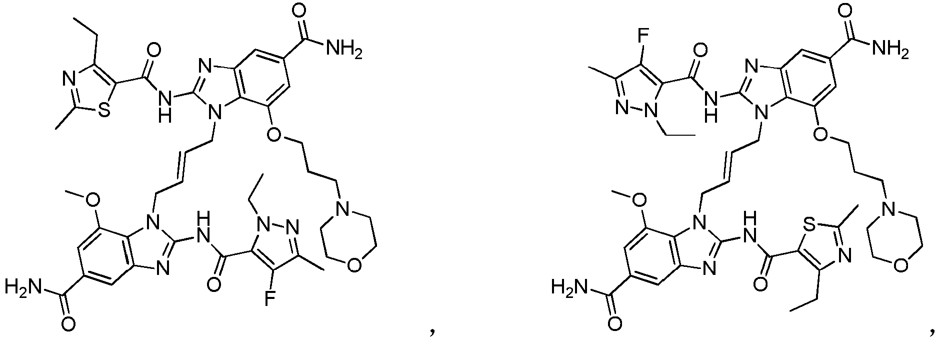

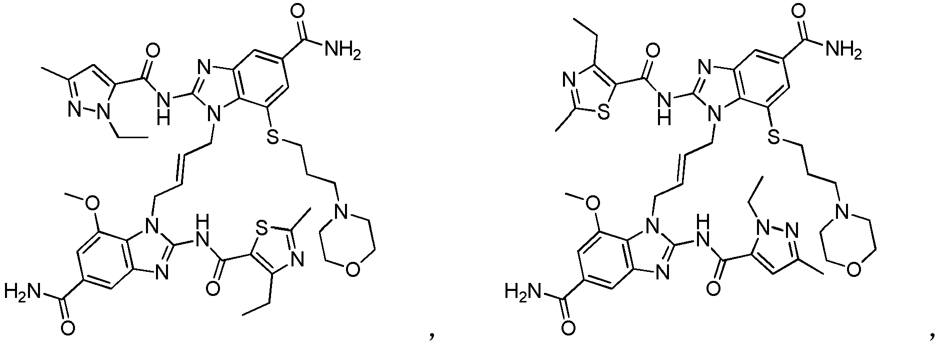

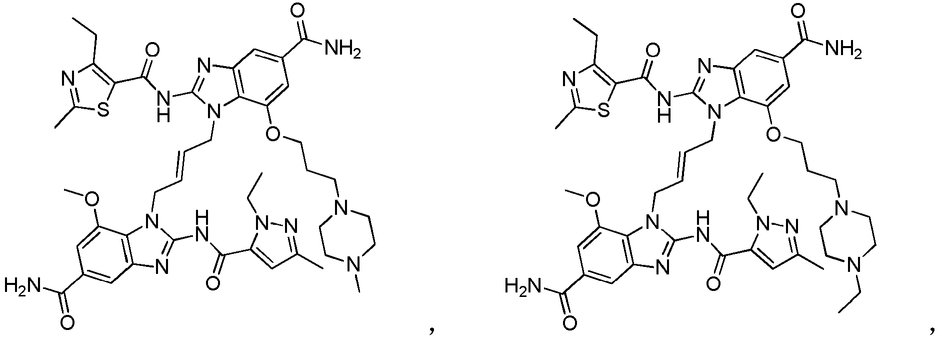

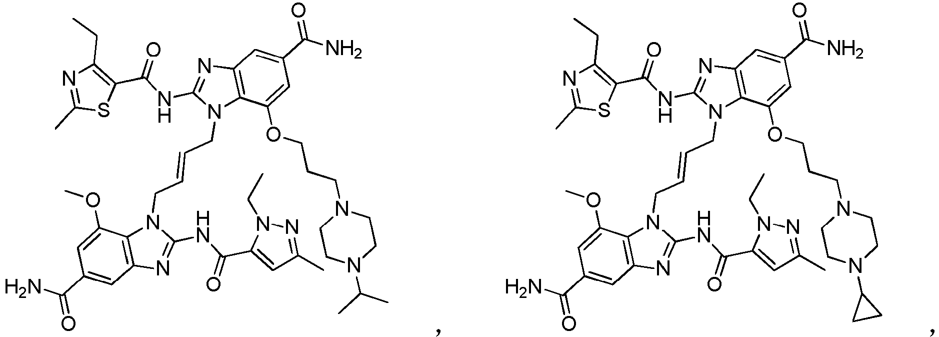

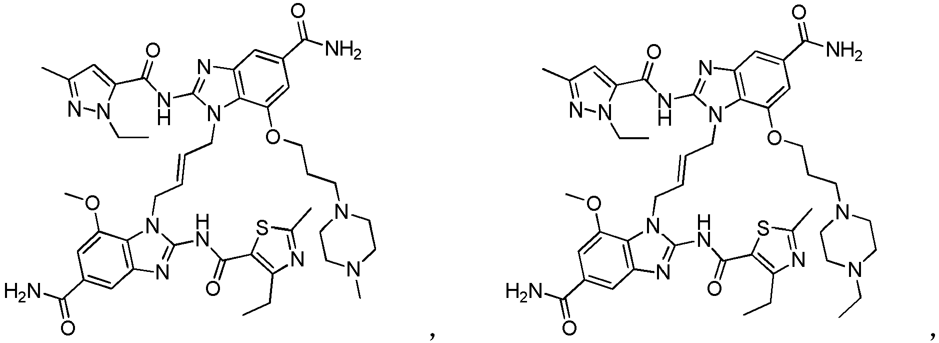

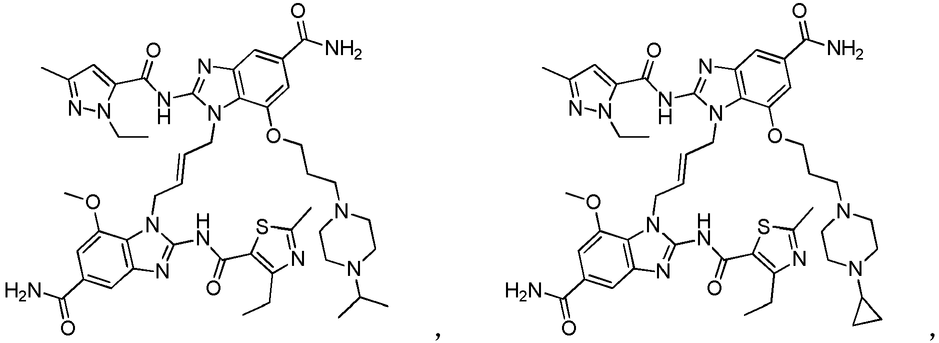

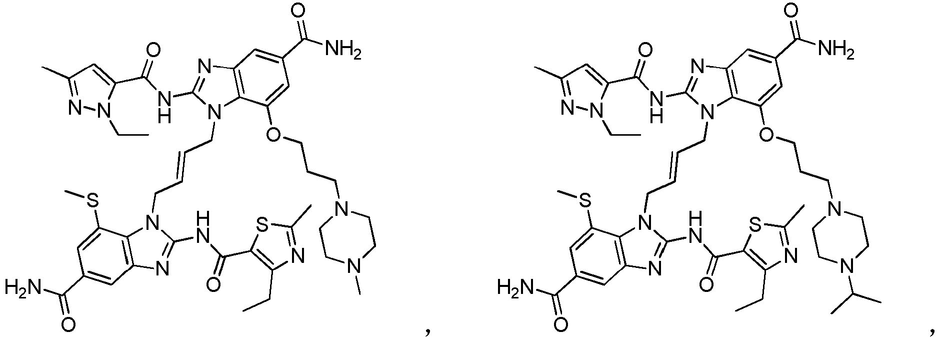

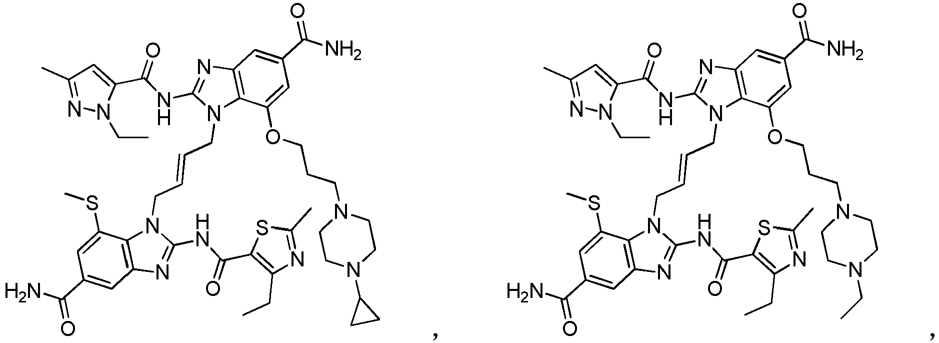

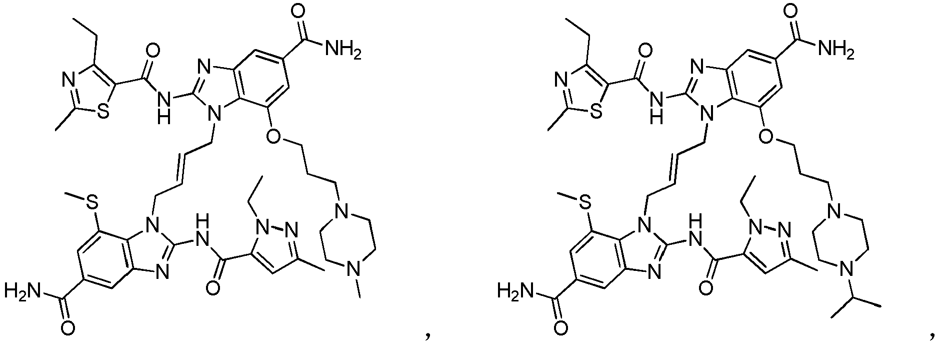

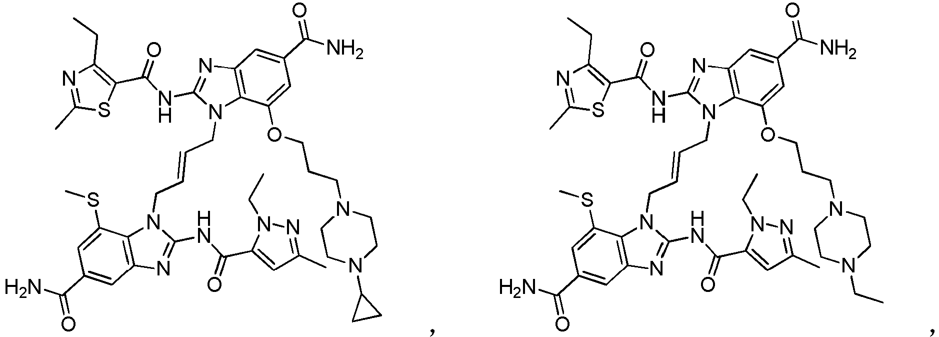

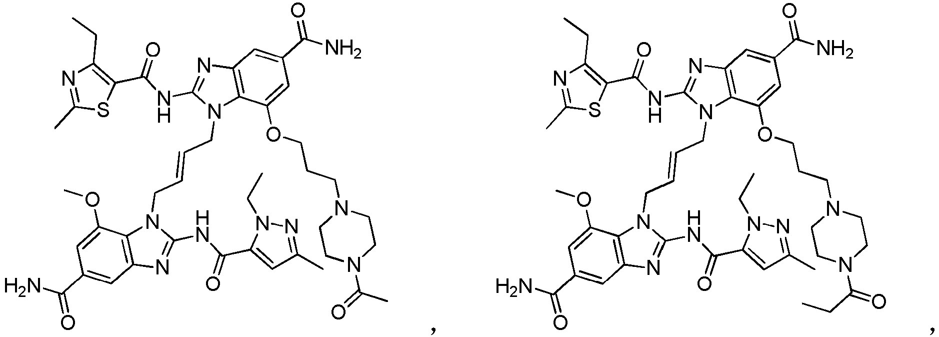

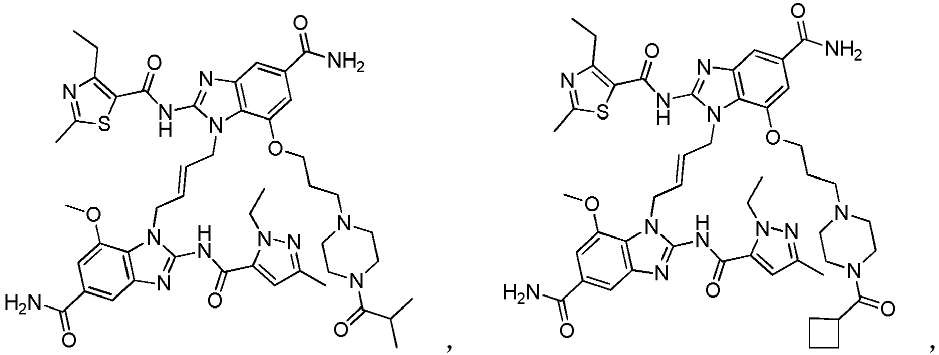

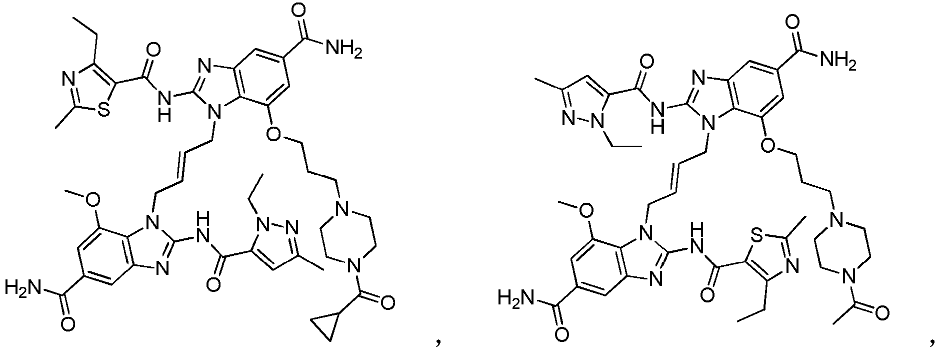

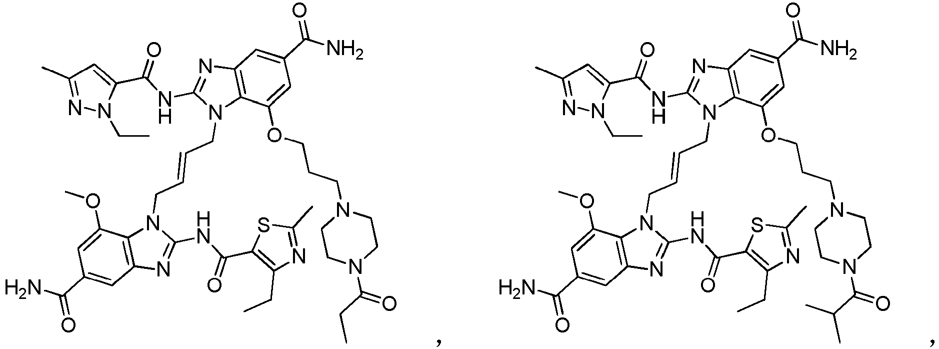

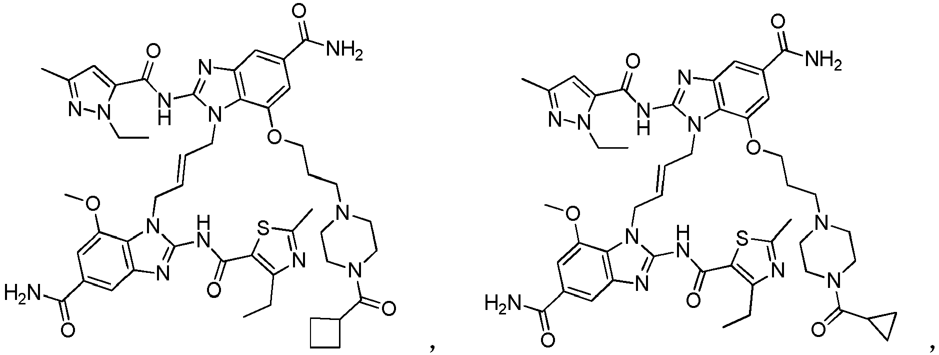

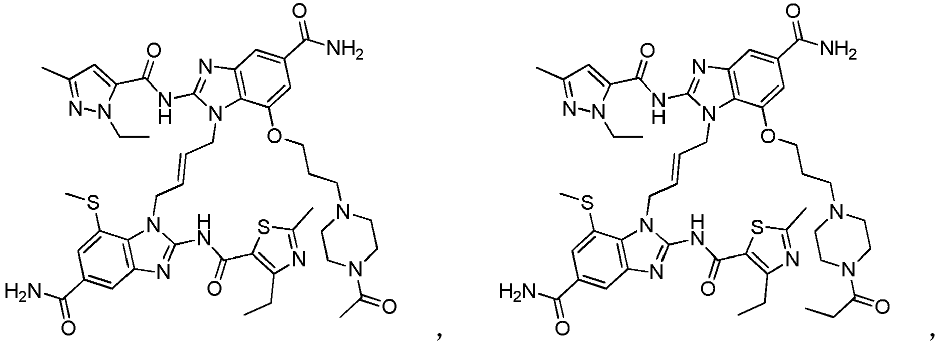

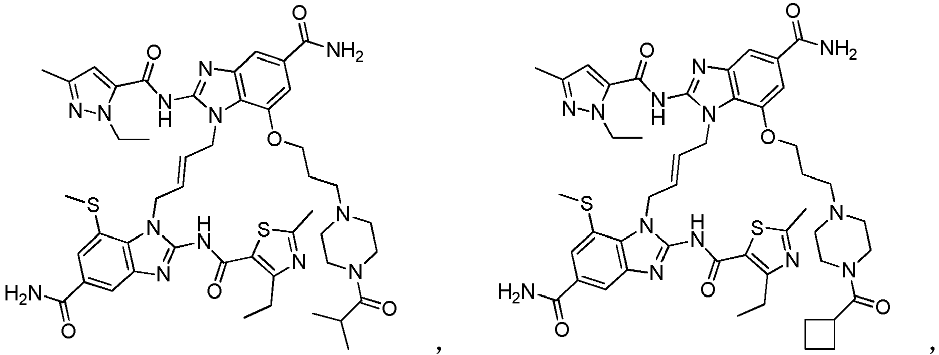

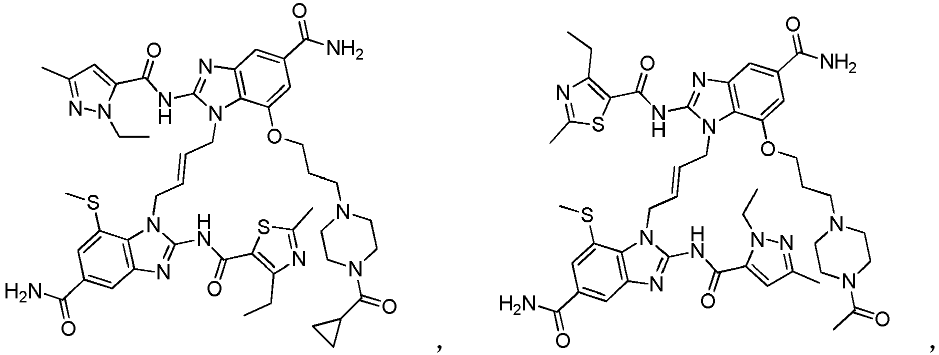

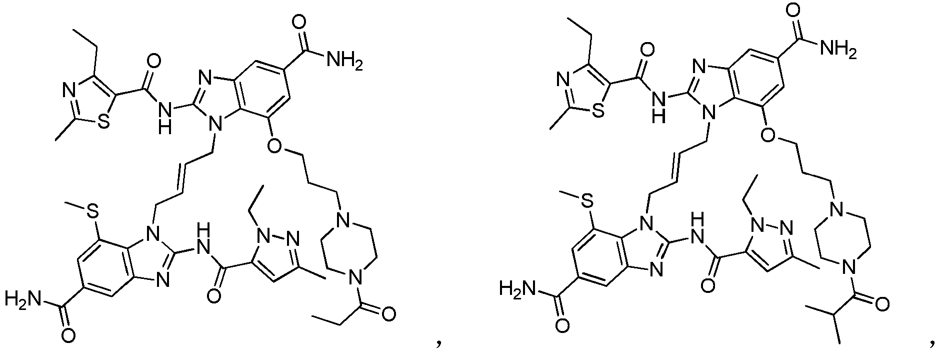

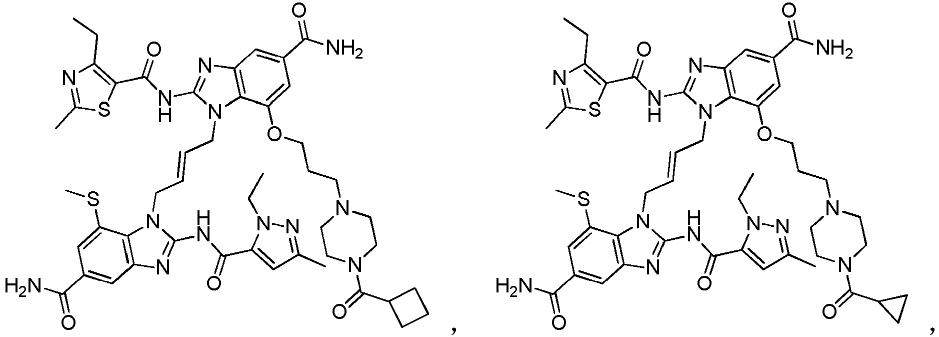

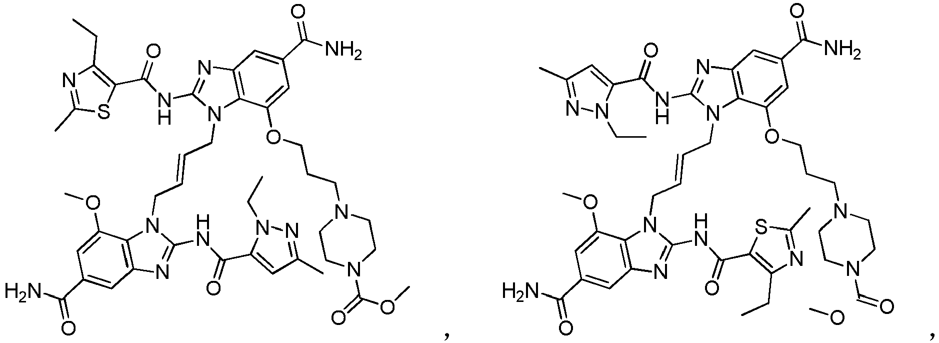

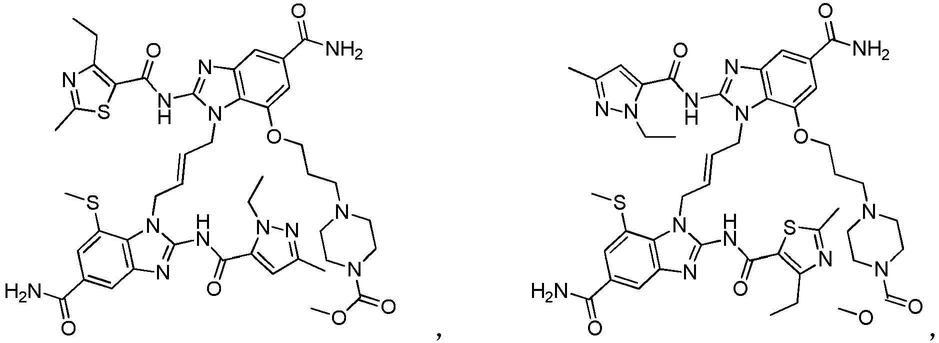

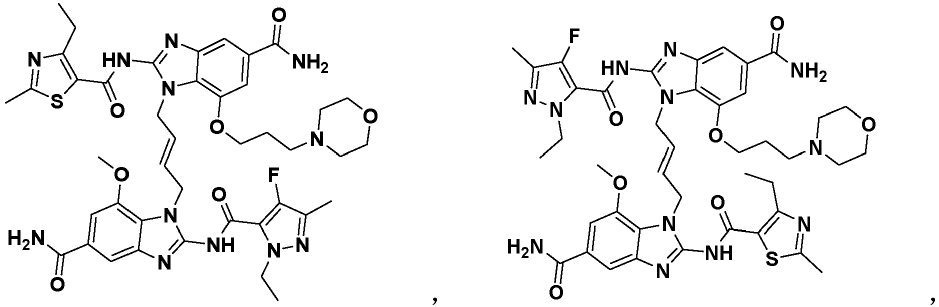

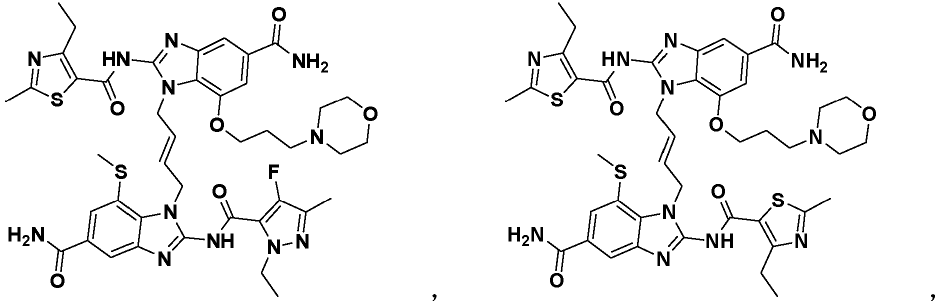

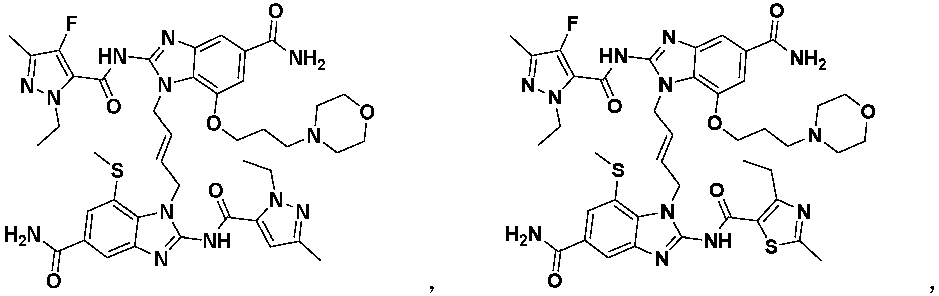

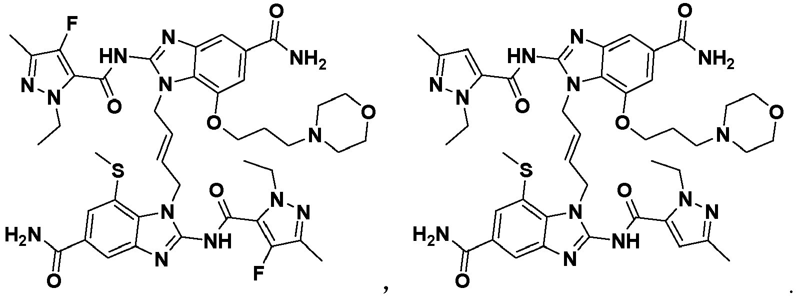

The present invention further provides a compound, or the stereoisomer, or the pharmaceutically acceptable salt thereof, wherein the compound is represented by formula III as defined in claim 3.

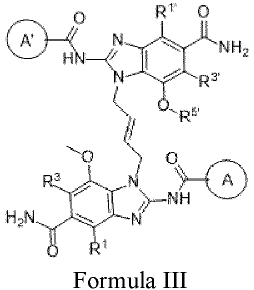

The present invention further provides the compound, or the stereoisomer, or the pharmaceutically acceptable salt thereof, wherein the compound is:

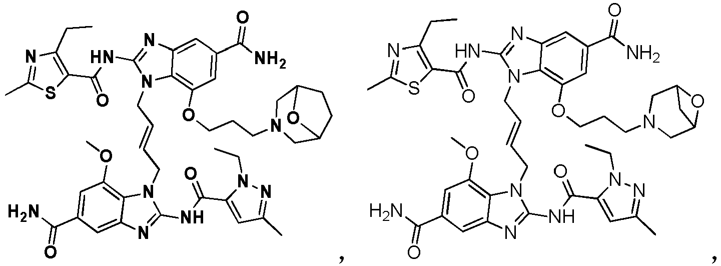

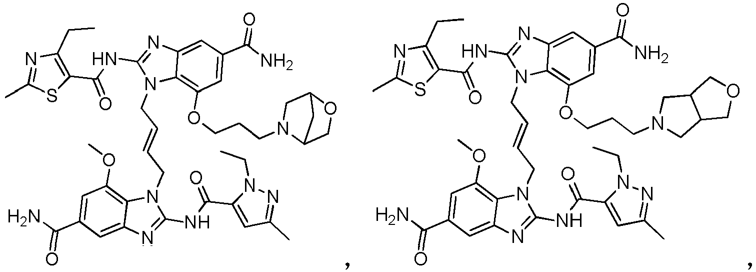

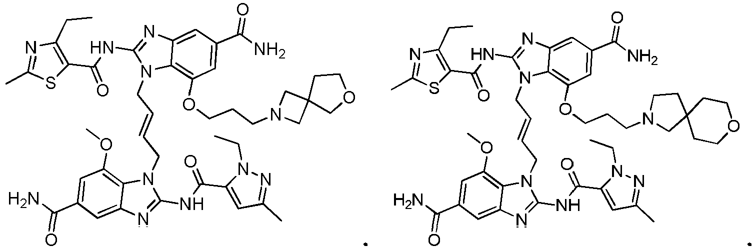

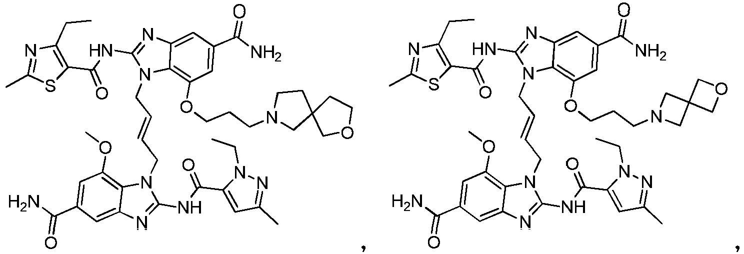

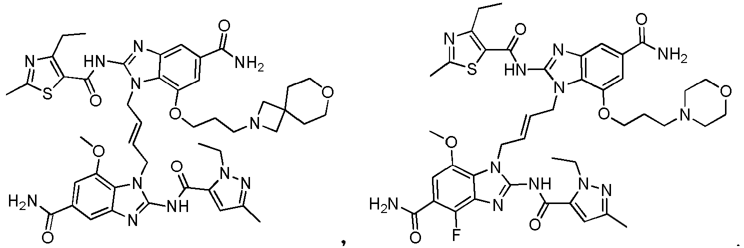

The present invention also provides the above-mentioned compound, or a stereoisomer, or a pharmaceutically acceptable salt thereof for use in treating diseases related to STING activity that are one or more of diseases related to inflammatory diseases, autoimmune diseases, infectious diseases, cancer, and precancerous syndrome

The present invention also provides the above-mentioned compound, or a stereoisomer, or a pharmaceutically acceptable salt thereof for use as an immune adjuvant.

The present invention also provides a medicament, which is a preparation prepared from the above-mentioned compound, or a stereoisomer, or a pharmaceutically acceptable salt thereof as the active ingredient, plus pharmaceutically acceptable auxiliary materials.

The diseases related to STING activity defined in the present invention are diseases in which STING plays an important role in the pathogenesis of the disease.

Diseases related to STING activity include inflammatory diseases, allergic diseases and autoimmune diseases, infectious diseases, cancer, and precancerous syndrome.

"Cancer" or "malignant tumor" refers to any of a variety of diseases characterized by uncontrolled abnormal cell proliferation, and the ability of affected cells to spread to other locations locally or through the bloodstream and lymphatic system of the body (i.e., metastasis) and any of many characteristic structural and/or molecular characteristics. "Cancer cells" refer to cells that undergo multiple stages of tumor progression in the early, middle, or late stages. Cancers include sarcoma, breast cancer, lung cancer, brain cancer, bone cancer, liver cancer, kidney cancer, colon cancer, and prostate cancer. In some embodiments, the compound of formula I is used to treat a cancer selected from colon cancer, brain cancer, breast cancer, fibrosarcoma, and squamous cell carcinoma. In some embodiments, the cancer is selected from melanoma, breast cancer, colon cancer, lung cancer, and ovarian cancer. In some embodiments, the cancer being treated is a metastatic cancer.

Inflammatory diseases include a variety of conditions characterized by histopathological inflammation. Examples of inflammatory diseases include acne vulgaris, asthma, enterocoelia diseases, chronic prostatitis, glomerulonephritis, inflammatory bowel disease, pelvic inflammation, reperfusion injury, rheumatoid arthritis, sarcoidosis, vasculitis, airway inflammation and interstitial cystitis caused by house dust mites. There is a significant overlap between inflammatory diseases and autoimmune diseases. Some embodiments of the present invention relate to the treatment of asthma, an inflammatory disease. The immune system is usually involved in inflammatory diseases, which are manifested in allergic reactions and some myopathy. Many immune system diseases lead to abnormal inflammation.

The compounds and derivatives provided in the present invention can be named according to the IUPAC (International Union of Pure and Applied Chemistry) or CAS (Chemical Abstracts Service, Columbus, OH) naming system.

Regarding the definition of terms used in the present invention: Unless otherwise specified, the initial definitions of radical groups or terms provided herein are applicable to the radical groups or terms throughout the specification; for terms not specifically defined herein, it should be, based on the disclosure and context, given the meaning that those skilled in the art can give them.

"Substitution" refers to the replacement of a hydrogen atom in a molecule by a different atom or molecule.

The minimum and maximum content of carbon atoms in a hydrocarbon group is indicated by a prefix. For example, the prefix (Cₐ-C_{b}) alkyl indicates any alkyl group containing "a" to "b" carbon atoms. Thus, for example, (C₁-C₆) alkyl refers to an alkyl group containing 1 to 6 carbon atoms.

In the present invention, "alkyl" refers to a saturated hydrocarbon chain having a specified number of member atoms. For example, C₁-C₆ alkyl refers to an alkyl group having 1 to 6 carbon atoms. Alkyl groups can be linear or branched. Representative branched alkyl groups have one, two, or three branches. The alkyl group may be optionally substituted with one or more substituents as defined herein. Alkyl groups include methyl, ethyl, propyl (n-propyl and isopropyl), butyl (n-butyl, isobutyl and tert-butyl), pentyl (n-pentyl, isopentyl and neopentyl) and hexyl. The alkyl group may also be a part of another group, such as a C₁-C₆ alkoxy group.

In the present invention, the Cₐ-C_{b} alkoxy group refers to a group obtained by connecting an alkyl group containing "a" to "b" carbon atoms to the corresponding oxygen atom.

In the present invention, "C₂-C₆ alkylene" refers to a divalent saturated aliphatic hydrocarbon group having 2 to 6 carbon atoms. Alkylene groups include branched and straight chain hydrocarbyl groups. For example, "(C₂-C₆)alkylene" is meant to include ethylene, propylene, 2-methylpropylene, dimethylethylene, pentylene and the like.

Similarly, "C₂-C₆ alkenylene" refers to a divalent aliphatic hydrocarbon group having 2 to 6 carbon atoms and containing one or more carbon-carbon double bonds. Alkenylene groups include branched and straight chain groups. The carbon-carbon double bond in the alkenylene group includes a *cis* double bond and a *trans* double bond.

Similarly, "C₂-C₆ alkynylene group" refers to a divalent aliphatic hydrocarbon group having 2 to 6 carbon atoms and containing one or more carbon-carbon triple bonds. Alkynylene groups include branched and straight chain groups.

In the present invention, the -C(O)- in -C(O)NR^{a}R^{b}, -NR^{a}C(O)R^{b}, -C(O)R^{a}, -C(O)OR^{a} and -C(O)R^{f} means a carbonyl group 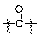 composed of carbon and oxygen in which the atoms are connected by a double bond.

In the present invention, "halogen" refers to a halogen group: fluorine, chlorine, bromine or iodine.

The immune adjuvant in the present invention is an immunomodulator, which refers to a substance that has the function of enhancing and regulating immunity in terms of anti-infection, anti-virus, anti-tumor, anti-allergic reaction, anti-asthma, etc. It is mainly used clinically for adjuvant treatment of inflammation, autoimmune diseases, infectious diseases, cancer, and precancerous syndromes. The term "pharmaceutically acceptable" refers to a certain carrier, vehicle, diluent, excipient, and/or the salt formed is usually chemically or physically compatible with other ingredients constituting a certain pharmaceutical dosage form, and physiologically compatible with the receptor.

The terms "salts" and "pharmaceutically acceptable salts" refer to the above-mentioned compounds or their stereoisomers, or acid salts and/or basic salts formed with inorganic and/or organic acids and bases, and also include zwitterionic salts (inner salt), also include quaternary ammonium salts, such as alkyl ammonium salts. These salts can be directly obtained in the final isolation and purification of the compound. They can also be obtained by appropriately mixing the above-mentioned compound, or its stereoisomer, with a certain amount of acid or base (for example, equivalent). These salts may form a precipitate in the solution and be collected by filtration, or recovered after evaporation of the solvent, or prepared by freeze-drying after reacting in an aqueous medium. The salt in the present invention can be the hydrochloride, sulfate, citrate, benzenesulfonate, hydrobromide, hydrofluoride, phosphate, acetate, propionate, succinate, oxalate, malate, succinate, fumarate, maleate, tartrate or trifluoroacetate of the compounds.

In certain embodiments, one or more compounds of the present invention may be used in combination with each other. Optionally, the compound of the present invention can be used in combination with any other active agent to prepare drugs or pharmaceutical compositions for regulating cell function or treating diseases. If a group of compounds are used, these compounds can be administered to the subject simultaneously, separately or sequentially.

The compound provided by the present invention can effectively combine with STING, has a good STING protein agonistic function, shows a good inhibitory effect on a variety of tumors, and can also activate the immune memory mechanism of mice and inhibit tumor re-stimulation. Therefore, the compounds of the present invention can be used as STING agonists and used to treat various related diseases. The compounds of the present invention have very good application prospects in the manufacture of a medicament for the treatment of diseases related to STING activity (especially medicaments for the treatment of inflammatory diseases, allergic diseases, autoimmune diseases, infectious diseases, cancer or precancerous syndromes) and in the manufacture of immune adjuvants, thus provide a new choice for clinical screening and/or manufacture of a medicament for treating diseases related to STING activity.

Apparently, according to the above-mentioned content of the present invention, according to common technical knowledge and conventional means in the field, various other modifications, replacements or changes can be made without departing from the above-mentioned basic technical idea of the present invention.

Hereinafter, the above-mentioned content of the present invention will be further described in detail through specific implementations in the form of examples.

### BRIEF DESCRIPTION OF DRAWINGS

Figure 1 is a graph showing the inhibitory effect of compounds prepared in the examples of the present invention on CT26 tumor model.
Figure 2 is a graph showing the inhibitory effect of the compound prepared in the examples of the present invention on the CT26 tumor re-excitation model, wherein the curves of Example 2 (4.5 mg/kg), Example 2 (1.5 mg/kg), and Example 5 (1.5 mg/kg), Example 15 (3mg/kg), Example 15 (1mg/kg), Example 17 (1.5mg/kg) and Example 29 (1mg/kg) overlap.

### DETAILED DESCRIPTION

The raw materials and equipments used in the specific embodiments of the present invention are all known products, which can be obtained by purchasing commercially available products.

The structures of the compounds were determined by nuclear magnetic resonance (NMR) or (and) mass spectrometry (MS). The NMR shift (δ) is given in unit of 10⁻⁶ (ppm). NMR was measured with (Bruker Avance III 400 and Bruker Avance 300) nuclear magnetic instrument, the solvent was deuterated dimethyl sulfoxide (DMSO-d₆), deuterated chloroform (CDCl₃), deuterated methanol (CD₃OD), and the internal standard was tetramethylsilane (TMS).

The LC-MS was measured with Shimadzu LC-MS (Shimadzu LC-MS 2020 (ESI)).

The Shimadzu high pressure liquid chromatograph (Shimadzu LC-20A) was used for the HPLC measurement.

Gilson GX-281 reversed-phase preparative chromatograph was used for reversed-phase preparative chromatography.

The thin layer chromatography silica gel plate used was Yantai Huanghai HSGF254 or Qingdao GF254 silica gel plate, and the specifications for thin layer chromatography separation and purification products were 0.4 mm-0.5 mm.

Column chromatography generally used Yantai Huanghai silica gel of 200-300 mesh as the carrier.

The known starting materials of the present invention can be synthesized by using or according to methods known in the art, or can be purchased from Anaiji Chemical, Chengdu Kelon Chemical, Shaoyuan Chemical Technology, J & K Scientific and other companies.

The hydrogen atmosphere means that the reaction flask is connected to a hydrogen balloon with a volume of about 1L.

The hydrogenation reaction is usually performed by vacuumizing, and filling with hydrogen, and the operations are repeated 3 times.

Unless there are special instructions in the examples, the reaction is carried out under a nitrogen atmosphere.

Unless there are special instructions in the examples, the solution refers to an aqueous solution.

Unless there are special instructions in the examples, the reaction temperature is room temperature.

Unless there are special instructions in the examples, M is mole per liter.

Room temperature is the most suitable reaction temperature, which is 20°C-30°C.

Overnight is 12±1h.

PE refers to petroleum ether; EA refers to ethyl acetate; DCM refers to dichloromethane; MeOH refers to methanol; DMF refers to N,N-dimethylformamide; DMSO refers to dimethyl sulfoxide; DMAP refers to 4-dimethylaminopyridine; DIPEA refers to diisopropylethylamine; Boc refers to tert-butyloxycarbonyl; TFA refers to trifluoroacetic acid; DBU refers to 1,8-diazabicycloundec-7-ene; HATU refers to 2-(7-azobenzotriazole)-N,N,N',N'-tetramethylurea hexafluorophosphate.

The following is the preparation of intermediate compounds:
1. Intermediate compound: Synthesis of 4-chloro-3-methoxy-5-nitrobenzamide

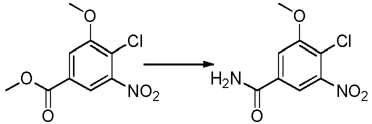

4-Chloro-3-methoxy-5-nitrobenzyl ester (18.5 g, 75.3 mmol) was added to a single-neck flask containing ammonium hydroxide (200 mL) and stirred at 60°C for 3 h. The reaction solution was concentrated to 100 mL, cooled and filtered, and the solid was washed with ice water. After drying, 4-chloro-3-methoxy-5-nitrobenzamide (12.5 g, 54.1 mmol) was obtained as a brown solid.
MS (ESI) m/z =231[M+H]⁺,
¹HNMR(400MHz, DMSO-*d₆*): δ 8.29(s, 1H), 8.04(d, 1H), 7.87(d, 1H), 7.78(s, 1H), 4.01(s, 3H)_{∘}

2. Intermediate compound: Synthesis of 4-chloro-3-hydroxy-5-nitrobenzamide

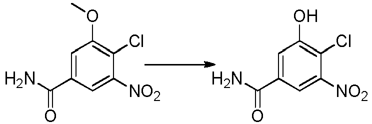

In an ice bath, 4-chloro-3-methoxy-5-nitrobenzamide (7.5 g, 32.5 mmol) was dispersed in dry DCM (90 mL), to which boron tribromide (120 mL , 1 M) was then slowly added dropwise. After the addition, the ice bath was removed, and the reaction was performed overnight at room temperature under protection of nitrogen. After the reaction was completed, the reaction solution was poured into ice water, stirred vigorously for 30 minutes, filtered, and the filter cake was washed with water. After the filter cake was dried, 4-chloro-3-hydroxy-5-nitrobenzamide (6.00 g, 27.7 mmol, 85.3% yield) was obtained as pale yellow solid.
MS (ESI) m/z =217[M+H]+
1H NMR (400MHz, DMSO-d₆): δ 11.73 (s, 1H), 8.21 (s, 1H), 7.92 (s, 1H), 7.80 (s, 1H), 7.66 (s, 1H).

3. Intermediate compound: Synthesis of 1-ethyl-3-methyl-1H-pyrazole-5-carbonyl isothiocyanate

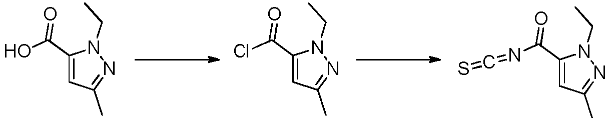

1-Ethyl-3-methyl-1H-pyrazole-5-carboxylic acid (4.00 g, 25.9 mmol) was dispersed in dry DCM (80 mL), to which was added oxalyl chloride (3.9 g, 31.1 mmol) and a catalytic amount of DMF. After reacting for 1 hour at room temperature, the volatiles were removed by rotary evaporation under reduced pressure. DCM (20 mL) was added to the crude product, and the solvent was removed by rotary evaporation to obtain 1-ethyl-3-methyl-1H-pyrazole-5-carbonyl chloride (4.46 g, 100% yield), which was used directly for the next reaction.

At 0°C, 1-ethyl-3-methyl-1H-pyrazole-5-carbonyl chloride (4.46 g, 25.9 mmol) was dissolved in dry acetone (20 mL) and added dropwise to a solution of potassium thiocyanate (5.0 g, 51.5 mmol) in acetone (100 ml) and stirred at room temperature for 3 h. The reaction system was filtered to remove inorganic salts. The crude product after concentration of the filtrate was purified by silica gel column (eluent: ethyl acetate/petroleum ether (v/v)=1/15), and 1-ethyl-3-methyl-1H-pyrazole-5-carbonyl isothiocyanate (4.0 g, 20.4 mmol, 78.7%) was obtained as a clear brown-yellow liquid.

MS (ESI) m/z =196[M+H]⁺.

4. Intermediate compound: Synthesis of 4-ethyl-2-methylthiazole-5-carbonyl isothiocyanate:

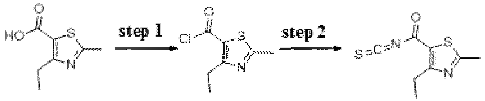

### Step 1: Synthesis of 4-ethyl-2-methylthiazole-5-carboxylic acid chloride

4-Ethyl-2-methylthiazole-5-carboxylic acid (2 g, 11.7 mmol) was dispersed in dry DCM (40 ml), to which oxalyl chloride (1.9 g, 15.1 mmol) and a catalytic amount of DMF were added dropwise under an ice bath. After reacting for 1 hour at room temperature, the volatiles were removed by rotary evaporation under reduced pressure. DCM (20ml) was added to the crude product, and the solvent was removed by rotary evaporation to obtain 4-ethyl-2-methylthiazole-5-carboxylic acid chloride (2.2 g, 100% yield), which was directly used in the next reaction.

### Step 2: Synthesis of 4-ethyl-2-methylthiazole-5-carbonyl isothiocyanate

At 0°C, 4-ethyl-2-methylthiazole-5-carboxylic acid chloride (2.2 g, 11.7 mmol) was dissolved in dry acetone (10 ml) and added dropwise to a solution of potassium thiocyanate (2.3 g, 23.4 mmol) in acetone (50 ml), stirred at room temperature for 3 hours. The reaction system was filtered to remove inorganic salts, the filtrate was concentrated and the crude product was purified by silica gel column (eluent: ethyl acetate/petroleum ether, v/v=1/15), and 4-ethyl-2-methylthiazole-5-carbonyl isothiocyanate (2.15 g, 10.2 mmol, yield 87%) was obtained as a clear brown liquid.

MS (ESI) m/z =213[M+H]⁺.

5. Intermediate compound: Synthesis of 4-chloro-3-(3-morpholino)-5-nitrobenzamide

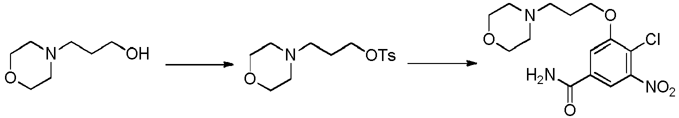

To a solution of 3-morpholinopropan-1-ol (1.0 g, 9.79 mmol) in dichloromethane (20 mL) were added TEA (2.7 mL, 19.58 mmol) and p-toluenesulfonyl chloride (2.0 g, 10.77 mmol). The mixture was reacted at room temperature for 2 h, and water and dichloromethane were added for extraction, and the organic phase was washed with water and saturated brine respectively, dried with anhydrous sodium sulfate, filtered and concentrated to obtain 4-morpholinopropyl 4-methylbenzenesulfonate, which was used directly in the next reaction.

To a solution of 4-chloro-3-hydroxy-5-nitrobenzamide (845 mg, 3.9 mmol) in DMF (10 mL), potassium carbonate (1.08 g, 7.8 mmol), potassium iodide (0.12 g, 0.72 mmol) and 4-morpholinopropyl 4-methylbenzenesulfonate (1.17 g, 3.9 mmol) were added. The reaction was performed at 75°C for 24 h. After the reaction, the inorganic salts were removed by filtration. The filtrate was poured into water and extracted with ethyl acetate. The organic phase was washed with water and saturated brine successively, and then dried and concentrated to obtain 4-chloro-3-(3-morpholino)-5-nitrobenzamide (1.0 g, 75.5% yield).

6. Intermediate compound: synthesis of tert-butyl 4-(3-(2-chloro-5-(methoxycarbonyl)-3-nitrophenoxy)propyl)piperazine-1-carboxylate

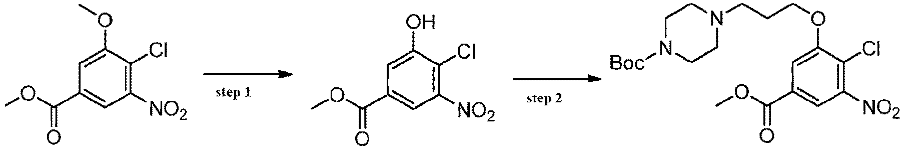

### Step 1: Synthesis of methyl 4-chloro-3-hydroxy-5-nitrobenzoate

Methyl 4-chloro-3-methoxy-5-nitrobenzoate (10 g, 40.7 mmol) was dispersed in anhydrous dichloromethane (100 mL). Boron tribromide (40.8 g, 162.8 mmol) was added slowly dropwise under an ice bath. After the addition, the mixture was slowly warmed to room temperature and stirred overnight. After the reaction was completed, methanol was slowly added dropwise to quench the reaction under an ice bath, and then it was spin-dried to dryness. Methanol (100 mL) and concentrated sulfuric acid (0.2 mL) were added to it, and the reaction solution was heated to 75°C and stirred overnight. After cooling, the solvent was removed by concentration under reduced pressure, and 150 mL of water was added. After ultrasonic dispersion, the mixture was filtered, the solid was washed with water again, and the solid was dried to obtain methyl 4-chloro-3-hydroxy-5-nitrobenzoate (8.89 g, 38.4 mmol).

### Step 2: synthesis of tert-butyl 4-(3-(2-chloro-5-(methoxycarbonyl)-3-nitrophenoxy) propyl)piperazine-1-carboxylate

Methyl 4-chloro-3-hydroxy-5-nitrobenzoate (10 g, 47.2 mmol), tert-butyl 4-(3-hydroxypropyl)piperazine-1-carboxylate (10.6 g, 47.2 mmol) ) and triphenylphosphine (20.4 g, 77.7 mmol) were dissolved in anhydrous THF (200 mL) under nitrogen protection. Under an ice bath, DIAD (15.8 g, 77.7 mmol) was added dropwise to the above reaction solution. After the addition, the reaction solution was raised to room temperature and stirred for 16 hours. The reaction solution was poured into water, extracted with ethyl acetate, and the organic phase was washed with saturated brine, dried over anhydrous magnesium sulfate and concentrated. The obtained crude product was purified by silica gel column (eluent: petroleum ether/ethyl acetate = 2/1) to obtain the target compound as a pale yellow solid (13 g, yield 61%).

7. Intermediate compound: synthesis of tert-butyl 4-(3-(2-chloro-5-(methoxycarbonyl)-3-nitrophenoxy)propyl)piperazine-1-carboxylate

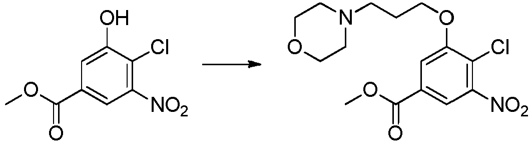

Methyl 4-chloro-3-hydroxy-5-nitrobenzoate (36.8 g, 159.3 mmol) was dissolved in DMF (300 mL), and 4-morpholinopropyl 4-methylbenzenesulfonate (74 g, 247.5 mmol) and K₂CO₃ (44.5 g, 318.6 mmol) were added. The reaction solution was stirred at 75° C for 16 h, the inorganic salt was filtered out, and DMF was distilled off under reduced pressure. The crude product was dissolved in ethyl acetate, washed with water and saturated brine respectively, dried over anhydrous magnesium sulfate, filtered and concentrated to the remaining ethyl acetate (30 mL), filtered to obtain the target compound as a yellow solid (48.7 g, yield 86%).

8. Intermediate compound: synthesis of methyl 4-fluoro-3-(methylthio)-5-nitrobenzoate

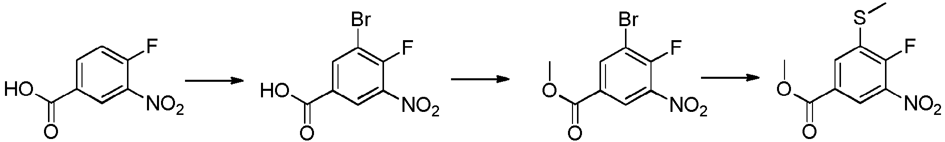

### Step 1: Synthesis of 3-bromo-4-fluoro-5-nitrobenzoic acid

4-Fluoro-3-nitrobenzoic acid (50g, 270mmol) was dissolved in concentrated sulfuric acid (200ml), and NBS (47.5g, 270mmol) was added. The mixture was warmed up to 75°C and stirred overnight. After cooling to room temperature, the mixture was slowly poured into ice water and stirred. A pale yellow solid precipitated out. The solid was filtered and dried to obtain the target compound (66 g).

### Step 2: Synthesis of methyl 3-bromo-4-fluoro-5-nitrobenzoate

In an ice bath, thionyl chloride (44.5g, 373.5mmol) was added dropwise to a solution of 3-bromo-4-fluoro-5-nitrobenzoic acid (66g, 249mmol) in methanol (400ml). The temperature was raised to 75°C. The mixture was stirred overnight, and concentrated to about 100ml of remaining solvent. After cooling, a solid continued to precipitate out. The solid was filtered and dried to obtain methyl 3-bromo-4-fluoro-5-nitrobenzoate (56g).

### Step 3: Synthesis of methyl 4-fluoro-3-(methylthio)-5-nitrobenzoate

To a solution of methyl 3-bromo-4-fluoro-5-nitrobenzoate (80 mg, 0.14 mmol) in toluene () and tert-butanol (mL), methyl mercaptan acetate (11 mg, 0.14 mmol), Pd₂(dba)₃ (13 mg, 0.014 mmol), xantphos (8 mg, 0.014 mmol), K₃PO₄ (36 mg, 0.166 mmol) were added. The mixture was reacted overnight at 110°C under nitrogen protection, and the reaction was completed. After cooling to room temperature, the reaction solution was concentrated, and the crude product obtained was purified by normal phase column (eluent: PE/EA=3/1, v/v) to separate and obtain the target compound (g, yield 45%).

9. Intermediate compound: Synthesis of methyl 4-fluoro-3-((3-morpholinopropyl)thio)-5-nitrobenzoate

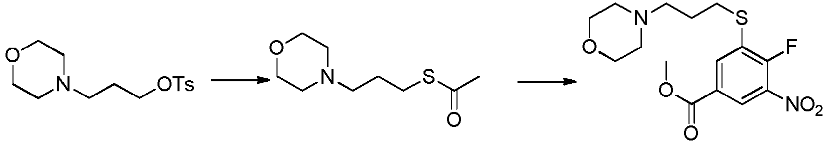

### Step 1: Synthesis of 3-morpholinopropyl mercaptan acetate

To a solution of 4-morpholinopropyl 4-methylbenzenesulfonate (9 g, 30.1 mmol) in DMF (100 mL) was added potassium thioacetate (6.86 g, 60.2 mmol), potassium carbonate (12.5 g, 90.3 mmol), the mixture was stirred at room temperature overnight. The inorganic salt was removed by filtration and the solvent was spin-dried. The crude product was purified by silica gel column (eluent: dichloromethane/methanol=20/1) to obtain the target compound (5 g, yield 81%).

### Step 2: Synthesis of methyl 4-fluoro-3-((3-morpholinopropyl)thio)-5-nitrobenzoate

To a solution of methyl 3-bromo-4-fluoro-5-nitrobenzoate (3.4 g, 12.3 mmol) in toluene (50 mL) and tert-butanol (5 mL) were sequentially added 3-morpholinopropyl mercaptan acetate (3 g, 14.8 mmol), Pd₂(dba)₃ (1.13 g, 1.23 mmol), xantphos (0.72 g, 1.23 mmol), K₃PO₄ (7.83 g, 36.9 mmol). The mixture was reacted overnight at 110°C under nitrogen protection, and the reaction was completed. After cooling to room temperature, the reaction solution was concentrated, and the crude product obtained was purified by normal phase column (eluent: PE/EA=1/1, v/v) to separate and obtain the target compound (1.8 g, yield 41%).

10. Intermediate compound: Synthesis of 1-ethyl-4-fluoro-3-methyl-1H-pyrazole-5-carbonyl isothiocyanate:

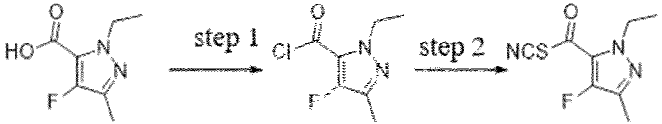

### Step 1: Synthesis of 1-ethyl-4-fluoro-3-methyl-1H-pyrazole-5-carbonyl chloride

1-Ethyl-4-fluoro-3-methyl-1H-pyrazole-5-carboxylic acid (2 g, 11.7 mmol) was dispersed in dry DCM (40 ml), to which oxalyl chloride (1.9 g, 15.1 mmol) and a catalytic amount of DMF were add dropwise under an ice bath. After reacting for 1 hour at room temperature, the volatiles were removed by rotary evaporation under reduced pressure. DCM (20ml) was added to the crude product, and the solvent was removed by rotary evaporation to obtain 4-ethyl-2-methylthiazole-5-carboxylic acid chloride (2.2 g, 100% yield), which was directly used in the next reaction.

### Step 2: Synthesis of 1-ethyl-4-fluoro-3-methyl-1H-pyrazole-5-carbonyl isothiocyanate

At 0°C, 1-ethyl-4-fluoro-3-methyl-1H-pyrazole-5-carbonyl chloride (2.2 g, 11.7 mmol) was dissolved in dry acetone (10 ml) and added dropwise to a solution of potassium thiocyanate (2.3 g, 23.4 mmol) in acetone (50 ml), and the mixture was stirred at room temperature for 3 hours. The reaction system was filtered to remove inorganic salts, and the crude product after concentration of the filtrate was purified by silica gel column (eluent: ethyl acetate/petroleum ether, v/v=1/15), and 4-ethyl-2-methylthiazole-5-carbonyl isothiocyanate (2.15 g, 10.2 mmol, yield 87%) was obtained as a clear brown-yellow liquid.
MS (ESI) m/z =214[M+H]⁺

The following is the preparation of the compounds of the present invention:

### Example 1

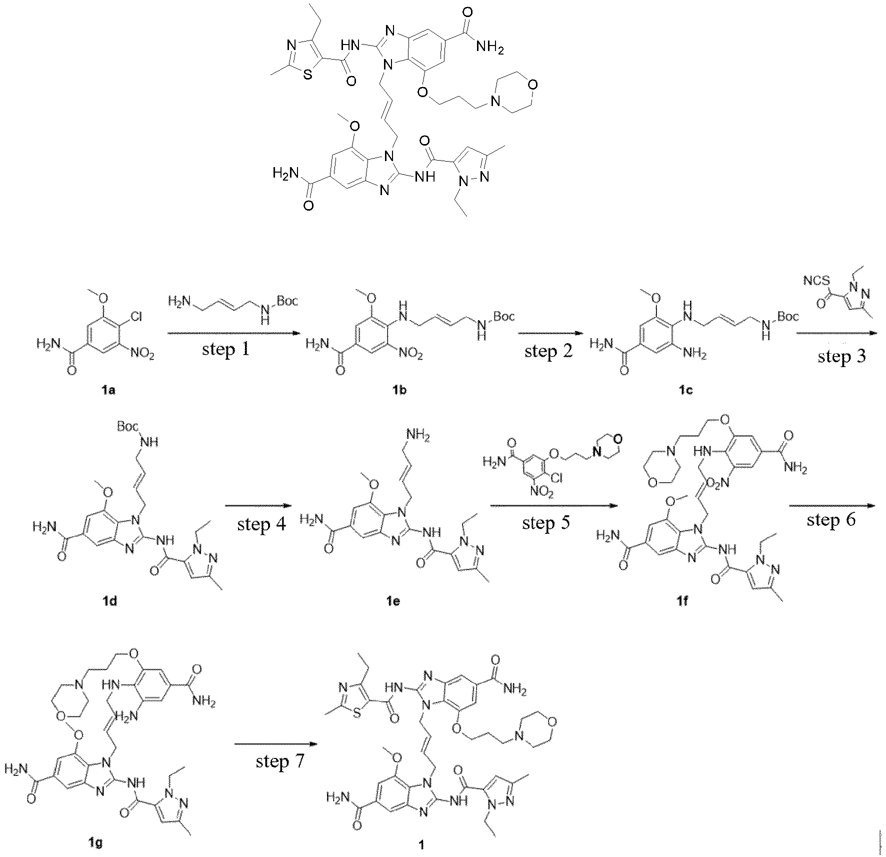

### Step 1: Synthesis of (trans)-tert-butyl (4-(4-carbamoyl-2-methoxy-6-nitrophenyl)amino) n-but-2-enyl)carbamate

4-Chloro-3-methoxy-5-nitrobenzamide (500 mg, 2.17 mmol) and tert-butyl (4-aminobut-2-en-1-yl)carbamate (966 mg, 4.34 mmol) were dissolved in dimethyl sulfoxide (10 mL), and triethylamine (1.1 g, 10.84 mmol) was added dropwise to the reaction solution. The reaction mixture was heated to 115 °C and stirred at this temperature overnight. After cooling to room temperature, the mixture was diluted with water, a large amount of orange solid precipitated, filtered, the filter cake was washed with water (15 mL × 3). Drying was performed by vacuum rotary evaporator to obtain (*trans*)-tert-butyl (4-(4-carbamoyl-2-methoxy-6-nitrophenyl)amino)n-but-2-enyl*)*carbamate (compound **1b)** (450 mg, yield 57%) as orange-yellow solid.

MS(ESI) m/z = 381 [M+H]⁺.

### Step 2: Synthesis of (trans)-tert-butyl (4-((2amino-4-carbamoyl-6-methoxyphenyl)amino)n-but-2-enyl)carbamate

Compound **1b** (450 mg, 1.18 mmol) was dissolved in methanol (15 mL), the solution was cooled to 0 °C, and then aqueous ammonia (1.62 mL, 11.80 mmol) and a solution of sodium dithionite (1.22 g, 7.01 mmol) in water (6 mL) were added successively. The reaction mixture was stirred at 0 °C for 1 h, and the color of the reaction liquid changed from orange-red to white. The reaction solution was rotated to remove methanol, then diluted with water, and extracted with ethyl acetate (20 mL × 4). The separated organic phase was washed with saturated brine (20 mL × 2), dried over anhydrous sodium sulfate, and then spin-dried to obtain (*trans*)-tert-butyl (4-((2-amino-4-carbamoyl-6-methoxyphenyl)amino)n-but-2-enyl)carbamate (compound **1c**) (260 mg, yield 58 %), as white solid.

MS(ESI) m/z = 351 [M+H]⁺.

### Step 3: Synthesis of (trans)-tert-butyl (4-(5-carbamoyl-2-(1-ethyl-3-methyl-1hydro-pyrazole-5carboxamide)-7-methoxy-1H-benzimidazolyl) n-but-2-enyl) carbamate

Compound **1c** (260 mg, 0.742 mmol) and compound 1-ethyl-3-methyl-1H-pyrazole-5-formyl isothiocyanate (145 mg, 0.74 mmol) were dissolved in N,N-dimethylformamide (5mL), and the mixture was stirred at room temperature for 1 h, then HATU (338 mg, 0.89 mmol) and 1,8-diazabicyclo[5.4.0]undec-7-ene (147 mg , 0.96 mmol) were added to the reaction solution and stirred at room temperature for 1 h. The reaction solution was diluted with water, and the white solid produced was collected by filtration and washed with water (5 mL×3), and then separated by reverse phase column (eluent: acetonitrile/water = 1/3, v/v) to obtain (*trans*)-tert-butyl (4-(5-carbamoyl-2-(1-ethyl-3-methyl-1hydro-pyrazole-5-carboxamide))-7-methoxy-1H-benzimidazolyl) -n-but-2-enyl)carbamate (compound 1d) (313 mg, yield 82%), as white solid.
MS(ESI)m/z = 512[M+H]⁺
¹H NMR (400 MHz, DMSO-d₆) δ 12.86(s,1H), 8.02(s,1H), 7.67(s,1H), 7.48-7.32 (m,2H), 7.00-6.87(m,1H), 6.65(s,1H), 5.85--5.50(m,2H), 4.93(d,2H), 4.61(q,2H), 3.98(s,3H), 3.51(m,2H), 2.55(m,2H) , 2.18 (s,3H), 1.35(t,3H), 1.32(s,9H).

### Step 4: Synthesis of (trans)-1-(4-amino-n-but-2-enyl)-2-(1-ethyl-3-methyl-1hydro-pyrazole-5-carboxamido)-7-methoxy-1H-benzimidazole-5-carboxamide

Compound **1d** (290 mg, 0.57 mmol) was dissolved in methanol (10 mL), and hydrochloric acid (6N aqueous solution, 3 mL, 18 mmol) was added dropwise. After the reaction solution was stirred at room temperature for 30 minutes, the temperature was raised to 40 °C and stirring was continued for 3 h. The reaction solution was spin-dried, and the obtained crude product was separated and purified by reversed-phase column (eluent: water/acetonitrile = 50/50, v/v) to obtain compound (*trans*)-1-(4-amino-n-but-2-ene)-2-(1-ethyl-3-methyl-1hydro-pyrazole-5-carboxamide) -7-methoxy-1hydro-benzimidazole-5-carboxamide (compound **1e**) (230 mg, yield 98%), as pale yellow solid.

MS(ESI) m/z = 412.0 [M+H]⁺.

### Step 5: Synthesis of (E)-1-(4-((4-carbamoyl-2-(3-morpholino)-6-nitrophenyl)amino)but-2-en-1-yl)-2-(1-ethyl-3-methyl-1H-pyrazole-5-carboxamide)-7-methoxy-1H-benzo[d]imidazole-5-carboxamide

Compound **1e** (150 mg, 0.37 mmol), 4-chloro-3-(3-morpholino)-5-nitrobenzamide (127 mg, 0.37 mmol) and DIPEA (220 mg, 1.85 mmol) were dissolved in n-butanol (5 mL), the reaction mixture was heated to 120 °C and stirred at this temperature for 48 h. After cooling to room temperature, the mixture was diluted with water, extracted with ethyl acetate (20 mL × 3), and the combined organic phase was washed with saturated brine (15 mL × 2), dried with anhydrous sodium sulfate. The solvent was spin-dried, the crude product obtained was separated by reversed-phase column chromatography (eluent: acetonitrile/water = 30/70, v/v) to obtain (E)-1-(4-((4-carbamoyl-2-(3-morpholino)-6-nitrophenyl)amino)but-2-en-1-yl)-2-(1-ethyl-3-methyl-1H-pyrazole-5-carboxamide)-7-methoxy-1H-benzo[d]imidazole-5-carboxamide (compound **1f)** (130 mg, yield 48.9%).

MS(ESI) m/z = 719.3 [M+H]⁺.

### Step 6: Synthesis of (E)-1-(4-((2-amino-4-carbamoyl-6-(3-morpholino)phenyl)amino) but-2-en-1-yl)-2-(1-ethyl-3-methyl-1H-pyrazole-5-carboxamide)-7-methoxy-1H-benzo[d] imidazole-5-carboxamide

Under an ice bath, ammonium hydroxide (0.35 mL) was added dropwise to a solution of compound **1f** (130 mg, 0.18 mmol) in methanol (5 mL). After the reaction solution was stirred at 0 °C for 5 minutes, an aqueous solution (2 mL) of sodium dithionite (154 mg, 0.28 mmol) was slowly added dropwise. The reaction mixture was slowly heated to room temperature, and stirring was continued for 3 h. The mixture was dilute with water, filtered, and the filtrate was spin-dried. The obtained crude product was separated by reverse phase column chromatography (eluent: acetonitrile/water=50/50, v/v) to obtain (E)-1-(4-((2-amino)-4-carbamoyl-6-(3-morpholino)phenyl) amino)but-2-en-1-yl)-2-(1-ethyl -3-methyl-1H-pyrazole-5-formylamino)-7-methoxy-1H-benzo[d] imidazole-5-carboxamide **1g** (91 mg, yield 74%), as white solid.

MS(ESI) m/z = 688.3 [M+H]⁺.

### Step 7: Synthesis of (E)-N-(5-carbamoyl-1-(4-(5-carbamoyl-2-(1-ethyl-3-methyl -1H-pyrazole-5-formylamino)-7-methoxy-1H-benzo[d]imidazol-1-yl)but-2-en-1-yl)-7-(3-morpholinomethyl)-1H-benzo[d]imidazol-2-yl)-4-ethyl-2-methylthiazole-5-carboxamide

Compound **1g** (91 mg, 0.13 mmol) and 4-ethyl-2-methylthiazole-5-carbonyl isothiocyanate (28 mg, 0.13 mmol) were mixed and dissolved in DMF (3 mL), and stirred at room temperature for 0.5 h, then HATU (49 mg, 0.13 mmol) and DIPEA (34 mg, 0.26 mmol) were added successively, and stirring was continued at room temperature for 2 h. The reaction solution was purified by reverse phase HPLC to obtain compound **1** (21 mg, yield 19%) as white solid.
MS(ESI)m/z = 867.4 [M+H]⁺
¹H NMR (400M, DMSO-d₆) δ 12.80-12.92(m, 1H), 9.64-9.73(m,1H), 7.93-8.01 (m, 1H), 7.64-7.67(m, 2H), 7.28-7.43(m, 3H), 6.52-6.57(m, 2H), 5.76-5.82(m, 2H), 4.85-4.94(m, 4H), 4.51-4.58(m, 2H), 3.99-4.05(m, 3H), 3.90-3.97(m, 4H), 3.71(s, 3H), 3.24-3.32(m, 4H), 3.11-3.17(m, 4H), 2.92-3.06(m, 4H), 2.65-2.70(m, 4H)), 2.54-2.55(m, 4H), 2.31-2.36(m, 4H), 2.11 (s, 3H), 1.86-1.95(m, 2H), 1.26-1.33(m, 3H), 1.14-1.21(m, 3H).

### Example 2

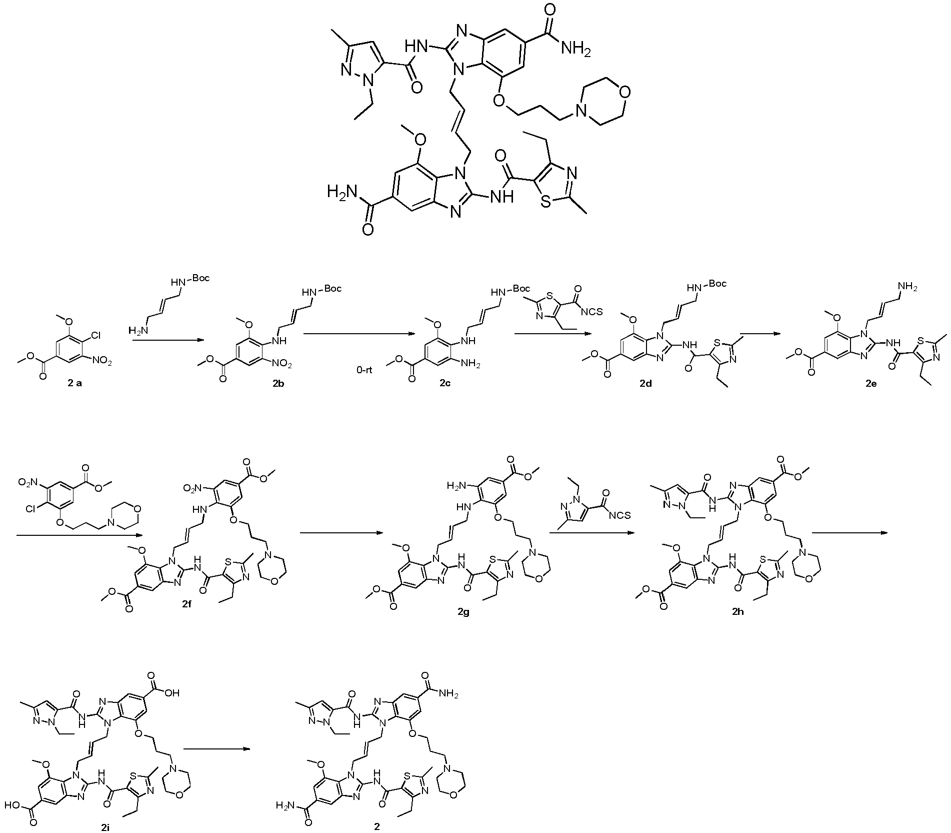

### Step 1: Synthesis of methyl (E)-4-((4-((tert-butoxycarbonyl)amino)but-2-en-l-yl)amino)-3-methoxy-5-nitrobenzoate

Methyl 4-chloro-3-methoxy-5-nitrobenzoate (30 g, 122 mmol) was dispersed in n-butanol (500 mL), then tert-butyl (E)-(4-aminobutane-2-en-1-yl)carbamate (22.8 g, 122 mmol) and DIPEA (78.9 g, 609 mmol) were added. The reaction solution was heated to 120°C and stirred for 18 h. The crude product obtained by distillation under reduced pressure was dissolved in ethyl acetate, and an appropriate amount of dilute hydrochloric acid (0.5M) was added in an ice bath to adjust the pH to neutral. The separated organic phase was washed with water and saturated brine respectively, dried over anhydrous magnesium sulfate and spin-dried to obtain **2b** (48.3 g, yield 99%).
MS(ESI)m/z = 396.3[M+H]

### Step 2: Synthesis of methyl (E)-3-amino-4-((4-((tert-butoxycarbonyl)amino)but-2-en-1-yl)amino)-5-methoxybenzoate

Under an ice bath, ammonium hydroxide (120 mL) was added to a solution of 2b (48.3 g, 122 mmol) in methanol (400 mL). After 10 minutes, an aqueous solution of sodium dithionite (106.3 g, 611 mmol) was added and the mixture was slowly heated to room temperature and reacted for 2h. Inorganic salts were filtered off add ethyl acetate was added for extraction. The organic phase was washed with saturated brine, dried over anhydrous magnesium sulfate and spin-dried to obtain the crude product, which was purified on a silica gel column (eluent: petroleum ether/ethyl acetate = 1/1) to obtain compound **2c** (28.6 g, yield 64 %).
MS(ESI)m/z = 366.3[M+H]

### Step 3: synthesis of methyl (E)-1-(4-((tert-butoxycarbonyl)amino)but-2-en-1-yl)-2-(4-ethyl-2-methylthiazole-5-formylamido)-7-methoxy-1H-methyl-benzo[d]imidazole-5-carboxylate

Under an ice bath, 4-ethyl-2-methylthiazole-5-carbonyl isothiocyanate (8.7 g, 41.1 mmol) was added to a solution of **2c** (15 g, 41.1 mmol) in DMF (200 mL). After reacting for 0.5 h, DIPEA (15.9 g, 123.3 mmol) and HATU (18.8 g, 49.3 mmol) were added, and then heated to room temperature to react for 12 h. The reaction solution was slowly poured into water, and a yellow solid precipitated out. The solid was filtered and dried to obtain **2d** (14.4 g, yield 64%).
MS(ESI)m/z = 544.3[M+H]

### Step 4: synthesis of methyl (E)-1-(4-aminobut-2-en-1-yl)-2-(4-ethyl-2-methylthiazole-5-formylamino)-7-methoxy-1H-benzo[d]imidazole-5-carboxylate

Under an ice bath, TFA (6 mL) was added to a solution of 2d (6 g, 11 mmol) in DCM (200 mL), and then the mixture was heated to room temperature and reacted for 2 h. The solvent was spin-dried under reduced pressure, ethyl acetate was added and then free TFA was removed by spin-drying under reduced pressure to obtain compound **2e** (4.9 g, yield 99%).
MS(ESI)m/z =444.3[M+H]

### Step 5: Synthesis of methyl (E)-2-(4-ethyl-2-methylthiazole-5-formylamino)-7-methoxy-1-(4-((4-(methoxycarbonyl)-2-(3-morpholinopropoxy)-6-nitrophenyl)amino)but-2-en-1-yl)-1H-benzo[d]imidazole-5-carboxylate

2e (4.9 g, 11 mmol) was dispersed in n-butanol (100 mL), and methyl 4-chloro-3-(3-morpholinopropoxy)-5-nitrobenzoate (4.0 g , 11 mmol) and DIPEA (7.2 g, 55 mmol) were added. The reaction solution was heated to 120°C and stirred for 18 h. The crude product obtained by distillation under reduced pressure was dissolved in ethyl acetate, washed with water and saturated brine, dried over anhydrous magnesium sulfate and spin-dried, and purified by silica gel column (eluent: petroleum ether/ethyl acetate = 1/2) to obtain **2f** (8.4 g, yield 99%).
MS(ESI)m/z = 766.3[M+H]

### Step 6: Synthesis of methyl (E)-1-(4-((2-amino-4-(methoxycarbonyl)-6-(3-morpholinopropoxy)phenyl)amino)but-2-ene-1-yl)-2-(4-ethyl)-2-methylthiazole-5-formylamino)-7-methoxy-1H-benzo[d]imidazole-5-carboxylate

Under an ice bath, ammonium hydroxide (11 mL) was added to a solution of **2f** (8.4 g, 11 mmol) in methanol (50 mL). After 10 minutes, an aqueous solution of sodium dithionite (9.6 g, 55 mmol) was added and slowly warmed to room temperature to react for 2h. Inorganic salts were filtered off, and ethyl acetate was added for extraction. The organic phase was washed with saturated brine, dried over anhydrous magnesium sulfate and spin-dried to obtain the crude product, and then purified with a silica gel column (eluent: petroleum ether/ethyl acetate = 1/1) to obtain compound **2g** (5.1 g, yield 63%).
MS(ESI)m/z = 736.3[M+H]

### Step 7: synthesis of methyl (E)-2-(4-ethyl-2-methylthiazole-5-formylamino)-1-(4-(2-(1-ethyl-3-methyl-1H-pyrazole-5-formylamino)-5-(methoxycarbonyl)-7-(3-morpholinomethyl)-1H-benzo[d]imidazol-1-yl)but-2-ene-1-yl)-7-methoxy-1H-benzo[d]imidazole-5-carboxylate

Under an ice bath, to a solution of **2g** (5.1 g, 6.94 mmol) in DMF (100 mL) was added 1-ethyl-3-methyl-1H-pyrazole-5-carbonyl isothiocyanate (1.4 g, 6.94 mmol). After reacting for 0.5 h, DIPEA (2.7 g, 20.82 mmol) and HATU (3.2 g, 8.33 mmol) were added, and then heated to room temperature to react for 12 h. The reaction solution was slowly poured into water, a yellow solid precipitated out. The solution was filtered and the solid was dried to obtain **2h** (4.8 g, yield 77%).
MS(ESI)m/z = 897.3[M+H]

### Step 8: Synthesis of (E)-1-(4-(5-carboxy-2-(1-ethyl-3-methyl-1H-pyrazole-5-formylamino)-7-(3-morpholinomethyl)-1H-benzo[d]imidazol-1-yl)but-2-en-1-yl)-2-(4-ethyl-2-methylthiazole-5-formylamino)-7-methoxy-1H-benzo[d]imidazole-5-carboxylic acid

2h (4.8 g, 5.38 mmol) was dissolved in a mixed solution (20 ml) of methanol, tetrahydrofuran and water (volume ratio: 1/1/1), and lithium hydroxide (2.3 g 53.8 mmol) was added. The temperature was raised to 75°C and the reaction was stirred overnight. The organic solvent was removed by rotary evaporation, the mixture was cooled to room temperature, and adjusted with dilute hydrochloric acid (1M) in an ice bath until the solid no longer precipitated. The solid was filtered and dried to obtain compound **2i** (4.65 g, yield 99%).
MS(ESI)m/z = 869.3[M+H]

### Step 9: synthesis of (E)-N-(5-carbamoyl-1-(4-(5-carbamoyl-2-(1-ethyl-3-methyl-1H-pyrazole-5-formylamino)-7-(3-morpholino)-1H--benzo[d]imidazol-1-yl)but-2-en-1-yl)-7-methoxy-1H-benzo[d]imidazol-2-yl)-4-ethyl-2-methylthiazole-5-formamide

**2i** (4.65 g, 5.38 mmol) was dissolved in DMF (100 mL), and HATU (4.9 g, 12.86 mmol) and DIPEA (4.2 g, 32.14 mmol) were added. After 0.5h, ammonium bicarbonate (1.3 g, 16.07 mmol) was added, and the mixture was stirred at room temperature for 2h. The reaction solution was concentrated and purified by preparative HPLC to obtain compound 2 (2.5 g, yield 54%).
MS(ESI)m/z = 867.3[M+H]
¹H NMR (400M, DMSO-d₆) δ 12.79-12.96(m, 1H), 11.30-11.46(m, 1H), 7.92-8.14 (m, 2H), 7.63-7.69 (m, 2H), 7.33-7.45( m, 3H), 7.26-7.32(m, 1H), 6.53-6.59(m, 1H), 5.81-5.92(m, 1H), 5.68-5.79(m, 1H), 4.51-4.59(m, 2H), 3.97-4.04(m, 2H), 3.85-3.93(m, 2H), 3.81(s, 3H), 3.72-3.79(m, 2H), 3.02-3.24(m, 6H), 2.85-2.98(m, 2H) ), 2.58(s, 3H), 2.13(s, 3H), 1.90-2.01(m, 2H), 1.30(t, J=7.08 Hz, 3H), 1.17(t, J=7.44 Hz, 3H).

### Example 3

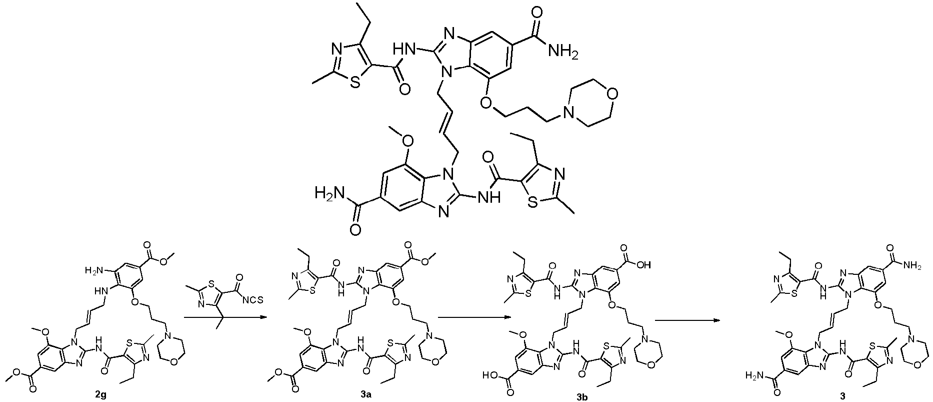

### Step 1: synthesis of methyl (E)-2-(4-ethyl-2-methylthiazole-5-formylamino)-1-(4-(2-(4-ethyl-2-methylthiazole-5-formylamino)-5-(methoxycarbonyl)-7-methyl ester-(3-morpholinomethyl)-1H-benzo[d]imidazol-1-yl)but -2-ene-1-yl)-7-methoxy-1H-benzo[d]imidazole-5-carboxylate

Under an ice bath, to a solution of **2g** (0.5 g, 0.69 mmol) in DMF (5 mL) was added 4-ethyl-2-methylthiazole-5-carbonyl isothiocyanate (0.18 g, 0.83 mmol). After reacting for 0.5 h, DIPEA (0.27 g, 2.08 mmol) and HATU (0.32 g, 0.83 mmol) were added, and then heated to room temperature to react for 12 h. The reaction solution was slowly poured into water, and a yellow solid precipitated out. The solid was filtered and dried to obtain **3a** (623 mg, yield 99%).
MS(ESI)m/z = 914.3.3[M+H]

### Step 2: synthesis of (E)-1-(4-(5-carboxy-2-(4-ethyl-2-methylthiazole-5-formylamino)-7-(3-morpholinomethyl)-1H-benzo[d]imidazol-1-yl)but-2-en-1-yl)-2-(4-ethyl-2-methylthiazole-5-formylamino)-7-methoxy-1H-benzo[d]imidazole-5-carboxylic acid

3a (623 mg, 0.68 mmol) was dissolved in a mixed solution of methanol, tetrahydrofuran and water (volume ratio: 1/1/1), and hydrated lithium hydroxide (0.17 g 4.08 mmol) was added. The temperature was raised to 75°C and the reaction was stirred overnight. The organic solvent was removed by rotatory evaporation, the mixture was cooled to room temperature, adjusted with dilute hydrochloric acid (1M) in an ice bath until the solid no longer precipitates, filtered, and the solid was dried to obtain compound **3b** (610 mg, yield 99%).
MS(ESI)m/z = 886.3[M+H]

### Step 3: synthesis of (E)-N-(5-carbamoyl-1-(4-(5-carbamoyl-2-(4-ethyl-2-methylthiazole-5-formylamino)-7-(3-morpholinomethyl)-1H-benzo[d]imidazol-1-yl)but-2-en-1-yl)-7-methoxy-1H-benzo[d]imidazole-2-yl)-4-ethyl-2-methylthiazole-5-formamide

3b (610 mg, 0.68 mmol) was dissolved in DMF (10 mL), and HATU (4.9 g, 1.36 mmol) and DIPEA (0.53 g, 4.08 mmol) were added. After 0.5h, ammonium bicarbonate (0.16 g, 2.04 mmol) was added, and the mixture was stirred at room temperature for 2h. The reaction solution was concentrated and purified by preparative HPLC to obtain compound **3** (206 mg, yield 34%).
MS(ESI)m/z = 884.3[M+H]
¹H NMR (400M, DMSO-d₆) δ 12.80-12.91(m, 1H), 11.10-11.23(m, 1H), 7.91-8.12 (m, 2H), 7.60-7.69 (m, 2H), 7.26-7.48( m, 4H), 5.71-5.95(m, 2H), 3.98-4.11(m, 2H), 3.85-3.95(m, 2H), 3.71-3.83(m, 5H), 3.04-3.29(m, 8H), 2.88-3.01(m, 2H), 2.56-2.60(m, 4H), 1.95-2.04(m, 2H), 1.12-1.24(m, 6H).

### Example 4

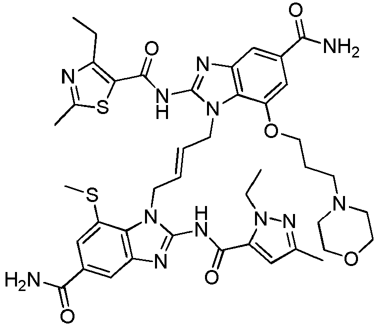

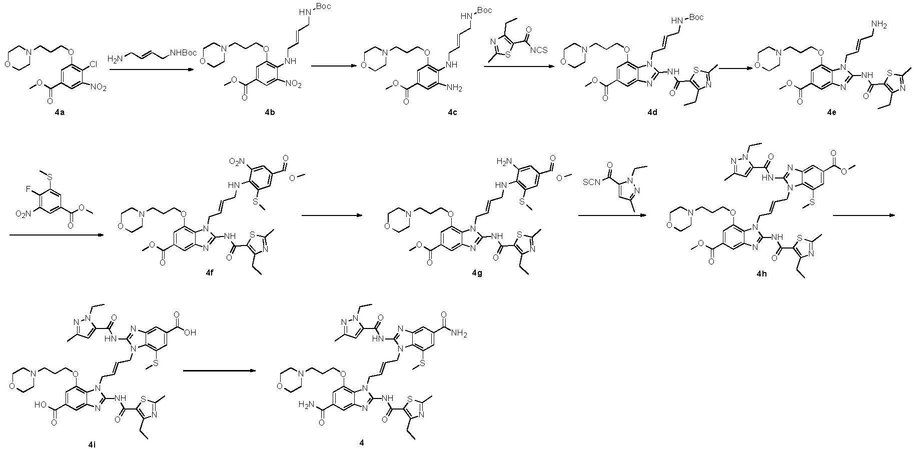

### Step 1: synthesis of methyl (E)-4-((4-((tert-butoxycarbonyl)amino)but-2-en-1-yl)amino)-3-(3-morpholinopropoxy)-5-nitrobenzoate

Methyl 4-chloro-3-(3-morpholinopropoxy)-5-nitrobenzoate (6.8 g, 19 mmol) was dispersed in n-butanol (100 mL), and then tert-butyl (E)-(4-aminobut-2-en-1-yl)carbamate (3.5 g, 19 mmol) and DIPEA (12.3 g, 95 mmol) were added. The reaction solution was heated to 120°C and stirred for 18 h. The crude product obtained by distillation under reduced pressure was dissolved in ethyl acetate, and an appropriate amount of dilute hydrochloric acid (0.5M) was added in an ice bath to adjust the pH to neutral. The separated organic phase was washed with water and saturated brine respectively, dried over anhydrous magnesium sulfate and spin-dried to obtain **4b** (7.3 g, yield 76%).
MS(ESI)m/z = 509.3[M+H]

### Step 2: synthesis of methyl (E)-3-amino-4-((4-((tert-butoxycarbonyl)amino)but-2-en-1-yl)amino)-5-(3-morpholinopropoxyl)benzoate

Under an ice bath, ammonium hydroxide (15 mL) was added to a solution of 4b (7.3 g, 14.4 mmol) in methanol (100 mL). After 10 minutes, aqueous solution of sodium dithionite (12.5 g, 71.9 mmol) was added and the mixture was slowly heated to react at room temperature for 2 h. Inorganic salts were removed by filtration, and ethyl acetate was added for extraction. The organic phase was washed with saturated brine, dried over anhydrous magnesium sulfate and spin-dried to obtain the crude product, which was purified on a silica gel column (eluent: petroleum ether/ethyl acetate = 1/2) to obtain compound **4c** (4.5 g, yield 65 %).
MS(ESI)m/z = 479.3 [M+H]

### Step 3: synthesis of methyl (E)-1-(4-((tert-butoxycarbonyl)amino)but-2-en-1-yl)-2-(4-ethyl-2-methylthiazole-5-formylamino)-7-(3-morpholinomethyl)-1H-benzo[d]imidazole-5-carboxylate

Under an ice bath, to a solution of **4c** (1.8 g, 3.76 mmol) in DMF (30 mL) was added 4-ethyl-2-methylthiazole-5-carbonyl isothiocyanate (0.96 g, 4.52 mmol). After reacting for 0.5 h, DIPEA (1.46 g, 11.3 mmol) and HATU (1.72 g, 4.52 mmol) were added, and then heated to room temperature to react for 12 h. The reaction solution was slowly poured into water, and a yellow solid precipitated out. The solid was filtered and dried to obtain **4d** (1.9 g, yield 77%).
MS(ESI)m/z = 657.3[M+H]

### Step 4: synthesis of methyl (E)-1-(4-aminobut-2-en-1-yl)-2-(4-ethyl-2-methylthiazole-5-formylamino)-7-(3-morpholinopropoxy)-1H-benzo[d]]imidazole-5-carboxylate

Under an ice bath, TFA (3 mL) was added to 4d (1.9 g, 2.9 mmol) in DCM (40 mL), and then heated to room temperature to react for 2 h. The solvent was spin-dried under reduced pressure, ethyl acetate was added and then free TFA was removed by spin-drying under reduced pressure to obtain compound **4e** (1.6 g, yield 99%).
MS(ESI)m/z = 557.3[M+H]

### Step 5: synthesis of methyl (E)-2-(4-ethyl-2-methylthiazole-5-formylamino)-1-(4-((4-(methoxycarbonyl)-2-(methylthio))-6-nitrophenyl)amino)but-2-ketoen-1-yl)-7-(3-morpholinomethyl)-1H-benzo[d]imidazole-5-carboxylate

DIPEA (387 mg, 3 mmol) and methyl 4-fluoro-3-methylthio-5-nitrobenzoate (245 mg, 1 mmol) were added to a solution of methyl (E)-1-(4-aminobut-2-en-1-yl)-2-(4-ethyl-2-methylthiazole-5-formylamino)-7-(3-morpholinepropoxy)-1H-benzo[d]]imidazole-5-carboxylate (556 mg, 1 mmol) in DMF (10 mL), and the mixture was reacted at room temperature for 3 hours. The reaction solution was poured into water, extracted with ethyl acetate, washed with saturated brine and dried over anhydrous sodium sulfate, and the solvent was spin-dried under reduced pressure to obtain compound **4f** (616 mg, yield 79%).
MS(ESI)m/z = 782.3[M+H]

### Step 6: synthesis of methyl (E)-1-(4-((2-amino-4-(methoxycarbonyl)-6-(methylthio)phenyl)amino)but-2-en-1-yl)-2-(4-ethyl-2)methyl ester-methylthiazole-5-formylamino)-7-(3-morpholinomethyl)-1H-benzo[d]imidazole -5-carboxylate

Under an ice bath, ammonium hydroxide (1 mL) was added to a solution of 4f (616 mg, 0.79 mmol) in methanol (5 mL). After 10 minutes, an aqueous solution of sodium dithionite (686 mg, 3.94 mmol) was added and the mixture was slowly heated to react at room temperature for 2 h. Inorganic salts were removed by filtration, and ethyl acetate was added for extraction. The organic phase was washed with saturated brine, dried over anhydrous magnesium sulfate and spin-dried to obtain the crude product, which was then purified with a silica gel column (eluent: petroleum ether/ethyl acetate = 1/1) to obtain compound **4g** (435 mg, yield 73%).
MS(ESI)m/z = 752.3[M+H]

### Step 7: Synthesis of methyl (E)-2-(4-ethyl-2-methylthiazole-5-formylamino)-1-(4-(2-(1-ethyl-3-methyl-1H-pyrazole-5-formylamino)-5-(methoxycarbonyl)-7-(methylthio)-1H-benzo[d] imidazol-1-yl)but-2-en-1-yl)-7-(3-morpholinomethyl)-1H-benzo[d]imidazole-5-carboxylate

Under an ice bath, to a solution of **4g** (145 mg, 0.193 mmol) in DMF (5 mL) was added 1-ethyl-3-methyl-1H-pyrazole-5-carbonyl isothiocyanate (46 mg, 0.232 mmol). After 0.5 h of reaction, DIPEA (75 mg, 0.579 mmol) and HATU (89 mg, 0.232 mmol) were added, and then heated to room temperature to react for 12 h. The reaction solution was slowly poured into water, and a yellow solid precipitated out. The solid was filtered and dried to obtain **4h** (150 mg, yield 85%).
MS(ESI)m/z = 913.3[M+H]

### Step 8: synthesis of (E)-1-(4-(5-carboxy-2-(1-ethyl-3-methyl-1H-pyrazole-5-formylamino)-7-(methylthio)-1H-benzo[d]imidazol-1-yl)but-2-en-1-yl)-2-(4-ethyl-2-methylthiazole-5-formylamino)-7-(3-morpholinopropoxy)-1H-benzo[d]imidazole-5-carboxylic acid

4h (150 mg, 0.16 mmol) was dissolved in a mixed solution of methanol, tetrahydrofuran and water (volume ratio: 1/1/1), and lithium hydroxide (69 mg, 1.64 mmol) was added. The temperature was raised to 75°C and the reaction was stirred overnight. The mixture was spin-dried to remove the organic solvent, cooled to room temperature, adjusted with dilute hydrochloric acid (1M) in an ice bath until the solid no longer precipitated, filtered, and the solid was dried to obtain compound **4i** (126 mg, yield 86%).
MS(ESI)m/z = 885.3[M+H]

### Step 9: Synthesis of (E)-N-(5-carbamoyl-1-(4-(5-carbamoyl-2-(1-ethyl-3-methyl-1H-pyrazole-5-formylamino)-7-(methylthio)-1H-benzo[d]imidazol-1-yl)but-2-en-1-yl)-7-(3-morpholinomethyl)-1H-benzo[d]imidazol-2-yl)-4-ethyl-2-methylthiazole-5--formylamide

**4i** (126 mg, 0.143 mmol) was dissolved in DMF (5 mL), and HATU (131 mg, 0.342 mmol) and DIPEA (110 mg, 0.855 mmol) were added. After 0.5h, ammonium bicarbonate (34 mg, 0.428 mmol) was added and stirred at room temperature for 2h. The reaction solution was concentrated and purified by preparative HPLC to obtain compound **4** (54 mg, yield 43%).
MS(ESI)m/z = 883.3[M+H]
¹H NMR (400M, DMSO-d₆) δ 12.79-12.88(m, 1H), 11.10-11.12(m, 1H), 7.95-8.12 (m, 2H), 7.85-7.89(m, 1H), 7.62-7.68( m, 2H), 7.33-7.46(m, 3H), 6.52(s, 1H), 5.71-5.78(m, 2H), 5.11-5.18(m, 2H), 4.85-4.94(m, 2H), 4.47- 4.55(m, 2H), 4.05-4.10(m, 4H), 3.87-3.91(m, 4H), 3.71-3.81(m, 4H), 3.22-3.29(m, 2H), 3.08-3.18(m, 8H) ), 2.89-3.01(m, 2H), 2.57(s, 3H), 2.46(s, 3H), 2.10(s, 3H), 2.00-2.07(m, 2H), 1.28(t, J= 7.08 Hz, 3H), 1.17(t, J = 7.48 Hz, 3H).

### Example 5

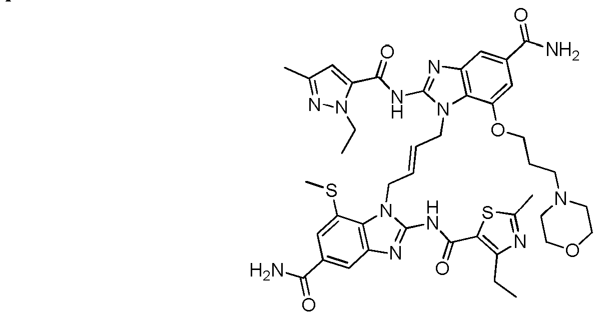

Compound 5 was prepared by the same method as compound 4, except that the ring-closure materials used in step 3 and step 7 were exchanged, that is, 1-ethyl-3-methyl-1H-pyrazole-5-carbonyl isothiocyanate was used in step 3 for ring closure, and 4-ethyl-2-methylthiazole-5-carbonyl isothiocyanate was used in step 7 for ring closure. The other steps were the same as the experiment method, and compound 5 (67 mg) was obtained.
MS(ESI)m/z = 883.3[M+H]
¹H NMR (400M, DMSO-d₆) δ 12.90-13.04(m, 1H), 10.92-11.05(m, 1H), 7.93-8.14 (m, 2H), 7.83-7.87(m, 1H), 7.62-7.70( m, 2H), 7.34-7.48(m, 2H), 7.27-7.32(m, 1H), 6.51-6.67(m, 1H), 5.66-5.83(m, 4H), 5.06-5.12(m, 2H), 4.89-4.96(m, 2H), 4.50-4.59(m, 2H), 3.99-4.05(m, 2H), 3.86-3.94(m, 2H), 3.70-3.80(m, 2H), 3.19-3.27(m , 2H), 3.06-3.15(m, 4H), 2.88-3.00(m, 2H), 2.49(s, 3H), 2.12(s, 3H), 1.95-2.03(m, 2H), 1.30(t, J = 7.08 Hz, 3H), 1.17(t, J = 7.48 Hz, 3H).

### Example 6

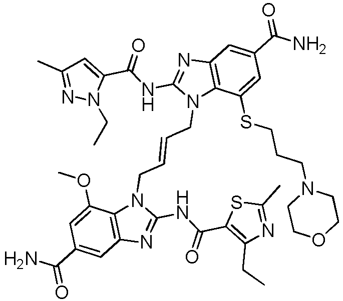

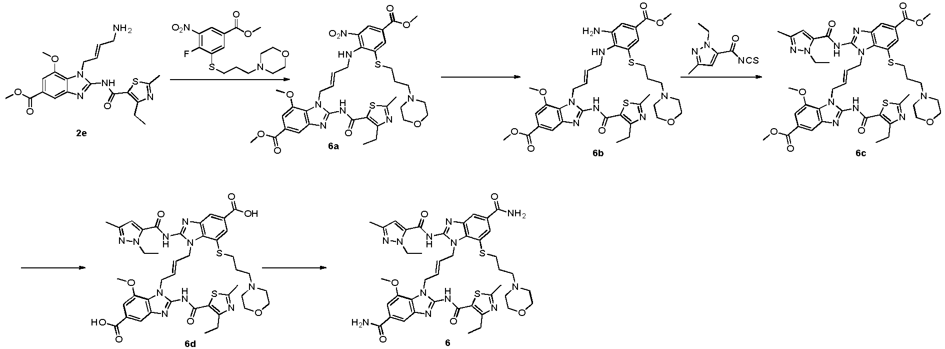

The compound 6 was prepared by the same method as the synthesis of compound 2. Methyl 4-chloro-3-(3-morpholinopropoxy)-5-nitrobenzoate used in the synthesis of compound 2 was replaced with methyl 4-fluoro-3-((3-morpholinopropyl)thio)-5-nitrobenzoate, and other methods were the same.
MS(ESI)m/z = 883.3[M+H]
¹H NMR (400M, DMSO-d₆) δ 12.73-12.84(m, 1H), 10.59-10.82(m, 1H), 7.98-8.14 (m, 2H), 7.91-7.98(m, 1H), 7.75-7.83( m, 1H), 7.59-7.67(m, 1H), 7.31-7.49 (m, 3H), 6.51-6.59(m, 1H), 5.87-5.96(m, 1H), 5.69-5.78(m, 1H), 5.18-5.29(m, 2H), 4.83-4.90(m, 4H), 4.51-4.58(m, 4H), 3.88-3.97(m, 2H), 3.81(s, 3H), 3.67-3.79(m, 2H) ), 3.27-3.37(m, 2H), 3.04-3.15(m, 4H), 2.91-3.03(m, 4H), 2.55(s, 3H), 2.11(s, 3H), 1.77-1.88(m, 2H) ), 1.29(t, J= 7.04 Hz, 3H), 1.14(t, J= 7.48Hz, 3H).

### Example 7

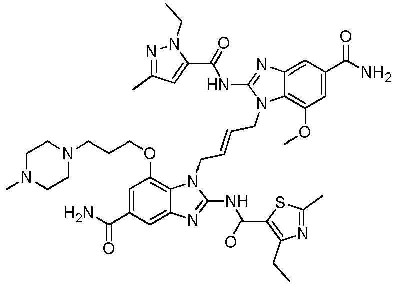

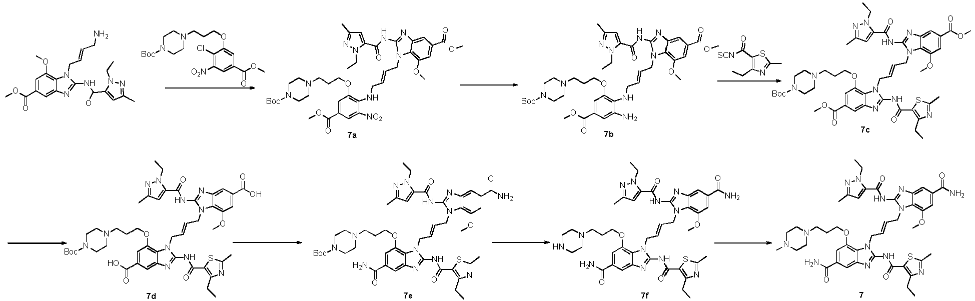

### Step 1: synthesis of methyl (E)-1-(4-((2-(3-(4-(tert-butoxycarbonyl)piperazin-1-yl)propoxy)-4-(methoxycarbonyl)-6-nitrophenyl)amino)but-2-ketoen-1-yl)-2-(1-ethyl-3-methyl-1H-pyrazole-5-formylamino)-7-methoxy-1H-benzo[d]imidazole-5-carboxylate

Methyl (E)-1-(4-aminobut-2-en-1-yl)-2-(1-ethyl-3-methyl-1H-pyrazole-5-formylamino) -7-methoxy-1H-benzo[d]]imidazole-5-carboxylate (0.65 g, 1.4 mmol) was dispersed in n-butanol (15 mL), and tert-butyl 4-(3-(2-chloro-5-(methoxycarbonyl)-3-nitrophenoxy)propyl) piperazine-1-carboxylate (630 mg, 1.4 mmol) and DIPEA (1.8 g, 14 mmol) were added. The reaction solution was heated to 120°C and stirred for 18 h. The crude product obtained by distillation under reduced pressure was dissolved in ethyl acetate, washed with water and saturated brine respectively, dried over anhydrous magnesium sulfate and spin-dried, and purified by silica gel column (eluent: petroleum ether/ethyl acetate = 1/2) to obtain **7a** (312 mg, yield 26%).
MS(ESI)m/z = 865.2 [M+H]+

### Step 2: synthesis of methyl (E)-1-(4-((2-amino-6-(3-(4-(tert-butoxycarbonyl)piperazin-1-yl)propoxy)-4-(methoxycarbonyl)phenyl)amino)but-2-en-1-yl)-2-(1-ethyl-3-methyl-1H-pyrazole-5-formylamino)-7-methoxy 1-H-benzo[d]imidazole-5-carboxylaye

Under an ice bath, ammonium hydroxide (0.5 mL) was added to a solution of 7a (285 mg, 0.36 mmol) in methanol (10 mL). After 10 minutes, an aqueous solution of sodium dithionite (311 mg, 1.8 mmol) was added and the mixture was slowly heated to react at room temperature for 2 h. Inorganic salts were filtered off, and ethyl acetate was added for extraction. The organic phase was washed with saturated brine, dried over anhydrous magnesium sulfate and spin-dried to obtain the crude product, compound **7b** (296 mg, purity 90%, yield 96%).
MS(ESI)m/z = 835.0 [M+H]+

### Step 3: synthesis of methyl (E)-7-(3-(4-(tert-butoxycarbonyl)piperazin-1-yl)propoxy)-2-(4-ethyl-2-methylthiazole-5-formylamino)-1-(4-(2)-(1-ethyl-3-methyl-1H-pyrazole-5-formylamino)-7-methoxy-5-(methoxycarbonyl)-1H-benzo[d]imidazole-1-yl)but-2-en-1-ylmethyl)-1H-benzo[d]imidazole-5-carboxylate

Under an ice bath, to a solution of **7b** (296 mg, 0.34 mmol) in DMF (3 mL) was added 4-ethyl-2-methylthiazole-5-carbonyl isothiocyanate (66 mg, 0.34 mmol). After 0.5 h of reaction, HATU (155 mg, 0.41 mmol) and DIPEA (88 mg, 0.68 mmol) were added, and then heated to room temperature for 12 h. The reaction solution was slowly poured into water, and a yellow solid precipitated out. The solid was filtered and dried to obtain **7c** (346 mg, yield 97%).
MS(ESI)m/z = 996.4 [M+H]+

### Step 4: synthesis of (E)-7-(3-(4-(tert-butoxycarbonyl)piperazin-1-yl)propoxy)-1-(4-(5-carboxy-2-(1-ethyl)-3-methyl-1H-pyrazole-5-formylamino)-7-methoxy-1H-benzo[d]imidazol-1-yl)but-2-en-1-yl)-2-(4-ethyl-2-methylthiazole-5-formylamino)-1H- -benzo[d]imidazole-5-carboxylic acid

7c (340 mg, 0.34 mmol) was dissolved in a mixed solution (5.5 ml) of methanol, tetrahydrofuran and water (volume ratio: 1/1/0.5), and lithium hydroxide (142 mg, 3.4 mmol) was added. The temperature was raised to 75°C and the reaction was stirred overnight. The organic solvent was removed by rotary evaporation, and the crude product was dissolved in water, extracted by adding ethyl acetate. The resulting aqueous phase obtained by separation was adjusted with dilute hydrochloric acid (1 M) in an ice bath until the solid did not precipitate, the solid was filtered and dried to obtain compound **7d** (220 mg, yield 67%).
MS(ESI)m/z = 968.4 [M+H]⁺

### Step 5: synthesis of tert-butyl (E)-4-(3-((5-carbamoyl-1-(4-(5-carbamoyl-2-(1-ethyl-3-methyl-1H-pyrazole- 5-formylamino)-7-tert-butyl ester methoxy-1H-benzo[d]imidazol-1-yl)but-2-en-1-yl)-2-(4-ethyl -2-methylthiazole-5-formylamino)-1H-benzo[d]imidazol-7-yl)oxy)propyl) piperazine-1-carboxylate

7d (158 mg, 0.16 mmol) was dissolved in DMF (2 mL), and HATU (182 mg, 0.48 mmol) and DIPEA (103 mg, 0.8 mmol) were added. After 0.5 h, ammonium bicarbonate (64.5 mg, 0.82 mmol) was added, and the mixture was stirred at room temperature for 2 h. The reaction solution was added with water to precipitate a solid, which was filtered, washed with pure water, and dried to obtain compound **7e** (140 mg, yield 90%).
MS(ESI)m/z = 966.5 [M+H]+

### Step 6: synthesis of (E)-N-(5-carbamoyl-1-(4-(5-carbamoyl-2-(1-ethyl-3-methyl-1H-pyrazole-5-formylamino)-7-methoxy-1H-benzo[d]imidazol-1-yl)but-2-en-1-yl)-7-(3-(piperazin-1-yl)propoxy)-1H-benzo[d]imidazol-2-yl)-4-ethyl-2-methylthiazole-5-formylamide

Under ice bath, TFA(2 mL) was added to a solution of compound 7e (140 mg, 0.14 mmol) in DCM (5 mL), the mixture was slowly warmed to room temperature, reacted for 1 h and spin-dried to remove the solvent. The free TFA was removed under reduced pressure with an oil pump to obtain compound **7f** (130 mg, 80% purity).
MS(ESI)m/z = 866.4 [M+H]

### Step 7: synthesis of (E)-N-(5-carbamoyl-1-(4-(5-carbamoyl-2-(1-ethyl-3-methyl-1H-pyrazole-5-formylamino)-7-methoxy-1H-benzo[d]imidazol-1-yl)but-2-en-1-yl)-7-(3-(4-methylpiperazin-1-yl)propoxy)-1H-benzo[d]imidazol-2-yl)-4-ethyl-2-methylthiazole-5-formylamide

30% formaldehyde solution (2 drops) was added to a solution of compound **7f** (10 mg, 0.011 mmol) in methanol (1 mL), acetic acid was added after 0.5 h to adjust the pH to about 4, then sodium borohydride (1.3 mg) was added. After 3 h of reaction, the reaction was quenched by adding water. After the reaction solution was concentrated, compound 7 (8.5 mg, yield 87%) was obtained by purification through preparative HPLC.

¹H NMR (400 MHz, DMSO-d₆ + D₂O) δ 7.59 (s, 1H), 7.58 (s, 1H), 7.31 (s, 1H), 7.26 (s, 1H), 6.46 (s, 1H), 5.87 - 5.67 (m, 2H), 4.99 - 4.78 (m, 4H), 4.53 - 4.38 (m, 2H), 4.10 (t, J = 6.1 Hz, 2H), 3.70 (s, 3H), 3.48 (s, 8H) , 3.17 (dt, J = 13.6, 5.6 Hz, 2H), 3.04 (dd, J = 15.0, 7.7 Hz, 2H), 2.86 (s, 3H), 2.07 (s, 3H), 2.06 - 1.97 (m, 2H), 1.21 (t, J = 7.1 Hz, 3H), 1.09 (t, J = 7.5 Hz, 3H).

MS(ESI) m/z = 880.4 [M+H]⁺.

### Example 8

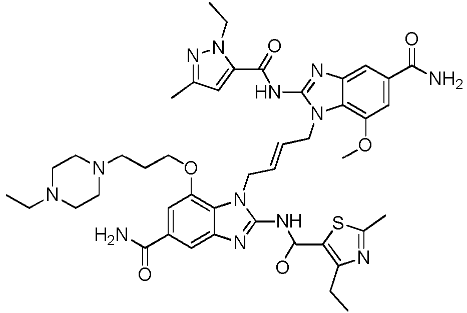

Acetaldehyde (18 mg) was added to a solution of compound 7f (60 mg, 0.07 mmol) in methanol, and acetic acid was added after 0.5 h to adjust the pH to about 4, then sodium cyanoborohydride (13 mg, 0.21 mmol) was added. After 3h of reaction, water was added to quench the reaction. After the reaction solution was concentrated, it was purified by preparative HPLC to obtain compound **8** (31 mg, yield 49.5%).

¹H NMR (400 MHz, MeOD) δ 7.67 (s, 1H), 7.63 (s, 1H), 7.38 (s, 2H), 6.76 (s, 1H), 6.07 - 5.96 (m, 1H), 5.93 - 5.79 (m, 1H), 5.19 (d, J = 5.5 Hz, 2H), 5.07 (d, J = 5.1 Hz, 2H), 4.68 (q, J = 7.1 Hz, 2H), 4.21 (t, J = 5.9 Hz, 2H), 3.87 (s, 7H), 3.64 (s, 4H), 3.51 - 3.40 (m, 3H), 3.40 - 3.34 (m, 2H), 3.29 - 3.22 (m, 2H), 2.85 (s, 3H) , 2.28 (s, 3H), 2.27 - 2.17 (m, 2H), 1.44 (t, J = 7.2 Hz, 6H), 1.33 (t, J = 7.6 Hz, 3H).

MS(ESI) m/z = 894.41 [M+H]⁺.

### Example 9

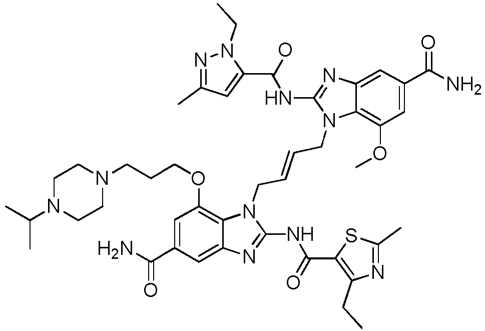

Acetone (3 mL) was added to a solution of compound 7f (50 mg, 0.058 mmol) in methanol, and the pH was adjusted to about 4 with acetic acid. The mixture was heated to 50 °C and reacted for 4 hours, and the reaction solution was cooled to 0 °C, then sodium cyanoborohydride (11 mg, 0.17 mmol) was added. The mixture was reacted overnight at room temperature, and the reaction was quenched by adding water. After the reaction solution was concentrated, it was purified by preparative HPLC to obtain compound **9** (23 mg, yield 43%).

¹H NMR (400 MHz, DMSO-d₆ + D₂O) δ 7.61 (s, 1H), 7.57 (s, 1H), 7.33 (s, 1H), 7.26 (s, 1H), 6.48 (s, 1H), 5.87 - 5.62 (m, 2H), 4.90 (s, 4H), 4.45 (q, J = 6.7 Hz, 2H), 4.13 (t, J = 5.7 Hz, 2H), 3.69 (s, 3H), 3.67 - 3.32 (m, 10H), 3.32 - 3.23 (m, 2H), 3.04 (q, J = 7.3 Hz, 2H), 2.58 (s, 3H), 2.18 - 2.09 (m, 2H), 2.07 (s, 3H), 1.29 ( d, J = 6.6 Hz, 6H), 1.21 (t, J = 7.1 Hz, 3H), 1.10 (t, J = 7.5 Hz, 3H).

MS(ESI) m/z = 908.43 [M+H]⁺.

### Example 10

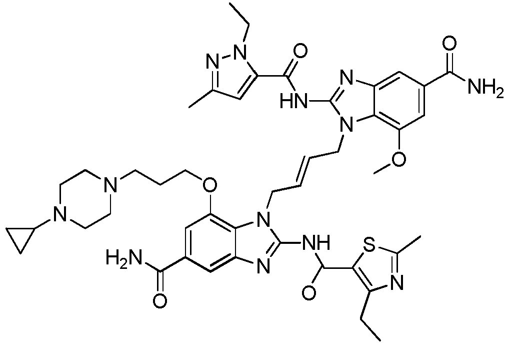

1-ethoxy-1-trimethylsiloxycyclopropane (48 mg, 0.277 mmol) was added to a solution of compound **7f** (60 mg, 0.055 mmol) in methanol, and the pH was adjusted to about 4 with acetic acid, and then sodium borohydride acetate (47 mg, 0.22 mmol) was added and heated to 50°C and reacted overnight. LCMS showed that all was converted to intermediate imine. After cooling to room temperature, sodium cyanoborohydride (10 mg, 0.16 mmol) was added. The reaction was quenched by adding water. After the reaction solution was concentrated, it was purified by preparative HPLC to obtain compound **10** (27.7 mg, yield 55%).

¹H NMR (400 MHz, DMSO-d₆+D₂O) δ 7.57 (s, 2H), 7.23 (s, 1H), 7.21 (s, 1H), 6.46 (s, 1H), 5.83 - 5.63 (m, 2H), 4.85 (d, J = 10.9 Hz, 4H), 4.45 (dd, J = 13.3, 6.5 Hz, 2H), 3.64 (s, 4H), 3.27 - 2.91 (m, 10H), 2.87 (s, 3H), 2.05 (s, 3H), 2.01 (s, 1H), 1.85 (s, 2H), 1.21 (t, J = 7.1 Hz, 3H), 1.09 (t, J = 7.5 Hz, 3H), 0.55 (d, J = 6.5 Hz, 2H), 0.43 (s, 2H).

MS(ESI) m/z = 906.41 [M+H]⁺.

### Example 11

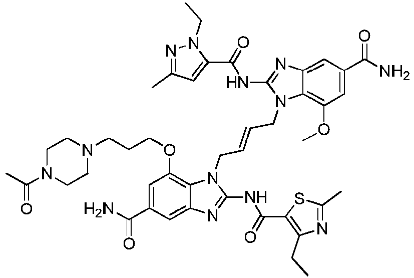

TEA (5.5 mg, 0.055 mmol) and acetic anhydride (3.3 mg, 0.033 mmol) were added to a solution of **7f** (10 mg, 0.011 mmol) in DMF (1 mL), and the mixture was reacted for 2 h at room temperature. The reaction solution was purified by preparative HPLC and compound **11** was obtained (6.5 mg, yield 62%).

¹H NMR (400 MHz, DMSO-d₆ + D₂O) δ 7.61 (d, J = 1.6 Hz, 2H), 7.27 (s, 2H), 6.48 (s, 1H), 5.84 - 5.58 (m, 2H), 4.87 ( d, J = 4.9 Hz, 4H), 4.47 (dd, J = 13.8, 6.8 Hz, 2H), 4.01 (s, 2H), 3.68 (s, 3H), 3.33 (s, 4H), 3.17 - 3.04 (m , 5H), 2.87 (s, 4H), 2.54 (s, 3H), 2.07 (s, 3H), 2.02 (s, 3H), 1.97 (s, 2H), 1.23 (t, J = 7.1 Hz, 3H) , 1.12 (t, J = 7.5 Hz, 3H).

MS(ESI) m/z = 908.39 [M+H]⁺.

According to the synthesis method of Example 7, compounds 12-23 of Examples 12-23 were also prepared.

### Example 12

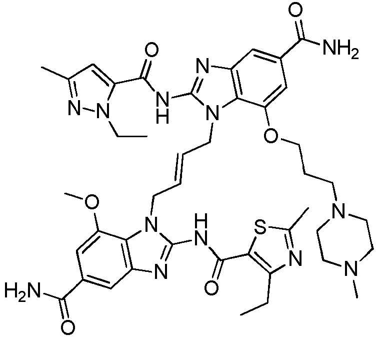

To a solution of (E)-N-(5-carbamoyl-1-(4-(5-carbamoyl-2-(1-ethyl-3-methyl-1H-pyrazole-5-formylamino)-7-(3-(piperazin-1-yl)propoxy)-1H-benzo[d]imidazol-1-yl)but-2-en-1-yl)-7-methoxy-1H-benzo[d]imidazol-2-yl)-4-ethyl-2-methylthiazole-5-formylamide (40 mg, 0.044 mmol) in methanol (2mL) was added 30% formaldehyde solution (2 drops). After 0.5 h, acetic acid was added to adjust the pH to about 4, then sodium borohydride (5.2 mg) was added, and the reaction was quenched by adding water after 3 h. After the reaction solution was concentrated, it was purified by preparative HPLC to obtain compound **12** (29 mg, yield 75%).

¹H NMR (400 MHz, DMSO) δ 12.79 (s, 1H), 11.85 (s, 2H), 8.03 (s, 1H), 7.97 (s, 1H), 7.65 (s, 1H), 7.62 (s, 1H) , 7.36 (s, 2H), 7.34 (s, 2H), 6.49 (s, 1H), 5.97 - 5.84 (m, 1H), 5.79 - 5.65 (m, 1H), 4.96 (d, J = 3.9 Hz, 2H ), 4.86 (d, J = 5.3 Hz, 2H), 4.49 (q, J = 7.0 Hz, 3H), 4.15 - 4.08 (m, 4H), 3.83 (s, 8H), 3.64 (s, 5H), 3.44 (s, 4H), 3.22 (s, 2H), 3.09 (q, J = 7.5 Hz, 2H), 2.86 (d, J = 20.8 Hz, 3H), 2.55 (s, 3H), 2.09 (s, 5H) , 1.25 (t, J = 7.1 Hz, 3H), 1.14 (t, J = 7.5 Hz, 3H).

MS(ESI) m/z = 880.4 [M+H]⁺.

### Example 13

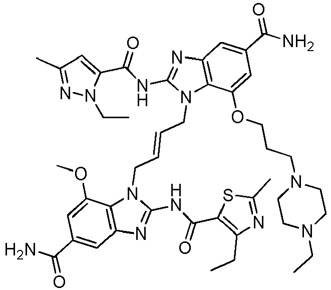

To a solution of (E)-N-(5-carbamoyl-1-(4-(5-carbamoyl-2-(1-ethyl-3-methyl-1H-pyrazole-5-formylamino)-7-(3-(piperazin-1-yl)propoxy)-1H-benzo[d]imidazol-1-yl)but-2-en-1-yl)-7-methoxy-1H-benzo[d]imidazol-2-yl)-4-ethyl-2-methylthiazole-5-formylamide (50 mg, 0.058 mmol) in methanol, acetaldehyde (18 mg) was added, after 0.5 h acetic acid was added to adjust the pH to about 4, and then sodium cyanoborohydride (13 mg, 0.21 mmol) was added. After reacting for 3 h, the reaction was quenched by adding water. After the reaction solution was concentrated, it was purified by preparative HPLC to obtain compound **13** (30 mg, yield 58%).

¹H NMR (400 MHz, DMSO) δ 12.80 (s, 1H), 11.80 (s, 1H), 8.03 (s, 1H), 7.96 (s, 1H), 7.65 (s, 1H), 7.63 (s, 1H), 7.36 (s, 3H), 7.34 (s, 1H), 6.50 (s, 1H), 5.94 - 5.89 (m, 1H), 5.78 - 5.70 (m, 1H), 4.96 (d, J = 3.8 Hz, 2H ), 4.86 (d, J = 5.3 Hz, 2H), 4.50 (q, J = 6.9 Hz, 2H), 4.09 (t, J = 5.5 Hz, 2H), 3.68 (s, 4H), 3.38 (s, 4H ), 3.20 (s, 4H), 3.09 (q, J = 7.5 Hz, 2H), 2.54 (s, 3H), 2.09 (s, 5H), 1.26 (q, J = 6.9 Hz, 6H), 1.14 (t , J = 7.5 Hz, 3H).

MS(ESI) m/z = 894.41 [M+H]⁺.

### Example 14

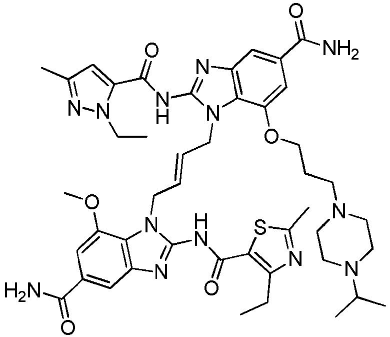

To a solution of (E)-N-(5-carbamoyl-1-(4-(5-carbamoyl-2-(1-ethyl-3-methyl-1H-pyrazole-5-formylamino)-7-(3-(piperazin-1-yl)propoxy)-1H-benzo[d]imidazol-1-yl)but-2-en-1-yl)-7-methoxy-1H-benzo[d]imidazol-2-yl)-4-ethyl-2-methylthiazole-5-formylamide (50 mg, 0.058 mmol) in methanol, acetone (3 mL) was added, and the pH was adjusted with acetic acid to about 4, the mixture was heated to 50 °C and react for 4 hours. The reaction solution was cooled to 0°C, then sodium cyanoborohydride (11 mg, 0.17 mmol) was added, reacted overnight at room temperature, and the reaction was quenched by adding water. After concentrating the reaction solution, it was purified by preparative HPLC to obtain compound **14** (23 mg, yield 43.7%).

1H NMR (400 MHz, DMSO) δ 12.80 (s, 1H), 11.86 (s, 1H), 8.03 (s, 1H), 7.96 (s, 1H), 7.65 (s, 1H), 7.63 (s, 1H) , 7.36 (s, 3H), 7.34 (s, 1H), 6.50 (s, 1H), 5.99 - 5.87 (m, 1H), 5.79 - 5.70 (m, 1H), 4.96 (d, J = 4.2 Hz, 2H), 4.86 (d, J = 5.5 Hz, 2H), 4.50 (q, J = 6.9 Hz, 2H), 4.10 (t, J = 5.5 Hz, 2H), 3.83 (s, 3H), 3.71 - 3.37 (m , 8H), 3.22 (s, 2H), 3.09 (q, J = 7.4 Hz, 2H), 2.54 (s, 3H), 2.09 (s, 5H), 1.30 (d, J = 6.5 Hz, 5H), 1.25 (t, J = 7.1 Hz, 3H), 1.14 (t, J = 7.5 Hz, 3H).

MS(ESI) m/z = 908.43 [M+H]⁺.

### Example 15

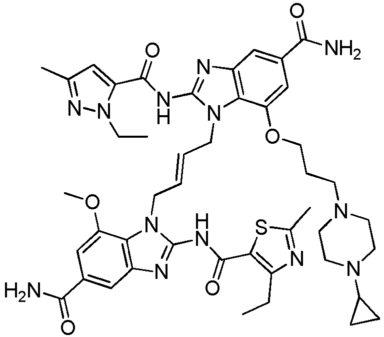

To a solution of (E)-N-(5-carbamoyl-1-(4-(5-carbamoyl-2-(1-ethyl-3-methyl-1H-pyrazole-5-formylamino)-7-(3-(piperazin-1-yl)propoxy)-1H-benzo[d]imidazol-1-yl)but-2-en-1-yl)-7-methoxy-1H-benzo[d]imidazol-2-yl)-4-ethyl-2-methylthiazole-5-formylamide (50 mg, 0.058 mmol) in methanol, 1-ethoxy-1-trimethylsilyloxycyclopropane (48 mg, 0.277 mmol) was added, the pH was adjusted to about 4 with acetic acid, then sodium borohydride acetate (47 mg, 0.22 mmol) was added, and the mixture was heated to 50°C and reacted overnight. LCMS showed that all was converted to intermediate imine. After cooling to room temperature, sodium cyanoborohydride (10 mg, 0.16 mmol) was added. The reaction was quenched by adding water. After the reaction solution was concentrated, it was purified by preparative HPLC to obtain compound **15** (9 mg, yield 17%).

1H NMR (400 MHz, DMSO) δ 12.82 (s, 1H), 8.03 (s, 1H), 7.96 (s, 1H), 7.66 (s, 1H), 7.64 (s, 1H), 7.45 - 7.26 (m, 3H), 6.52 (s, 1H), 5.98 - 5.84 (m, 1H), 5.79 - 5.68 (m, 1H), 4.95 (s, 2H), 4.85 (d, J = 5.4 Hz, 2H), 4.52 (dd, J = 14.2, 7.2 Hz, 2H), 4.03 (s, 1H), 3.79 (s, 2H), 3.66 - 3.14 (m, 6H), 3.10 (dd, J = 15.0, 7.5 Hz, 2H), 2.53 ( s, 2H), 2.11 (s, 2H), 1.99 (s, 1H), 1.27 (t, J = 7.1 Hz, 2H), 1.15 (t, J = 7.5 Hz, 2H), 0.99 (s, 2H), 0.74 (s, 2H).

MS (ESI) m/z = 906.41 [M+H]⁺.

### Example 16

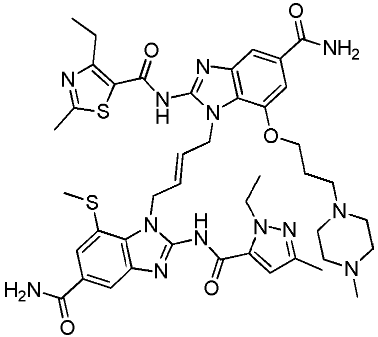

To a solution of compound (E)-N-(5-carbamoyl-1-(4-(5-carbamoyl-2-(1-ethyl-3-methyl-1H-pyrazole-5-formylamino)-7-(methylthio)-1H-benzo[d]imidazol-1-yl)but-2-en-1-yl)-7-(3-(piperazin-1-yl)propoxy)-1H-benzo[d]imidazol-2-yl)-4-ethyl-2-methylthiazole-5-formylamide (20 mg, 0.023 mmol) in methanol, 30% formaldehyde solution (2 drops) was added, after 0.5 h, acetic acid was added to adjust the pH to about 4, then sodium borohydride (2.6 mg, 0.069 mmol) was added, and the reaction was quenched by adding water after 3 h. After the reaction solution was concentrated, it was purified by preparative HPLC to obtain compound **16** (10 mg, yield 48%).

MS(ESI) m/z = 896.37 [M+H]⁺.

### Example 17

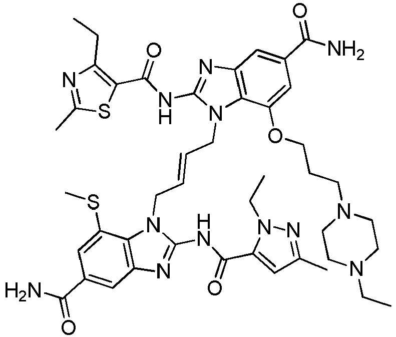

To a solution of (E)-N-(5-carbamoyl-1-(4-(5-carbamoyl-2-(1-ethyl-3-methyl-1H-pyrazole-5-formylamino)-7-(methylthio)-1H-benzo[d]imidazol-1-yl)but-2-en-1-yl)-7-(3-(piperazin-1-yl)propoxy)-1H-benzo[d]imidazol-2-yl)-4-ethyl-2-methylthiazole-5-formylamide (60 mg, 0.07 mmol) in methanol, acetaldehyde (18 mg) was added. After 0.5 h, acetic acid was added to adjust the pH to about 4, and then sodium cyanoborohydride (13 mg, 0.21 mmol) was added. After reacting for 3 h, the reaction was quenched by adding water. After concentrating the reaction solution, it was purified by preparative HPLC to obtain compound **17** (26 mg, yield 41%).

1H NMR (400 MHz, DMSO) δ 12.80 (s, 1H), 11.77 (s, 1H), 8.06 (s, 1H), 7.98 (s, 1H), 7.85 (s, 1H), 7.63 (s, 2H) , 7.40 (s, 1H), 7.36 (s, 2H), 6.51 (s, 1H), 5.92 - 5.79 (m, 1H), 5.79 - 5.68 (m, 1H), 5.15 (d, J = 2.6 Hz, 2H), 4.91 (d, J = 3.9 Hz, 2H), 4.51 (dd, J = 14.1, 7.0 Hz, 4H), 4.14 (t, J = 5.3 Hz, 2H), 3.70 (s, 4H), 3.39 (s , 5H), 3.20 (s, 4H), 3.09 (q, J = 7.4 Hz, 2H), 2.53 (s, 3H), 2.45 (s, 3H), 2.13 (s, 2H), 2.09 (s, 3H) , 1.33 - 1.19 (m, 7H), 1.14 (t, J = 7.5 Hz, 3H).

MS(ESI) m/z = 910.39 [M+H]⁺.

### Example 18

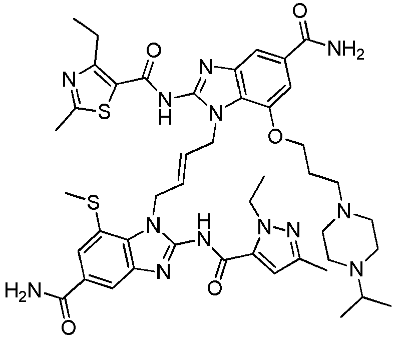

To a solution of ((E)-N-(5-carbamoyl-1-(4-(5-carbamoyl-2-(1-ethyl-3-methyl-1H-pyrazole-5-formylamino)-7-(methylthio)-1H-benzo[d]imidazol-1-yl)but-2-en-1-yl)-7-(3-(piperazin-1-yl)propoxy)-1H-benzo[d]imidazol-2-yl)-4-ethyl-2-methylthiazole-5-formylamide (60 mg, 0.07 mmol) in methanol, acetone (3 mL) was added, and then the pH was adjusted to about 4 with acetic acid, the mixture was heated to 50 °C and reacted for 4 hours. The reaction solution was cooled to 0 °C, then sodium cyanoborohydride (11 mg, 0.17 mmol) was added. The mixture was reacted overnight at room temperature, and water was added to quench the reaction. The reaction solution was concentrated, then it was purified by preparative HPLC to obtain compound 18 (17 mg, 26%).

1H NMR (400 MHz, DMSO) δ 12.81 (s, 1H), 12.12 (s, 1H), 11.84 (s, 1H), 8.06 (s, 1H), 7.98 (s, 1H), 7.85 (s, 1H) , 7.63 (s, 2H), 7.41 (s, 1H), 7.37 (s, 2H), 6.52 (s, 1H), 5.91 - 5.67 (m, 2H), 5.15 (d, J = 2.5 Hz,2H), 4.91 (d, J = 3.5 Hz, 2H), 4.51 (q, J = 7.0 Hz, 2H), 4.16 (t, J = 5.6 Hz, 2H), 3.81 - 3.39 (m, 10H), 3.24 (s, 2H ), 3.09 (q, J = 7.4 Hz, 2H), 2.54 (s, 3H), 2.45 (s, 4H), 2.14 (s, 2H), 2.09 (s, 3H), 1.30 (d, J = 6.5 Hz, 6H), 1.26 (t, J = 7.1 Hz, 3H), 1.14 (t, J = 7.5 Hz, 3H).

MS(ESI) m/z = 924.40 [M+H]⁺.

### Example 19

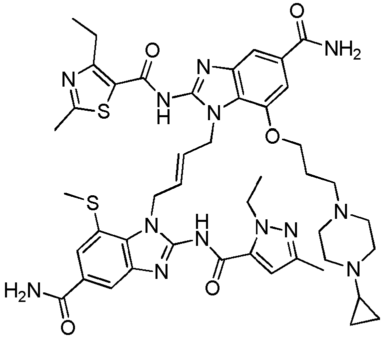

To a solution of (E)-N-(5-carbamoyl-1-(4-(5-carbamoyl-2-(1-ethyl-3-methyl-1H-pyrazole-5-formylamino)-7-(methylthio)-1H-benzo[d]imidazol-1-yl)but-2-en-1-yl)-7-(3-(piperazin-1-yl)propoxy)-1H-benzo[d]imidazol-2-yl)-4-ethyl-2-methylthiazole-5-formylamide (60 mg, 0.07 mmol) in methanol, 1-ethoxy-1-trimethylsiloxycyclopropane (48 mg, 0.277 mmol) was added, the pH was adjusted to about 4 with acetic acid, then sodium borohydride acetate (47 mg, 0.22 mmol) was added, the mixture was heated to 50°C and reacted overnight. LCMS showed that all was converted to intermediate imine. The mixture was cooled to room temperature and sodium cyanoborohydride (10 mg, 0.16 mmol) was added. The reaction was quenched by adding water. After the reaction solution was concentrated, it was purified by preparative HPLC to obtain compound **19** (16 mg, yield 25%).

MS(ESI) m/z = 922.39 [M+H]⁺.

### Example 20

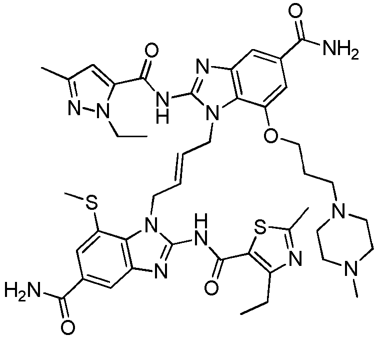

To a solution of (E)-N-(5-carbamoyl-1-(4-(5-carbamoyl-2-(1-ethyl-3-methyl-1H-pyrazole-5-formylamino)-7-(3-(piperazin-1-yl)propoxy)-1H-benzo[d]imidazol-1-yl)but-2-en-1-yl)-7-(methylthio)-1H-benzo[d]imidazol-2-yl)-4-ethyl-2-methylthiazole-5-formylamide (10 mg, 0.011 mmol) in methanol, 30% formaldehyde solution (2 drops) was added. After 0.5 h, acetic acid was added to adjust the pH to about 4, then sodium borohydride (1.3 mg, 0.033 mmol) was added, and the reaction was quenched with water after 3 h. After the reaction solution was concentrated, it was purified by preparative HPLC to obtain compound **20** (5 mg, yield 51%).

MS(ESI) m/z = 896.37 [M+H]⁺.

### Example 21

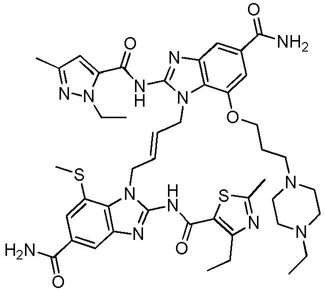

To a solution of (E)-N-(5-carbamoyl-1-(4-(5-carbamoyl-2-(1-ethyl-3-methyl-1H-pyrazole-5-formylamino)-7-(3-(piperazin-1-yl)propoxy)-1H-benzo[d]imidazol-1-yl)but-2-en-1-yl)-7-(methylthio)-1H-benzo[d]imidazol-2-yl)-4-ethyl-2-methylthiazole-5-formylamide (60 mg, 0.07 mmol) in methanol, acetaldehyde (18 mg) was added. After 0.5 h, acetic acid was added to adjust the pH to about 4, and then sodium cyanoborohydride (13 mg, 0.21 mmol) was added. After reacting for 3 h, the reaction was quenched by adding water. After concentrating the reaction solution, it was purified by preparative HPLC to obtain compound **21** (8 mg, yield 12%).

1H NMR (400M, MeOD-d₄) δ 7.83-7.90 (m, 1H), 7.74-7.79(m, 1H), 7.68-7.74(m, 1H), 7.34-7.45(m, 1H), 6.75-6.83( m, 1H), 5.82-6.02(m, 2H), 5.18-5.34(m, 4H), 4.67-4.79(m, 2H), 4.17-4.28(m, 2H), 3.54-3.99(m, 8H), 3.36-3.47(m, 4H), 3.26-3.30(m, 2H), 2.89(s, 3H), 2.58(s, 3H), 2.32(s, 3H), 2.17-2.27(m, 2H), 1.42- 1.53(m, 6H), 1.32-1.39(m, 3H).

MS(ESI) m/z = 910.39 [M+H]⁺.

### Example 22

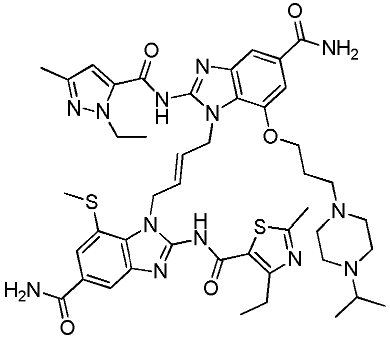

To a solution of (E)-N-(5-carbamoyl-1-(4-(5-carbamoyl-2-(1-ethyl-3-methyl-1H-pyrazole-5-formylamino)-7-(3-(piperazin-1-yl)propoxy)-1H-benzo[d]imidazol-1-yl)but-2-en-1-yl)-7-(methylthio)-1H-benzo[d]imidazol-2-yl)-4-ethyl-2-methylthiazole-5-formylamide (60 mg, 0.07 mmol) in methanol, acetone (3 mL) was added, and then the pH was adjusted with acetic acid to about 4, the mixture was heated to 50 °C to react for 4 hours, the reaction solution was cooled to 0 °C, then sodium cyanoborohydride (11 mg, 0.17 mmol) was added, the mixture was reacted overnight at room temperature, and water was added to quench the reaction. After the reaction solution was concentrated, it was purified by preparative HPLC to obtain compound **22** (17 mg, 26%).

1H NMR (400M, MeOD-d₄) δ 7.85-7.90 (m, 1H), 7.74-7.79(m, 1H), 7.70-7.74(m, 1H), 7.37-7.41(m, 1H), 6.78-6.83( m, 1H), 5.82-6.01(m, 2H), 5.18-5.34(m, 4H), 4.68-4.78(m, 2H), 4.19-4.26(m, 2H), 3.66-3.87(m, 8H), 3.40-3.47(m, 2H), 3.28-3.32(m, 2H), 2.89(s, 3H), 2.58(s, 3H), 2.32(s, 3H), 2.18-2.28(m, 2H), 1.44- 1.53(m, 9H), 1.31-1.40(m, 3H).

MS(ESI) m/z = 924.40 [M+H]⁺.

### Example 23

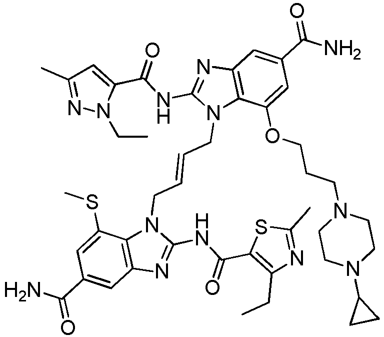

To a solution of (E)-N-(5-carbamoyl-1-(4-(5-carbamoyl-2-(1-ethyl-3-methyl-1H-pyrazole-5-formylamino)-7-(3-(piperazin-1-yl)propoxy)-1H-benzo[d]imidazol-1-yl)but-2-en-1-yl)-7-(methylthio)-1H-benzo[d]imidazol-2-yl)-4-ethyl-2-methylthiazole-5-formylamide (40 mg, 0.045 mmol) in methanol, 1-ethoxy-1-trimethylsiloxycyclopropane (39 mg, 0.225 mmol) was added, the pH was adjusted to about 4 with acetic acid, then sodium acetate borohydride (47 mg, 0.22 mmol) was added, the mixture was heated to 50°C and reacted overnight, then water was added to quench the reaction. After the reaction solution was concentrated, it was purified by preparative HPLC to obtain compound **23** (10 mg, yield 24%).

MS(ESI) m/z = 922.39 [M+H]⁺.

To a solution of (E)-N-(5-carbamoyl-1-(4-(5-carbamoyl-2-(1-ethyl-3-methyl-1H-pyrazole-5-formylamino)-7-methoxy-1H-benzo[d]imidazol-1-yl)but-2-en-1-yl)-7-(3-(piperazin-1-yl)propoxy)-1H-benzo[d]imidazol-2-yl)-4-ethyl-2-methylthiazole-5-formylamide in DMS, TEA and the corresponding acid chloride was added, the mixture was reacted at room temperature for 2h. The reaction solution was purified by preparative HPLC, and Compounds **24, 25, 26, 27, 28** were obtained. The compound structures and characterization are shown in Table 1 below:

**Table 1 Structures and characterization of some example compounds**

| **Example** | **Structure of the compound** | **corresponding acyl chloride** | **MS(ESI)m/z** | **¹H NMR** |
|---|---|---|---|---|
| 24 | 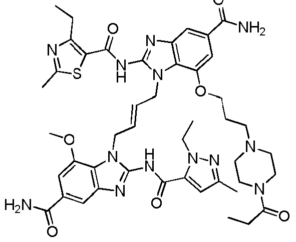 | 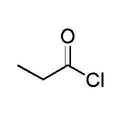 | 922.42 [M+H]⁺ | - |
| 25 | 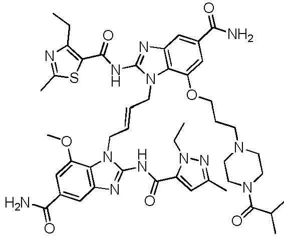 | 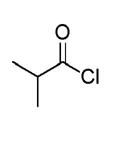 | 936.42 [M+H]⁺ | - |
| 26 | 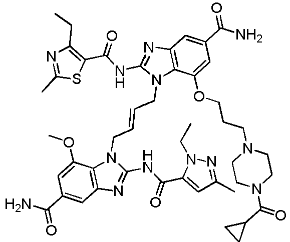 | 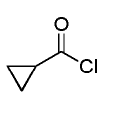 | 934.41 [M+H]⁺ | - |
| 27 | 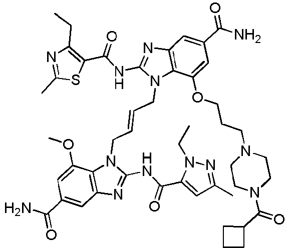 | 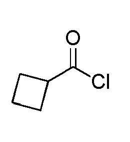 | 948.42 [M+H]⁺ | - |
| 28 | 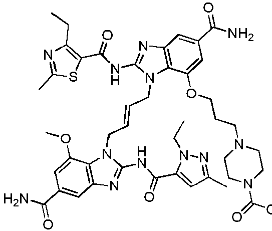 | 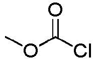 | 924.39 [M+H]⁺ | (400 MHz, DMSO) δ 12.85 (s, 1H), 9.87 (s, 1H), 7.99 (s, 1H), 7.94 (s, 1H), 7.67 (s, 1H), 7.65 (s, 1H), 7.38 (d, *J* = 10.0 Hz, 2H), 7.32 (s, 1H), 7.25 (s, 1H), 6.54 (s, 1H), 5.92 - 5.79 (m, 2H), 5.76 - 5.66 (m, 2H), 4.91 (d, *J* = 2.7 Hz, 2H), 4.84 (d, *J* = 4.5 Hz, 2H), 4.55 (dd, *J* = 13.8, 6.8 Hz, 2H), 4.01 (s, 1H), 3.93 (s, 2H), 3.71 (s, 3H), 3.64 (s, 3H), 3.29 (s, 2H), 3.20 - 3.01 (m, 5H), 2.92 (s, 2H), 2.13 (s, 3H), 1.84 (s, 2H), 1.30 (t, *J =* 7.1 Hz, 3H), 1.16 (t, *J* = 7.5 Hz, 3H). |

To a solution of (E)-N-(5-carbamoyl-1-(4-(5-carbamoyl-2-(1-ethyl-3-methyl-1H-pyrazole-5-formylamino)-7-(3-(piperazin-1-yl)propoxy)-1H-benzo[d]imidazol-1-yl)but-2-en-1-yl)-7-methoxy-1H-benzo[d]imidazol-2-yl)-4-ethyl-2-methylthiazole-5-formylamide in DMF, TEA and the corresponding acid chloride were added, the mixture was reacted at room temperature for 2h, and the reaction solution was purified by preparative HPLC. Compounds **29, 30, 31, 32, 33,** and 34 were obtained. The compound structures and characterization are shown in Table 2 below:

**Table 2 Structures and characterization of some example compounds**

| **Example** | **Structure of the compound** | **corresponding acyl chloride** | **MS(ESI) m/z** | **¹H NMR** |
|---|---|---|---|---|
| 29 | 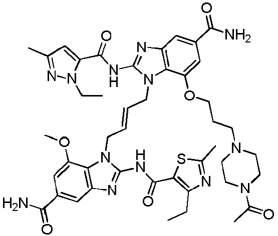 | 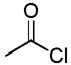 | 908.39 [M+H]⁺ | (400 MHz, DMSO) δ 12.86 (s, 1H), 11.18 (s, 1H), 8.04 (s, 1H), 7.96 (s, 1H), 7.66 (d, *J* = 5.1 Hz, 2H), 7.39 (s, 1H), 7.36 (s, 2H), 7.28 (s, 1H), 6.54 (s, 1H), 5.94 - 5.80 (m, 2H), 5.75 - 5.67 (m, 2H), 4.92 (s, 2H), 4.85 (d, *J* = 4.9 Hz, 2H), 4.54 (q, *J* = 7.0 Hz, 2H), 4.39 (d, *J* = 13.7 Hz, 1H), 3.98 (s, 2H), 3.90 (d, *J* = 14.6 Hz, 1H), 3.78 (s, 3H), 3.50 (t, *J* = 12.9 Hz, 1H), 3.26 (d, *J =* 9.9 Hz, 2H), 3.16 - 3.03 (m, 4H), 2.99 (t, *J* = 12.9 Hz, 1H), 2.93 - 2.77 (m, 2H), 2.53 (s, 3H), 2.12 (s, 3H), 2.02 (s, 3H), 1.95 (s, 2H), 1.29 (t, *J* = 7.0 Hz, 3H), 1.16 (t, *J* = 7.4 Hz, 3H). |
| 30 | 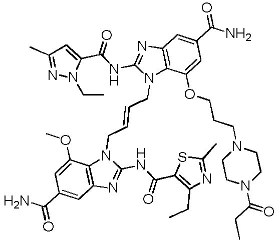 | 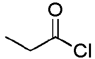 | 922.41 [M+H]⁺ | (400 MHz, DMSO) δ 12.84 (s, 1H), 9.76 (s, 1H), 8.00 (s, 1H), 7.94 (s, 1H), 7.66 (d, *J =* 6.0 Hz, 2H), 7.38 (d, *J =* 12.6 Hz, 2H), 7.33 (s, 1H), 7.25 (s, 1H), 6.54 (s, 1H), 5.93 - 5.77 (m, 1H), 5.77 - 5.59 (m, 1H), 4.90 (s, 2H), 4.84 (d, *J* = 4.6 Hz, 2H), 4.55 (q, *J* = 7.1 Hz, 2H), 4.43 (s, 1H), 3.93 (s, 3H), 3.73 (s, 3H), 3.11 (dd, *J =* 14.9, 7.5 Hz, 5H), 2.86 (s, 4H), 2.37 - 2.23 (m, 3H), 2.12 (s, 3H), 1.84 (s, 2H), 1.30 (t, *J* = 7.1 Hz, 3H), 1.16 (t, *J =* 7.5 Hz, 3H), 0.99 (t, *J =* 7.3 Hz, 3H). |
| 31 | 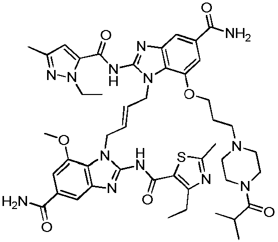 | 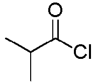 | 936.42 [M+H]⁺ | - |
| 32 | 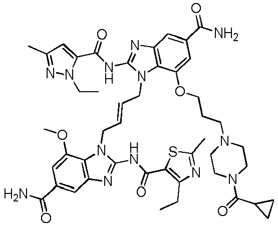 | 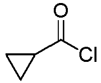 | 934.41 [M+H]⁺ | - |
| 33 | 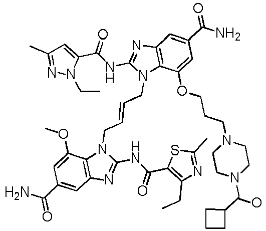 | 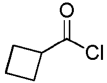 | 948.42 [M+H]⁺ | - |
| 34 | 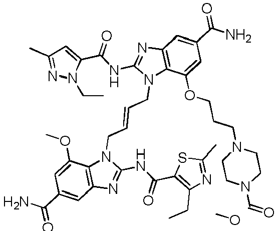 | 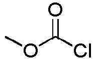 | 924.39 [M+H]⁺ | - |

To a solution of (E)-N-(5-carbamoyl-1-(4-(5-carbamoyl-2-(1-ethyl-3-methyl-1H-pyrazole-5-formylamino)-7-(methylthio)-1H-benzo[d]imidazol-1-yl)but-2-en-1-yl)-7-(3-(piperazin-1-yl)propoxy)-1H-benzo[d]imidazol-2-yl)-4-ethyl-2-methylthiazole-5-formylamide in DMF, TEA and the corresponding acid chloride were added, and the reaction was performed for 2h at room temperature. The reaction solution was purified by preparative HPLC. Compounds **35, 36, 37, 38, 39, 40** were obtained. The compound structures and characterization are shown in Table 3 below:

**Table 3 Structures and characterization of compounds in some examples**

| **Example** | **Structure of the compound** | **correspon ding acyl chloride** | **MS(ESI) m/z** | **¹H NMR** |
|---|---|---|---|---|
| 35 | 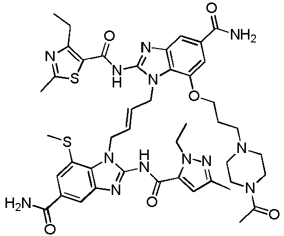 | 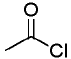 | 924.37 [M+H]⁺ | (400 MHz, DMSO) δ 12.82 (s, 1H), 11.04 (s, 1H), 8.08 (s, 1H), 7.97 (s, 1H), 7.87 (s, 1H), 7.63 (d, *J =* 4.8 Hz, 2H), 7.42 (s, 1H), 7.36 (s, 1H), 7.32 (s, 1H), 6.51 (s, 1H), 5.80 - 5.65 (m, 2H), 5.13 (s, 2H), 4.87 (s, 2H), 4.51 (q, *J =* 7.1 Hz, 2H), 4.41 (d, *J* = 12.9 Hz, 1H), 4.04 (s, 2H), 3.93 (d, *J* = 13.7 Hz, 1H), 3.50 (t, *J =* 13.1 Hz, 1H), 3.32 (d, *J =* 11.5 Hz, 2H), 3.20 - 3.05 (m, 4H), 3.00 (t*, J* = 12.6 Hz, 1H), 2.94 - 2.76 (m, 2H), 2.55 (s, 3H), 2.45 (s, 3H), 2.08 (s, 3H), 2.03 (s, 5H), 1.27 (t, *J =* 7.1 Hz, 3H), 1.16 (t, *J =* 7.5 Hz, 3H). |
| 36 | 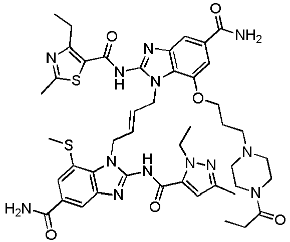 | 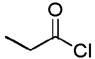 | 938.38 [M+H]⁺ | - |
| 37 | 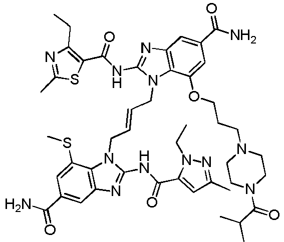 | 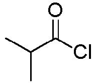 | 952.40 [M+H]⁺ | - |
| 38 | 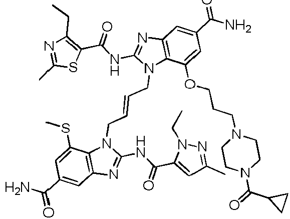 | 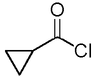 | 950.38 [M+H]⁺ | - |
| 39 | 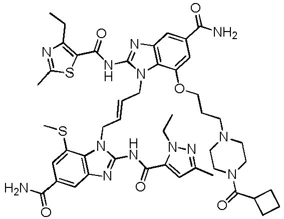 | 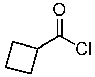 | 964.40 [M+H]⁺ | - |
| 40 | 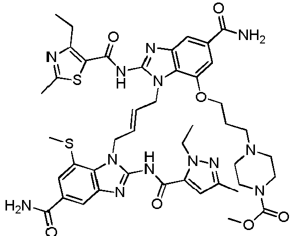 | 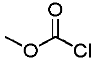 | 940.36 [M+H]⁺ | (400M, DMSO-d6) δ 7.80-7.85(m, 1H), 7.55-7.63(m, 2H), 7.22-7.28(m, 1H), 6.45-6.51(m, 1H), 5.61-5.79(m, 2H), 5.07-5.15(m, 2H), 4.81-4.88(m, 2H), 4.43-4.53(m, 2H), 3.96-4.04(m, 3H), 3.63(s, 3H), 3.22-3.38(m, 2H), 3.04-3.16(m, 6H), 2.86-2.99(m, 2H), 2.39(s, 3H), 2.07(s, 3H), 1.88-1.98(m, 2H), 1.24(t, J = 7.16 Hz, 3H), 1.11(t, J = 7.48 Hz, 3H). |

To a solution of (E)-N-(5-carbamoyl-1-(4-(5-carbamoyl-2-(1-ethyl-3-methyl-1H-pyrazole-5-formylamino)-7-(3-(piperazin-1-yl)propoxy)-1H-benzo[d]imidazol-1-yl)but-2-en-1-yl)-7-(methylthio)-1H-benzo[d]imidazol-2-yl)-4-ethyl-2-methylthiazole-5-formylamide in DMF, TEA and the corresponding acid chloride were added, and the reaction was performed for 2h at room temperature. The reaction solution was purified by preparative HPLC to obtain compounds **41, 42, 43, 44, 45, 46.** The compound structures and characterization are shown in Table 4 below:

**Table 4 Structures and characterization of some example compounds**

| **Exa mple** | **Structure of the compound** | **correspond ing acyl chloride** | **MS(ESI) m/z** | **¹H NMR** |
|---|---|---|---|---|
| 41 | 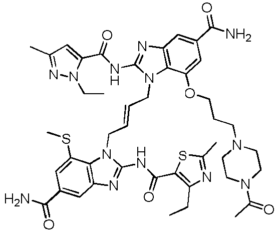 | 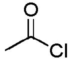 | 924.37 [M+H]⁺ | (400M, MeOD-d4) δ 7.82-7.89 (m, 1H), 7.68-7.76(m, 2H), 7.34-7.46(m, 1H), 6.77-6.89(m, 1H), 5.83-6.01(m, 2H), 5.17-5.33(m, 4H), 4.70-4.80(m, 2H), 4.61-4.69(m, 1H), 4.14-4.25(m, 2H), 3.63-3.77(m, 2H), 3.41-3.61(m, 2H), 3.25-3.32(m, 4H), 3.12-3.34(m, 2H), 2.94-3.04(m, 1H), 2.90(s, 3H), 2.56(s, 3H), 2.30-2.38(m, 1H), 2.20(s, 3H), 1.43-1.55(m, 3H), 1.29-1.41(m, 3H). |
| 42 | 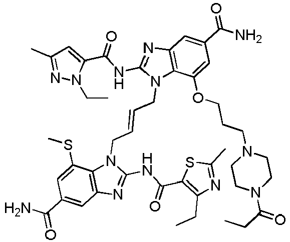 | 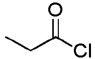 | 938.38 [M+H]⁺ | - |
| 43 | 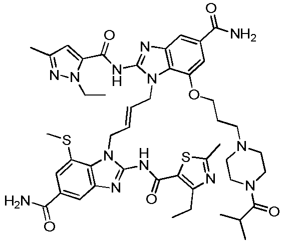 | 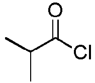 | 951.40 [M+H]⁺ | - |
| 44 | 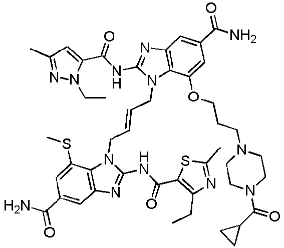 | 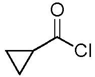 | 950.38 [M+H]⁺ | - |
| 45 | 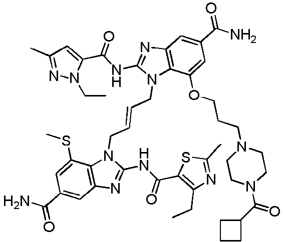 | 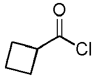 | 964.40 [M+H]⁺ | - |
| 46 | 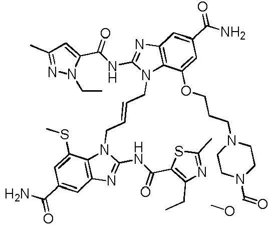 | 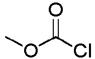 | 940.36 [M+H]⁺ | - |

### Example 47

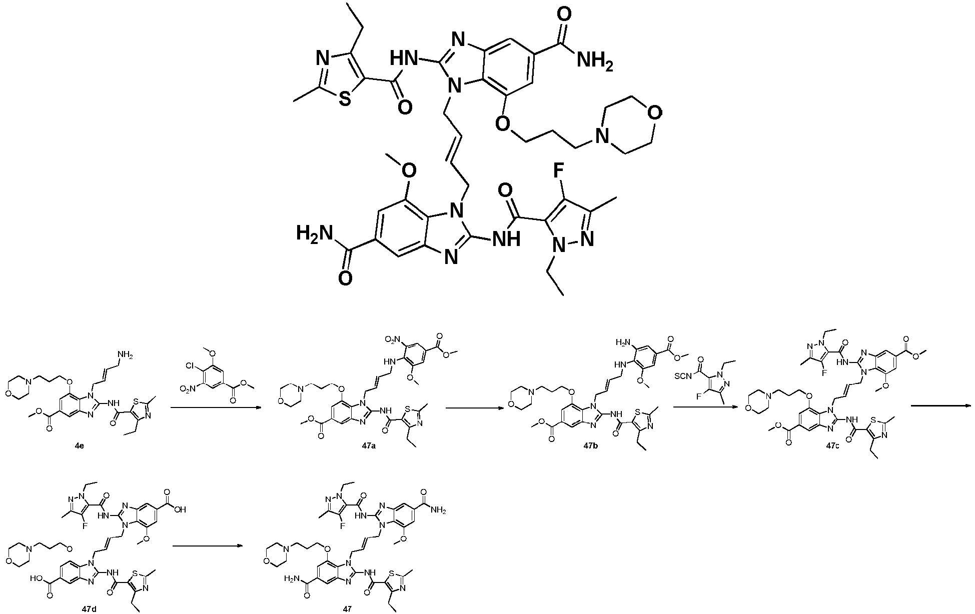

### Step 1: Synthesis of methyl (E)-methyl 2-(4-ethyl-2-methylthiazole-5-formylamino)-1-(4-(((2-methoxy-4-(methoxycarbonyl))-6-nitrophenyl)amino)but-2-en-1-yl)-7-(3-morpholinopropoxy)-1H-benzo[d]imidazole-5-carboxylate

4e (2.1 g, 3.8 mmol) was dispersed in n-butanol (20 mL), and methyl 4-chloro-3-(3-morpholinopropoxy)-5-nitrobenzoate (0.93 g, 3.8 mmol) and DIPEA (2.5 g, 19 mmol) were added. The reaction solution was heated to 120°C and stirred for 18 h. The crude product obtained by distillation under reduced pressure was dissolved in ethyl acetate, washed with water and saturated brine respectively, dried over anhydrous magnesium sulfate and spin-dried, and purified by silica gel column (eluent: petroleum ether/ethyl acetate = 1/2) to obtain 47a (2.0 g, yield 70%).
MS(ESI)m/z = 766.2[M+H]

### Step 2: synthesis of methyl (E)-methyl 1-(4-(((2-amino-6-methoxy-4-(methoxycarbonyl)phenyl)amino)but-2-en-1-yl)-2-(4-ethyl-2-methylthiazole-5-carboxyamino)-7-(3-morpholinopropoxy)-1H-benzo[d]imidazole-5-carboxylate

Under an ice bath, ammonium hydroxide (1 mL) was added to a solution of 47a (600 mg, 0.78 mmol) in methanol (5 mL). After 10 minutes, an aqueous solution of sodium dithionite (679 mg, 3.9 mmol) was added and the mixture was slowly heated to react at room temperature for 2 h. Inorganic salts were removed by filtration, and ethyl acetate was added for extraction. The organic phase was washed with saturated brine, dried over anhydrous magnesium sulfate and spin-dried to obtain compound **47b** (459 mg, yield 80%).
MS(ESI)m/z = 736.3[M+H]

### Step 3: synthesis of methyl (E)-methyl 2-(4-ethyl-2-methylthiazole-5-formylamino)-1-(4-(2-(1-ethyl-4-fluoro-3-methyl-1H-pyrazole-5-formylamino)-7-methoxy-5-(methoxycarbonyl)-1H-benzo[d]imidazol-1-yl)but-2-ene-1-yl)-7-(3-morpholinopropoxy)-1H-benzo[d]imidazole-5-carboxylate

Under an ice bath, 1-ethyl-4-fluoro-3-methyl-1H-pyrazole-5-carbonyl isothiocyanate (58 mg, 0.27 mmol) was added to a solution of 47b (200 mg, 0.27 mmol) in DMF (5 mL). After reacting for 0.5 h, DIPEA (70 mg, 0.54 mmol) and HATU (102 mg, 0.27 mmol) were added, and then the mixture was heated to room temperature for 12 h. The reaction solution was slowly poured into water, and a yellow solid precipitated out. The solid was filtered and dried to obtain **47c** (185 mg, yield 75%).
MS(ESI)m/z = 915.3[M+H]

### Step 4: synthesis of (E)-1-(4-(5-carboxy-2-(1-ethyl-4-fluoro-3-methyl-1H-pyrazole-5-carboxamino)-7-methoxy-1H-benzo[d]imidazol-1-yl)but-2-en-1-yl)-2-(4-ethyl-2-methylthiazole-5-formylamino)-7-(3-morpholinopropoxy)-1H-benzo[d]imidazole-5-carboxylic acid

47c (185 mg, 0.2 mmol) was dissolved in a mixed solution (5 mL) of methanol, tetrahydrofuran and water (volume ratio: 1/1/1), and lithium hydroxide (42 mg, 1.0 mmol) was added. The temperature was raised to 75°C and the reaction was stirred overnight. The organic solvent was removed by rotatory evaporation, the mixture was cooled to room temperature, adjusted with dilute hydrochloric acid (1M) under an ice bath until no solid precipitated, the solid was filtered and dried to obtain compound **47d** (141 mg, yield 80%).
MS(ESI)m/z = 887.3[M+H]

### Step 5: synthesis of (E)-N-(5-carbamoyl-1-(4-(5-carbamoyl-2-(1-ethyl-4-fluoro-3-methyl-1H-pyrazole-5-formylamino)-7-methoxy-1H)-benzo[d]imidazol-1-yl)but-2-en-1-yl)-7-(3-morpholinopropoxy)-1H-benzo[d]imidazol-2-yl)-4-ethyl-2-methylthiazole-5-formylamide

47d (141 mg, 0.16 mmol) was dissolved in DMF (5 mL), and HATU (152 mg, 0.4 mmol) and DIPEA (82 mg, 0.64 mmol) were added. After 0.5 h, ammonium bicarbonate (38 mg, 0.48 mmol) was added, and the mixture was stirred at room temperature for 2h. The reaction solution was concentrated and purified by preparative HPLC to obtain compound **47** (30 mg, yield 43%).
MS(ESI)m/z = 885.4[M+H]
¹H NMR (400M, DMSO-d₆) δ 12.81-12.98(m, 2H), 9.69-9.77(m, 1H), 7.93-8.02 (m, 2H), 7.61-7.69(m, 2H), 7.37-7.43(m, 2H), 7.26-7.34(m, 2H), 5.71-5.90(m, 4H), 4.80-4.94(m, 4H), 4.46-4.54(m, 2H), 3.90-4.00(m, 4H), 3.75(s, 3H), 3.58-3.64(m, 4H), 3.21-3.26(m, 2H), 3.05-3.20(m, 6H), 2.93-3.00(m, 2H), 2.64-2.70(m, 2H) ), 2.31-2.36(m, 2H), 2.12(s, 3H), 1.80-1.94(m, 4H), 1.29(t, J= 7.04 Hz, 3H), 1.16(t, J= 7.48 Hz, 3H).

### Example 48

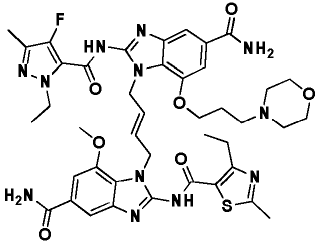

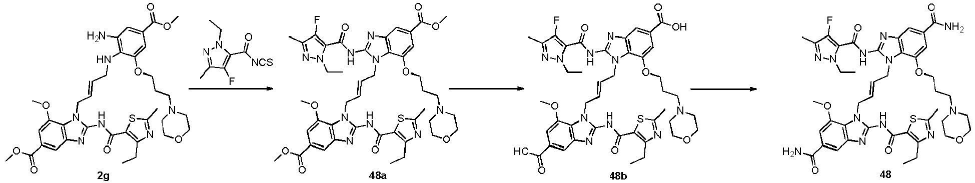

### Step 1: synthesis of methyl (E)-methyl 2-(4-ethyl-2-methylthiazole-5-formylamino)-1-(4-(2-(1-ethyl-4-fluoro-3-methyl-1H-pyrazole-5-formylamino)-5-(methoxycarbonyl)-7-(3-morpholinopropoxy)-1H-benzo[d]imidazol-1-yl)-but-2-en-1-yl)-7-methoxy-1H-benzo[d]imidazole-5-carboxylate

Under an ice bath, 1-ethyl-4-fluoro-3-methyl-1H-pyrazole-5-carbonyl isothiocyanate (0.15 g, 0.69 mmol) was added to a solution of **2g** (0.51 g, 0.69 mmol) in DMF (5 mL). After reacting for 0.5 h, DIPEA (0.27 g, 2.07 mmol) and HATU (0.32 g, 0.83 mmol) were added, and then the mixture was heated to room temperature to react for 12 h. The reaction solution was slowly poured into water, and a yellow solid precipitated out. The solid was filtered and dried to obtain 48a (0.48 g, yield 77%).
MS(ESI)m/z = 915.3[M+H]

### Step 2: synthesis of (E)-1-(4-(5-carboxy-2-(1-ethyl-4-fluoro-3-methyl-1H-pyrazole-5-carboxamino)-7-(3-morpholinopropoxy)-1H-benzo[d]imidazol-1-yl)but-2-en-1-yl)-2-(4-ethyl-2-methylthiazole-5-methylamido)-7-methoxy-1H-benzo[d]imidazole-5-carboxylic acid

48a (0.48 g, 0.52 mmol) was dissolved in a mixed solution (20 mL) of methanol, tetrahydrofuran and water (volume ratio: 1/1/1), and hydrated lithium hydroxide (0.22 g, 5.2 mmoL) was added. The temperature was raised to 75°C and the reaction was stirred overnight. The organic solvent was removed by rotary evaporation, the mixture was cooled to room temperature, adjusted with dilute hydrochloric acid (1M) under an ice bath until no solid precipitated. The solid was filtered and dried to obtain compound **48b** (0.45 g, yield 97%).
MS(ESI)m/z = 886.3[M+H]

### Step 3: synthesis of (E)-N-(5-carbamoyl-1-(4-(5-carbamoyl-2-(1-ethyl-4-fluoro-3-methyl-1H-pyrazole-5-formylamino))-7-(3-morpholinopropoxy)-1H-benzo[d]imidazol-1-yl)but-2-en-1-yl)-7-methoxy-1H-benzo[d]imidazol-2-yl)-4-ethyl-2-methylthiazole-5-formylamide

48b (0.45 g, 0.51 mmol) was dissolved in DMF (5 mL), and HATU (0.46 g, 1.22 mmol) and DIPEA (0.33 g, 2.55 mmol) were added. After 0.5h, ammonium bicarbonate (0.2 g, 2.55 mmol) was added, and the mixture was stirred at room temperature for 2h. The reaction solution was concentrated and purified by preparative HPLC to obtain compound **48** (0.26 g, yield 57%).
MS(ESI)m/z = 884.4[M+H]
¹H NMR (400M, DMSO-d₆) δ 12.82-12.97(m, 2H), 9.55-9.85(m, 1H), 7.92-8.02 (m, 2H), 7.61-7.67(m, 2H), 7.35-7.45(m, 2H), 7.28-7.35(m, 3H), 5.77-5.89(m, 2H), 4.86-4.93(m, 4H), 4.44-4.52(m, 2H), 4.03-4.07(m, 2H), 3.91-3.98(m, 2H), 3.73(s, 3H), 3.59-3.67(m, 4H), 3.26-3.32(m, 4H), 3.12-3.18(m, 4H), 2.95-3.04(m, 2H) ), 2.53(s, 3H), 2.09(s, 3H), 1.86-1.99(m, 2H), 1.26(t, J = 7.04 Hz, 3H), 1.17(t, J = 7.48 Hz, 3H).

### Example 49

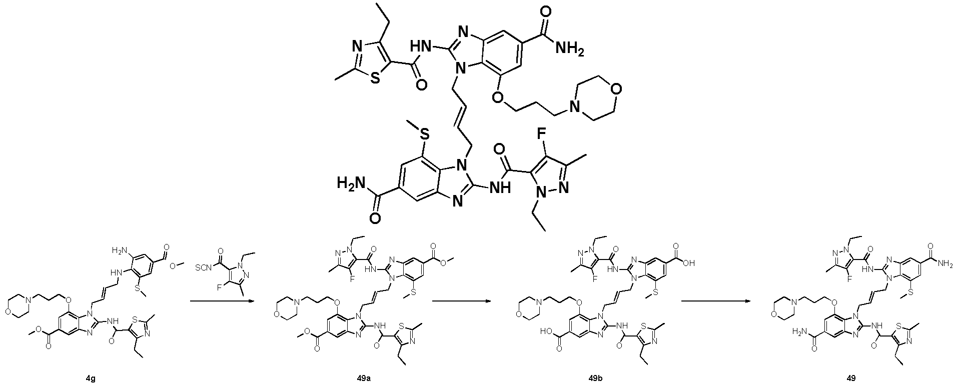

### Step 1: synthesis of methyl (E)-methyl 2-(4-ethyl-2-methylthiazole-5-formylamino)- 1-(4-(2-(1-ethyl-4-fluoro-3-methyl-1H-pyrazole-5-formylamino)-5-(methoxycarbonyl)-7-(methylthio)-1H-benzo[d]imidazol-1-yl)but-2-ene-1-yl)-7-(3-morpholinopropoxy)-1H-benzo[d]imidazole 5-carboxylate

Under an ice bath, 1-ethyl-4-fluoro-3-methyl-1H-pyrazole-5-carbonyl isothiocyanate (49 mg, 0.23 mmol) was added to a solution of 4g (145 mg, 0.19 mmol) in DMF (5 mL). After reacting for 0.5 h, DIPEA (75 mg, 0.58 mmol) and HATU (89 mg, 0.23 mmol) were added, and then the mixture was heated to room temperature to react for 12 h. The reaction solution was slowly poured into water, and a yellow solid precipitated out. The solid was filtered and dried to obtain **49a** (116 mg, yield 64%).
MS(ESI)m/z = 931.3[M+H]

### Step 2: synthesis of (E)-1-(4-(5-carboxy-2-(1-ethyl-4-fluoro-3-methyl-1H-pyrazole-5-formylamino)-7-(methylthio)-1H-benzo[d]imidazol-1-yl)but-2-en-1-yl)-2-(4-ethyl-2-methylthiazole-5-formylamino)-7-(3-morpholinopropoxy)-1H-benzo[d]imidazole-5-carboxylic acid

49a (116 mg, 0.12 mmol) was dissolved in a mixed solution of methanol, tetrahydrofuran and water (volume ratio: 1/1/1), and hydrated lithium hydroxide (54 mg, 12.4 mmol) was added. The temperature was raised to 75°C and the reaction was stirred overnight. The organic solvent was removed by rotary evaporation, the mixture was cooled to room temperature, adjusted with dilute hydrochloric acid (1M) under an ice bath until no solid precipitated. The solid was filtered and dried to obtain compound **49b** (102 mg, yield 90%).
MS(ESI)m/z = 903.3[M+H]

### Step 3: synthesis of (E)-N-(5-carbamoyl-1-(4-(5-carbamoyl-2-(1-ethyl-4-fluoro-3-methyl-1H-pyrazole-5-formylamino))-7-(methylthio)-1H-benzo[d]imidazol-1-yl)but-2-en-1-yl)-7-(3-morpholinopropoxy)-1H-benzo[d]imidazol-2-yl)-4-ethyl-2-methylthiazole-5-formylamide

49b (102 mg, 0.11 mmol) was dissolved in DMF (5 mL), and HATU (103 mg, 0.27 mmol) and DIPEA (88 mg, 0.68 mmol) were added. After 0.5h, ammonium bicarbonate (27 mg, 0.34 mmol) was added, and the mixture was stirred at room temperature for 2h. The reaction solution was concentrated and purified by preparative HPLC to obtain compound **49** (46 mg, yield 45%).
MS(ESI)m/z = 901.3[M+H]
¹H NMR (400M, DMSO-d₆) δ 12.77-13.08(m, 1H), 10.91-10.99(m, 1H), 7.94-8.14 (m, 2H), 7.83-7.90(m, 1H), 7.61-7.68( m, 2H), 7.31-7.48(m, 3H), 5.72-5.84(m, 2H), 5.09-5.17(m, 2H), 4.87-4.91(m, 2H), 4.41-4.49(m, 2H), 4.03-4.13(m, 2H), 3.86-3.97(m, 2H), 3.69-3.81(m, 2H), 3.22-3.33(m, 2H), 3.07-3.20(m, 4H), 2.91-3.04(m , 2H), 2.56(s, 3H), 2.47(s, 3H), 2.08(s, 3H), 1.99-2.07(m, 2H), 1.25(t, J = 7.04 Hz, 3H), 1.16(t, J = 7.48 Hz, 3H).

### Example 50

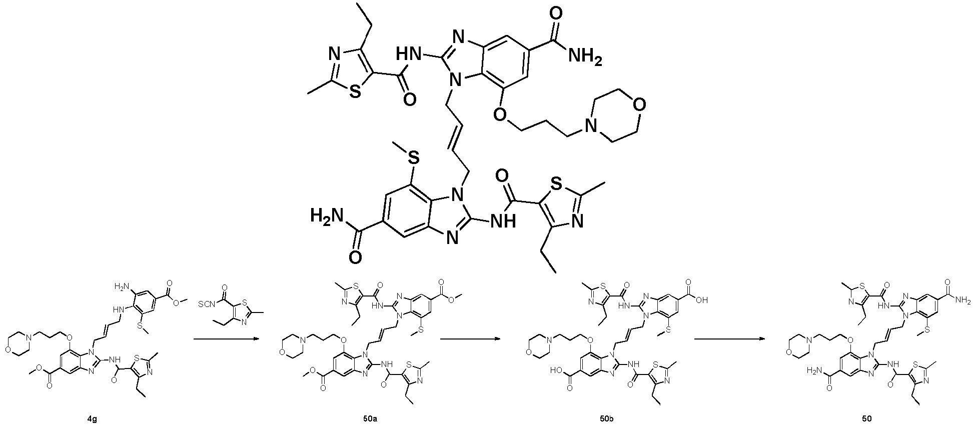

### Step 1: synthesis of methyl (E)-methyl 2-(4-ethyl-2-methylthiazole-5-formylamino)-1-(4-(2-(4-ethyl-2-methylthiazole-5-formylamino)-5-(methoxycarbonyl)-7-(3-morpholinopropoxy)-1H-benzo[d]imidazol-1-yl)but-2-en-1-yl)-7-(methylthio)-1H-benzo[d]imidazole-5-carboxylate

Under an ice bath, to a solution of **4g** (145 mg, 0.19 mmol) in DMF (5 mL), 4-ethyl-2-methylthiazole-5-carbonyl isothiocyanate (49 mg, 0.23 mmol) was added. After reacting for 0.5 h, DIPEA (75 mg, 0.58 mmol) and HATU (89 mg, 0.23 mmol) were added, and then the mixture was heated to room temperature to react for 12 h. The reaction solution was slowly poured into water, and a yellow solid precipitated out. The solid was filtered and dried to obtain **50a** (116 mg, yield 64%).
MS(ESI)m/z = 930.3[M+H]

### Step 2: synthesis of (E)-1-(4-(5-carboxy-2-(4-ethyl-2-methylthiazole-5-formylamino)-7-(3-morpholinopropoxy)-1H-benzo[d]imidazol-1-yl)but-2-en-1-yl)-2-(4-ethyl-2-methylthiazole-5-formylamino)-7-(methylthio)-1H-benzo[d]imidazole-5-carboxylic acid

**50a** (116 mg, 0.12 mmol) was dissolved in a mixed solution of methanol, tetrahydrofuran and water (volume ratio: 1/1/1), and hydrated lithium hydroxide (54 mg, 12.4 mmol) was added. The temperature was raised to 75°C and the reaction was stirred overnight. The organic solvent was removed by rotatory evaporation, the mixture was cooled to room temperature, adjusted with dilute hydrochloric acid (1M) under an ice bath until no solid precipitated. The solid was filtered and dried to obtain compound **50b** (103 mg, yield 91%).
MS(ESI)m/z = 902.3[M+H]

### Step 3: synthesis of (E)-1-(4-(5-carboxy-2-(4-ethyl-2-methylthiazole-5-formylamino)-7-(3-morpholinopropoxy)-1H-benzo[d]imidazol-1-yl)but-2-en-1-yl)-2-(4-ethyl-2-methylthiazole-5-formylamino)-7-(methylthio)-1H-benzo[d]imidazole-5-formylamide

50b (103 mg, 0.11 mmol) was dissolved in DMF (5 mL), and HATU (103 mg, 0.27 mmol) and DIPEA (88 mg, 0.68 mmol) were added. After 0.5h, ammonium bicarbonate (27 mg, 0.34 mmol) was added, and the mixture was stirred at room temperature for 2h. The reaction solution was concentrated and purified by preparative HPLC to obtain compound **50** (36 mg, yield 35%).
MS(ESI)m/z = 900.3[M+H]
¹H NMR (400M, DMSO-d₆) δ 12.79-12.99(m, 1H), 10.99-11.08(m, 1H), 7.89-8.16 (m, 2H), 7.83-7.87(m, 1H), 7.63-7.67( m, 2H), 7.29-7.48(m, 3H), 5.75-5.81(m, 2H), 5.08-5.12(m, 4H), 4.88-4.92(m, 4H), 4.01-4.10(m, 2H), 3.86-3.95(m, 2H), 3.70-3.81(m, 2H), 3.21-3.30(m, 2H), 3.07-3.17(m, 6H), 2.90-3.01(m, 2H), 2.56(s, 3H) ), 1.97-2.09(m, 2H), 1.17(dt, J = 7.44, 3.00 Hz, 6H).

### Example 51

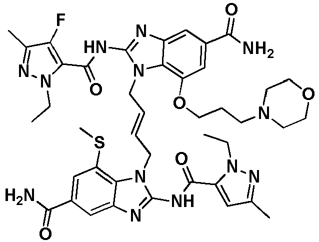

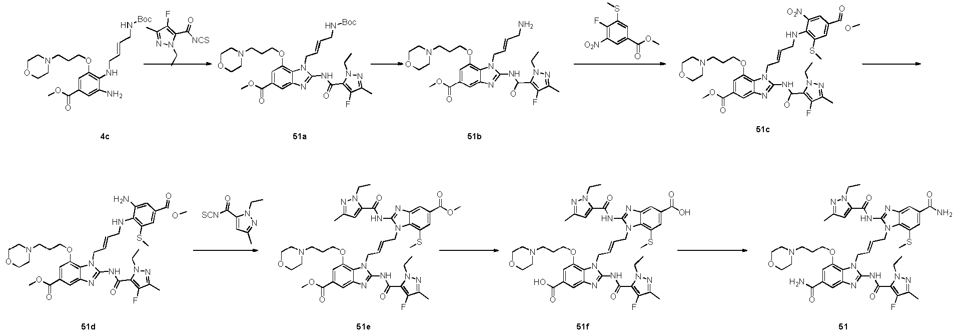

### Step 1: synthesis of methyl (E)-methyl 1-(4-((tert-butoxycarbonyl)amino)but-2-en-1-yl)-2-(1-ethyl-4-fluoro-3-methyl-1H-pyrazole-5-carboxamino)-7-(3-morpholinopropoxy)-1H-benzo[d]imidazole-5-carboxylate

Under an ice bath, 1-ethyl-4-fluoro-3-methyl-1H-pyrazole-5-carbonyl isothiocyanate (0.34 g, 1.55 mmol) was added to a solution of **4c** (0.74 g, 1.55 mmol) in DMF (5 mL). After reacting for 0.5 h, DIPEA (0.6 g, 4.65 mmol) and HATU (0.71 g, 1.86 mmol) were added, and then the mixture was heated to room temperature for 12 h. The reaction solution was slowly poured into water, and a yellow solid precipitated out. The solid was filtered and dried to obtain **51a** (0.88 g, yield 86%).
MS(ESI)m/z = 658.3[M+H]

### Step 2: synthesis of methyl (E)-methyl 1-(4-aminobut-2-en-1-yl)-2-(1-ethyl-4-fluoro-3-methyl-1H-pyrazole-5-formylamino)-7-(3-morpholinopropoxy)-1H-benzo[d]imidazole-5-carboxylate

Under an ice bath, TFA (3 mL) was added to 51a (0.88 g, 1.34 mmol) in DCM (40 mL), and then the mixture was heated to room temperature for 2 h. The solvent was spin-dried under reduced pressure, ethyl acetate was added and then the free TFA was removed by spin-drying under reduced pressure to obtain compound **51b** (0.66 g, yield 88%).
MS(ESI)m/z = 558.3[M+H]

### Step 3: synthesis of methyl (E)-methyl 2-(1-ethyl-4-fluoro-3-methyl-1H-pyrazole-5-formylamino)-1-(4-((4-(methoxycarbonyl)-2-(methylthio)-6-nitrophenyl)amino)but-2-en-1-yl)-7-(3-morpholinopropoxy)-1H-benzo[d]imidazole-5-carboxylate

To a solution of 51b (557 mg, 1 mmol) in DMF (10 mL), DIPEA (387 mg, 3 mmol) and methyl 4-fluoro-3-methylthio-5-nitrobenzoate (245 mg, 1 mmol) were added, the mixture was reacted at room temperature for 3h. The reaction solution was poured into water, extracted with ethyl acetate, washed with saturated brine and dried over anhydrous sodium sulfate, and the solvent was spin-dried under reduced pressure to obtain compound **51c** (451 mg, yield 58%).
MS(ESI)m/z = 783.3[M+H]

### Step 4: synthesis of methyl (E)-1-(4-((2-amino-4-(methoxycarbonyl)-6-(methylthio)phenyl)amino)but-2-en-1-yl)-2-(4-ethyl-2)methyl ester-methylthiazole-5-formylamino)-7-(3-morpholinomethyl)-1H-benzo[d]imidazole -5-carboxylate

Under an ice bath, ammonium hydroxide (1 mL) was added to a solution of **51c** (451 mg, 0.58 mmol) in methanol (5 mL). After 10 minutes, an aqueous solution of sodium dithionite (502 mg, 2.88 mmol) was added and the mixture was slowly heated to react at room temperature for 2 h. Inorganic salt was removed by filtration, and ethyl acetate was added for extraction. The organic phase was washed with saturated brine, dried over anhydrous magnesium sulfate and spin-dried to obtain the crude product, and then purified with a silica gel column (eluent: petroleum ether/ethyl acetate = 1/1) to obtain compound **51d** (417 mg, yield 94%).
MS(ESI)m/z = 753.3[M+H]

### Step 5: synthesis of methyl (E)-methyl 2-(1-ethyl-3-methyl-1H-pyrazole-5-formylamino)-1-(4-(2-(1-ethyl-4-fluoro-3-methyl-1H-pyrazole-5-formylamino)-5-(methoxycarbonyl)-7-(3-morpholinopropoxy)-1H-benzo[d]imidazole-1-yl)but-2-en-1-yl)-7-(methylthio)-1H-benzo[d]imidazole-5-carboxylate

Under an ice bath, to a solution of **51d** (139 mg, 0.193 mmol) in DMF (5 mL), 1-ethyl-3-methyl-1H-pyrazole-5-carbonyl isothiocyanate (46 mg, 0.232 mmol) was added. After 0.5 h of reaction, DIPEA (75 mg, 0.579 mmol) and HATU (89 mg, 0.232 mmol) were added, and then the mixture was heated to room temperature to react for 12 h. The reaction solution was slowly poured into water, and a yellow solid precipitated out. The solid was filtered and dried to obtain **51e** (120 mg, yield 71%).
MS(ESI)m/z = 914.3[M+H]

### Step 6: synthesis of (E)-1-(4-(5-carboxy-2-(1-ethyl-3-methyl-1H-pyrazole-5-formylamino)-7-(methylthio)-1H-benzo[d]imidazol-1-yl)but-2-en-1-yl)-2-(1-ethyl-4-fluoro-3-methyl-1H-pyrazole-5-carboxamino)-7-(3-morpholinopropoxy)-1H-benzo[d]imidazole-5-carboxylic acid

51e (120 mg, 0.13 mmol) was dissolved in a mixed solution of methanol, tetrahydrofuran and water (volume ratio: 1/1/1), and lithium hydroxide (55 mg, 1.31 mmol) was added. The temperature was raised to 75°C and the reaction was stirred overnight. The organic solvent was removed by rotatory evaporation, the mixture was cooled to room temperature, adjusted with dilute hydrochloric acid (1M) in an ice bath until no solid was precipitated. The solid was filtered and dried to obtain compound **51f** (112 mg, yield 96%).
MS(ESI)m/z = 886.3[M+H]

### Step 7: synthesis of (E)-1-(4-(5-(5-carbamoyl-2-)(1-styrene-3-methyl-1H-pyrazole-5-formylamino)-7-(methylthio)-1H-benzo[d]imidazol-1-yl)but-2-en-1-yl)-2-(1-styrene-4-fluoro-3-methyl-1H-pyrazole-5-anthralamido)-7-(3-morpholinopropoxy)-1H-benzo[d]imidazole-5-formylamide

51f (112 mg, 0.126 mmol) was dissolved in DMF (5 mL), and HATU (116 mg, 0.304 mmol) and DIPEA (98 mg, 0.759 mmol) were added. After 0.5h, ammonium bicarbonate (30 mg, 0.380 mmol) was added and stirred at room temperature for 2h. The reaction solution was concentrated and purified by preparative HPLC to obtain compound 51 (51 mg, yield 46%).
MS(ESI)m/z = 884.3[M+H]
¹H NMR (400M, DMSO-d₆) δ 12.76-13.12(m, 1H), 11.07-11.17(m, 1H), 7.95-8.13 (m, 2H), 7.85-7.90(m, 1H), 7.61-7.70( m, 2H), 7.31-7.46(m, 3H), 6.51(s, 1H), 5.71-5.76(m, 2H), 5.09-5.16(m, 2H), 4.89-4.96(m, 2H), 4.42- 4.47(m, 4H), 4.00-4.07(m, 2H), 3.85-3.94(m, 2H), 3.70-3.81(m, 2H), 3.18-3.28(m, 2H), 3.04-3.13(m, 2H)), 2.87-3.00(m, 2H), 2.45(s, 3H), 2.10(s, 6H), 1.95-2.04(m, 2H), 1.23-1.32(m, 6H).

### Example 52

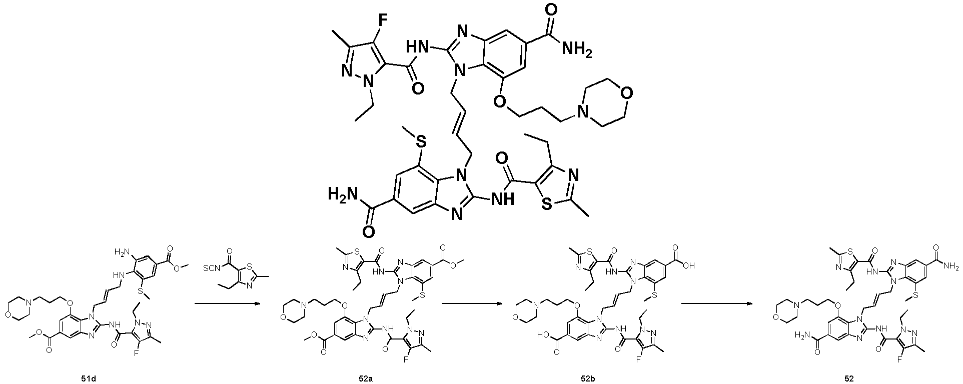

### Step 1: synthesis of methyl (E)-methyl 2-(4-ethyl-2-methylthiazole-5-formylamino)-1-(4-(2-(1-ethyl-4-fluoro-3-methyl-1H-pyrazole-5-formylamino)-5-(methoxycarbonyl)-7-(3-morpholinopropoxy)-1H-benzo[d]imidazol-1-yl)butan-2-en-1-yl)-7-(methylthio)-1H-benzo[d]imidazole 5-carboxylate

Under an ice bath, to a solution of **51d** (139 mg, 0.193 mmol) in DMF (5 mL), 4-ethyl-2-methylthiazole-5-carbonyl isothiocyanate (49 mg, 0.232 mmol) was added. After 0.5 h of reaction, DIPEA (75 mg, 0.579 mmol) and HATU (89 mg, 0.232 mmol) were added, and then the mixture was heated to room temperature to react for 12 h. The reaction solution was slowly poured into water, and a yellow solid precipitated. The solid was filtered and dried to obtain **52a** (118 mg, yield 68%).
MS(ESI)m/z = 931.3[M+H]

### Step 2: synthesis of (E)-1-(4-(5-carboxy-2-(1-ethyl-4-fluoro-3-methyl-1H-pyrazole-5-carboxamino)-7-(3-morpholinopropoxy)-1H-benzo[d]imidazol-1-yl)but-2-en-1-yl)-2-(4-ethyl-2-methylthiazole-5-formylamino)-7-(methylthio)-1H-benzo[d]imidazole-5-carboxylic acid

52a (118 mg, 0.13 mmol) was dissolved in a mixed solution of methanol, tetrahydrofuran and water (volume ratio: 1/1/1), and lithium hydroxide (55 mg, 1.31 mmol) was added. The temperature was raised to 75°C and the reaction was stirred overnight. The organic solvent was removed by rotatory evaporation, and the mixture was cooled to room temperature, adjusted with dilute hydrochloric acid (1M) under an ice bath until no solid precipitated. The solid was filtered and dried to obtain compound **52b** (110 mg, yield 96%).
MS(ESI)m/z = 903.3[M+H]

### Step 3: synthesis of (E)-N-(5-carbamoyl-1-(4-(5-carbamoyl-2-(1-ethyl-4-fluoro-3-methyl-1H-pyrazole-5-formylamino))-7-(3-morpholinopropoxy)-1H-benzo[d]imidazol-1-yl)but-2-en-1-yl)-7-(methylthio)-1H-benzo[d]imidazol-2-yl)-4-ethyl-2-methylthiazole-5-formylamide

52b (110 mg, 0.122 mmol) was dissolved in DMF (5 mL), and HATU (112 mg, 0.293 mmol) and DIPEA (95 mg, 0.732 mmol) were added. After 0.5h, ammonium bicarbonate (29 mg, 0.366 mmol) was added, and the mixture was stirred at room temperature for 2h. The reaction solution was concentrated and purified by preparative HPLC to obtain compound **52** (32 mg, yield 29%).
MS(ESI)m/z = 901.3[M+H]
¹H NMR (400M, DMSO-d₆) δ 12.93-12.98(m, 1H), 10.88-10.96(m, 1H), 7.94-8.13 (m, 2H), 7.84-7.88(m, 1H), 7.63-7.68( m, 2H), 7.28-7.46(m, 3H), 5.70-5.83(m, 2H), 5.06-5.12(m, 2H), 4.90-4.96(m, 2H), 4.45-4.52(m, 4H), 3.98-4.06(m, 2H), 3.86-3.94(m, 2H), 3.68-3.80(m, 2H), 3.19-3.27(m, 2H), 3.05-3.16(m, 4H), 2.88-3.00(m , 2H), 2.49(s, 6H), 2.10(s, 3H), 1.93-2.03(m, 2H), 1.28(t, J= 7.04 Hz, 3H), 1.16(t, J= 7.48 Hz, 3H).

### Example 53

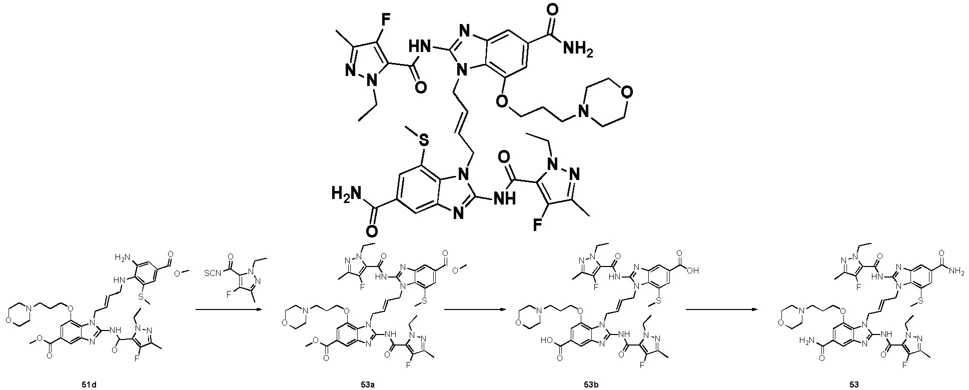

### Step 1: synthesis of methyl (E)-methyl 2-(1-ethyl-4-fluoro-3-methyl-1H-pyrazole-5-carboxamino)-1-(4-(2-(1-ethyl-4-fluoro-3-methyl-1H-pyrazole-5-formylamino)-5-(methoxycarbonyl)-7-(3-morpholinopropoxy)-1H-benzo[d]imidazol-1-yl)-2-en-1-yl)-7-(methylthio))-1H-benzo[d]imidazole-5-carboxylate

Under an ice bath, 1-ethyl-4-fluoro-3-methyl-1H-pyrazole-5-carbonyl isothiocyanate (49 mg, 0.232 mmol) was added to a solution of **51d** (139 mg, 0.193 mmol) in DMF (5 mL). After 0.5 h of reaction, DIPEA (75 mg, 0.579 mmol) and HATU (89 mg, 0.232 mmol) were added, and then the mixture was heated to room temperature to react for 12 h. The reaction solution was slowly poured into water, and a yellow solid precipitated out. The solid was filtered and dried to obtain **53a** (108 mg, yield 62%).
MS(ESI)m/z = 932.3[M+H]

### Step 2: synthesis of (E)-1-(4-(5-carboxy-2-(1-ethyl-4-fluoro-3-methyl-1H-pyrazole-5-carboxamino)-7-(3-morpholinopropoxy)-1H-benzo[d]imidazol-1-yl)but-2-en-1-yl)-2-(1-ethyl-4-fluoro-3-methyl-1H-pyrazole-5-formylamino)-7-(methylthio)-1H-benzo[d]imidazole-5-formamide

53a (108 mg, 0.12 mmol) was dissolved in a mixed solution of methanol, tetrahydrofuran and water (volume ratio: 1/1/1), and lithium hydroxide (49 mg, 1.16 mmol) was added. The temperature was raised to 75°C and the reaction was stirred overnight. The organic solvent was removed by rotatory evaporation, the mixture was cooled to room temperature, adjusted with dilute hydrochloric acid (1M) under an ice bath until no solid precipitated. The solid was filtered and dried to obtain compound **53b** (100 mg, yield 95%).
MS(ESI)m/z = 904.3[M+H]

### Step 3: synthesis of (E)-1-(4-(5-carbamoyl-2-(1-ethyl-4-fluoro-3-methyl-1H-pyrazole-5-carboxamino)-7-(3-morpholinopropoxy)-1H-benzo[d]imidazol-1-yl)but-2-en-1-yl)-2-(1-ethyl-4-fluoro-3-methyl-1H-pyrazole-5-formylamino)-7-(methylthio)-1H-benzo[d]imidazole-5-carboxamide

**53b** (100 mg, 0.111 mmol) was dissolved in DMF (5 mL), and HATU (102 mg, 0.266 mmol) and DIPEA (88 mg, 0.665 mmol) were added. After 0.5h, ammonium bicarbonate (27 mg, 0.333 mmol) was added, and the mixture was stirred at room temperature for 2h. The reaction solution was concentrated and purified by preparative HPLC to obtain compound 53 (41 mg, yield 41%).
MS(ESI)m/z = 902.3[M+H]
¹H NMR (400M, DMSO-d₆) δ 12.89-13.08(m, 1H), 10.47-10.57(m, 1H), 7.92-8.13 (m, 2H), 7.85-7.89(m, 1H), 7.61-7.69( m, 2H), 7.30-7.48(m, 3H), 5.74-5.79(m, 2H), 5.09-5.16(m, 2H), 4.89-4.95(m, 2H), 4.41-4.49(m, 6H), 4.03-4.07(m, 2H), 3.89-3.95(m, 2H), 3.66-3.75(m, 2H), 3.21-3.30(m, 2H), 3.08-3.17(m, 2H), 2.92-3.02(m , 2H), 2.45(s, 3H), 2.10(s, 3H), 2.08(s, 3H), 1.91-2.03(m, 2H), 1.20-1.30(m, 6H).

### Example 54

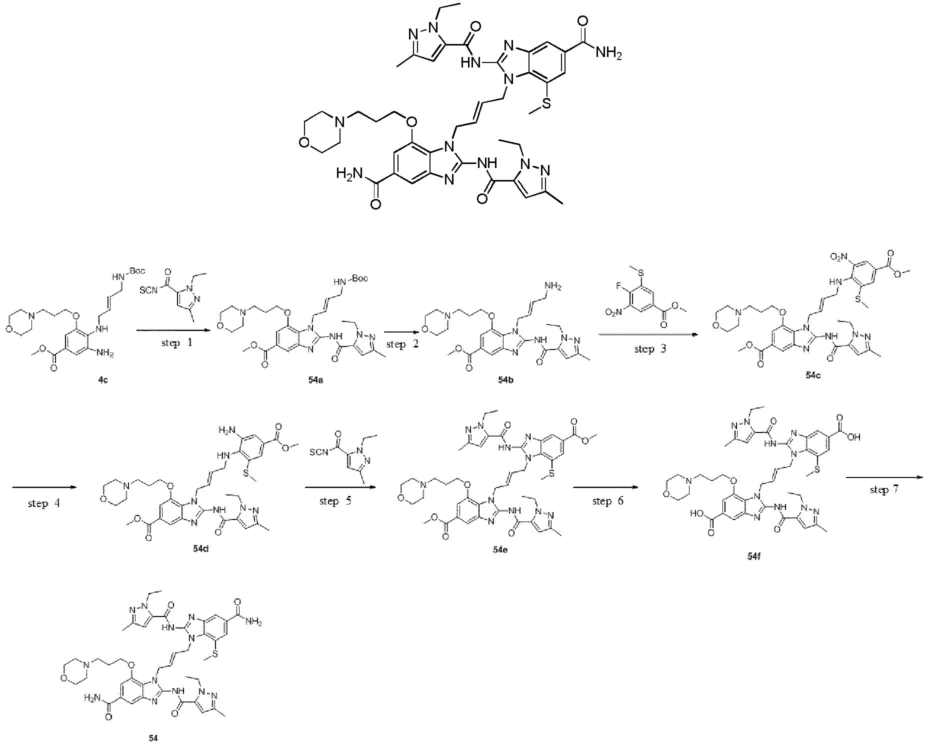

### Step 1: synthesis of methyl (E)-methyl 1-(4-((tert-butoxycarbonyl)amino)but-2-en-1-yl)-2-(1-ethyl-3-methyl-1H-pyrazole-5-formylamino)-7-(3-morpholinopropoxy)-1H-benzo[d]imidazole-5-carboxylate

Under an ice bath, to a solution of 4c (3.0 g, 6.27 mmol) in DMF (30 mL), 1-ethyl-3-methyl-1H-pyrazole-5-carbonyl isothiocyanate (1.35 g, 6.90 mmol) was added. After reacting for 0.5 h, DIPEA (1.62 g, 12.54 mmol) and HATU (2.62 g, 6.9 mmol) were added, and then the mixture was heated to room temperature to react for 3 h. The reaction solution was slowly poured into water, and a yellow solid precipitated out. The solid was filtered and dried to obtain **54a** (3.21 g, yield 80%).
MS(ESI)m/z = 640.3[M+H]

### Step 2: synthesis of methyl (E)-methyl 1-(4-aminobut-2-en-1-yl)-2-(1-ethyl-3-methyl-1H-pyrazole-5-formylamino)-7-(3-morpholinopropoxy)-1H-benzo[d]imidazole-5-carboxylate

Under an ice bath, TFA (5 mL) was added to 54a (3.20 g, 4.0 mmol) in DCM (30 mL), and then heated to room temperature for 2 h. The solvent was spin-dried under reduced pressure, ethyl acetate was added and then the free TFA was removed by spin-drying under reduced pressure to obtain compound **54b** (4.2 g), which was directly used in the next step.
MS(ESI)m/z = 540.3[M+H]

### Step 3: synthesis of methyl (E)-methyl 2-(1-ethyl-3-methyl-1H-pyrazole-5-formylamino)-1-(4-((4-(methoxycarbonyl)-2-(methylthio)-6-nitrophenyl)amino)but-2-en-1-yl)-7-(3-morpholinopropoxy)-1H-benzo[d]imidazole-5-carboxylate

To a solution of **54b** (296 mg, crude product, 0.37 mmol) in DMF (10 mL), DIPEA (143 mg, 1.1 mmol) and methyl 4-fluoro-3-methylthio-5-nitrobenzoate (91 mg, 0.37 mmol) were added, the mixture was reacted at room temperature for 3 h. After the reaction was completed, the mixture was poured into water, filtered, washed with water and dried to obtain **54c** as a yellow solid (120 mg, yield 42%).
MS(ESI)m/z = 765.3[M+H]

### Step 4: synthesis of methyl (E)-methyl 1-(4-(((2-amino-4-(methoxycarbonyl)-6-(methylthio)phenyl)amino)but-2-en-1-yl)-2-(1-ethyl-3-methyl-1H-pyrazole-5-formylamino)-7-(3-morpholinopropoxy)-1H-benzo[d]imidazole-5-carboxylate

Under an ice bath, ammonium hydroxide (1 mL) was added to a solution of **54c** (120 mg, 0.16 mmol) in methanol (5 mL). After 10 minutes, an aqueous solution of sodium dithionite (140 mg, 0.8 mmol) was added and the mixture was slowly heated to react at room temperature for 2 h. Inorganic salt was removed by filtration and ethyl acetate was added for extraction. The organic phase was washed with saturated brine, dried over anhydrous magnesium sulfate and spin-dried to obtain the crude product, which was purified by silica gel column (eluent: dichloromethane/methanol=10/1, v/v) to obtain compound **54d** (55 mg, yield 48%).
MS(ESI)m/z = 735.3[M+H]

### Step 5: synthesis of methyl (E)-methyl 2-(1-ethyl-3-methyl-1H-pyrazole-5-formylamino)-1-(4-(2-(1-ethyl-3-methyl-1H-pyrazole-5-formylamino)-5-(methoxycarbonyl)-7-(3-morpholinopropoxy)-1H-benzo[d]imidazol-1-yl)butan-2-en-1-yl)-7-(methylthio)-1H-benzo[d]imidazole-5-carboxylate

Under an ice bath, to a solution of **54d** (55 mg, 0.074 mmol) in DMF (5 mL), 1-ethyl-3-methyl-1H-pyrazole-5-carbonyl isothiocyanate (16 mg, 0.08 mmol) was added. After 0.5 h of reaction, DIPEA (20 mg, 0.15 mmol) and HATU (34 mg, 0.09 mmol) were added, and then the temperature was raised to room temperature to react for 3.5 h. The reaction solution was slowly poured into water, and a white solid precipitated out. The solid was filtered and dried to obtain **54e** (50 mg, yield 75%).
MS(ESI)m/z = 897.0[M+H]

### Step 6: synthesis of (E)-1-(4-(5-carboxy-2-(1-ethyl-3-methyl-1H-pyrazole-5-carboxamino)-7-(3-morpholinopropoxy)-1H-benzo[d]imidazol-1-yl)but-2-en-1-yl)-2-(1-ethyl-3-methyl-1H-pyrazole-5-carboxamino)-7-(methylthio)-1H-benzo[d]imidazole-5-carboxylic acid

**54e** (50 mg, 0.056 mmol) was dissolved in a mixed solution (3.5 mL) of methanol, tetrahydrofuran and water (volume ratio: 2/1/0.5), and lithium hydroxide (12 mg, 0.28 mmol) was added. The temperature was raised to 75°C and the reaction was stirred overnight. The organic solvent was removed by rotatory evaporation. The mixture was cooled to room temperature, adjusted with dilute hydrochloric acid (1M) under an ice bath until no solid precipitated. The solid was filtered and dried to obtain compound **54f** (38 mg, yield 78%).
MS(ESI)m/z = 867.0[M+H]

### Step 7: synthesis of (E)-1-(4-(5-carbamoyl-2-(1-ethyl-3-methyl-1H-pyrazole-5-formylamino)-7-(3-morpholinopropoxy)-1H-benzo[d]imidazol-1-yl)but-2-en-1-y1)-2-(1-ethyl-3-methyl-1H-pyrazole-5-carboxamino)-7-(methylthio)-1H-benzo[d]imidazole-5-formylamide

**54f** (38 mg, 0.043 mmol) was dissolved in DMF (5 mL), and HATU (49 mg, 0.129 mmol) and DIPEA (30 mg, 0.215 mmol) were added. After 0.5h, ammonium bicarbonate (16 mg, 0.131 mmol) was added, and the mixture was stirred at room temperature for 2 h. The reaction solution was concentrated and purified by preparative HPLC to obtain compound **54** (18 mg, yield 47%) as a white solid.
MS(ESI)m/z = 866.3[M+H]
¹H NMR (400 MHz, DMSO) δ 12.98 (s, 2H), 9.79 (s, 1H), 8.06 (s, 1H), 7.95 (s, 1H), 7.87 (d, J = 1.3 Hz, 1H), 7.66 (s, 1H), 7.63 (d, J = 1.3 Hz, 1H), 7.43 (s, 1H), 7.37 (s, 1H), 7.29 (s, 1H), 6.52 (s, 1H), 6.51 (s, 1H), 5.78 - 5.61 (m, 2H), 5.12 (s, 2H), 4.90 (s, 2H), 4.52 (p, J = 7.3 Hz, 4H), 4.00 (t, J = 5.6 Hz, 2H), 3.94 (d, J = 12.4 Hz, 2H), 3.26 (d, J = 11.8 Hz, 3H), 3.11 (s, 3H), 2.97 (s, 3H), 2.43 (d, J = 8.4 Hz, 3H), 2.12 (s, 3H), 2.10 (s, 3H), 1.96 - 1.81 (m, 2H), 1.28 (td, J = 7.1, 4.1 Hz, 6H).

### Example 56

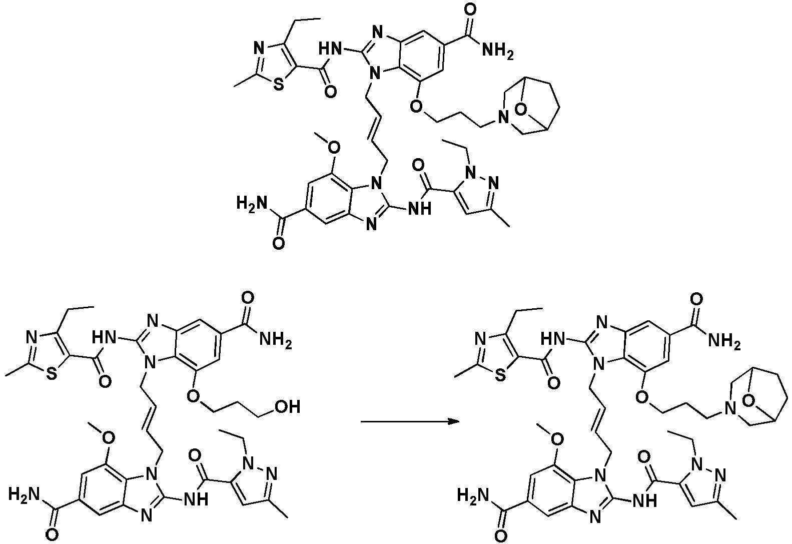

Under an ice bath, to a solution of compound **55** (100 mg, 0.12 mmol) in tetrahydrofuran (5 mL), triethylamine (36 mg, 0.36 mmol) and methanesulfonyl chloride (14 mg, 0.13 mmol) were added. The temperature was raised to room temperature and reacted for 2 h. LCMS showed that the reaction was completed. Potassium carbonate (50 mg, 0.36 mmol) and 8-oxa-3-azabicyclo[3.2.1]octane (68 mg, 0.6 mmol) were added to the reaction solution, and the temperature was raised to 50°C for reaction for 2h, and the solvent was spin-dried. The crude product was purified by preparative HPLC to obtain compound **56** (15 mg).

According to the preparation method of compound 56, using compound 55 and the corresponding amine as raw materials, compounds **57, 58, 59, 60, 61, 62, 63, 64** were obtained.

| **Example** | **structure of compound** | **corresponding amine** | **MS(ESI)m/z** |
|---|---|---|---|
| 57 | 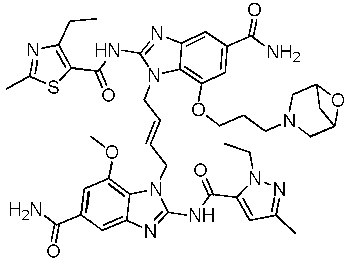 | 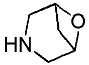 | 879.4 [M+H]⁺ |
| 58 | 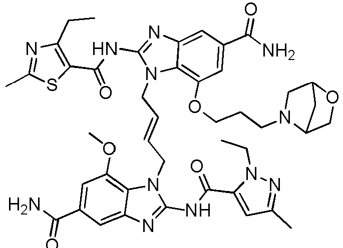 | 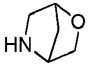 | 879.4 [M+H]⁺ |
| 59 | 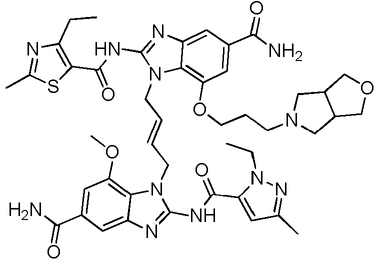 | 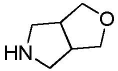 | 893.4 [M+H]⁺ |
| 60 | 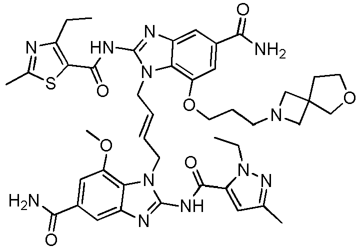 | 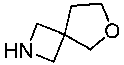 | 893.4 [M+H]⁺ |
| 61 | 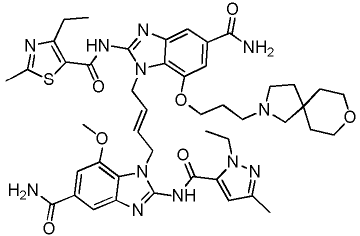 | 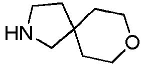 | 921.4 [M+H]⁺ |
| 62 | 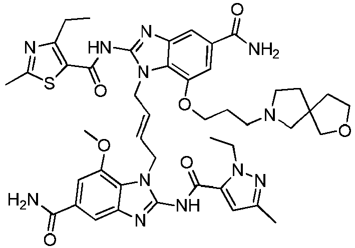 | 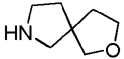 | 907.4 [M+H]⁺ |
| 63 | 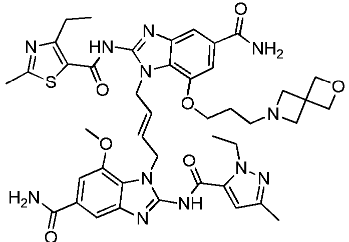 | 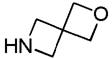 | 879.4 [M+H]⁺ |
| 64 | 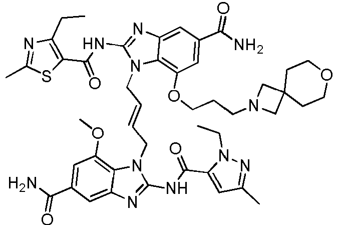 | 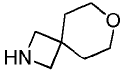 | 907.4 [M+H]⁺ |

### Example 65

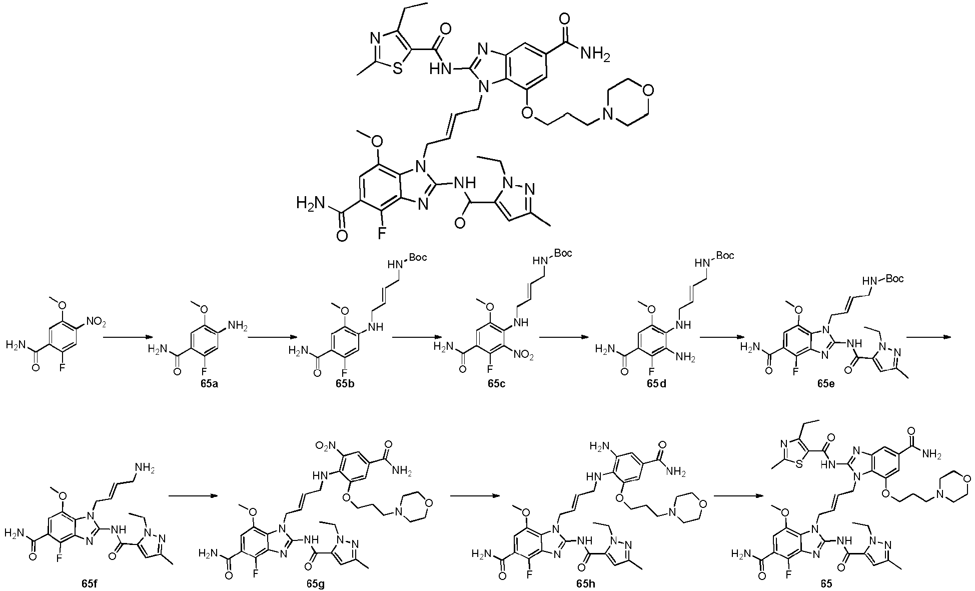

### Step 1: synthesis of 4-amino-2-fluoro-5-methoxybenzamide

At room temperature, 10% palladium on carbon (1.5 g) was added to a solution of 2-fluoro-5-methoxy-4-nitrobenzamide (10 g, 46.7 mmol) in methanol (100 mL), and hydrogenation was performed at room temperature for 5 h. The palladium carbon was filtered off, and the filtrate was spin-dried to obtain compound **65a** (7.7 g, yield 89%).
MS(ESI)m/z = 185[M+H]⁺

### Step 2: synthesis of tert-butyl (E)-(4-((4-carbamoyl-5-fluoro-2-methoxyphenyl)amino)but-2-en-1-yl)carbamate

Compound **65a** (7.5 g, 40.7 mmol) was dissolved in DMF (150 mL), and potassium carbonate (8.4 g, 61 mmol) and tert-butyl (E)-(4-bromobut-2-en-1-yl)carbamate (10.1 g, 40.7 mmol) were added sequentially. The mixture was heated to 60°C and reacted for 8 hours. After cooling, inorganic salts were removed by filtration. The filtrate was spin-dried, dissolved with ethyl acetate, washed with water and saturated brine respectively. The organic phase was spin-dried and purified by silica gel column (petroleum ether/ethyl acetate = 1/2) to obtain compound **65b** (5.2 g, yield 36%).
MS(ESI)m/z = 354[M+H]⁺

### Step 3: synthesis of tert-butyl (E)-(4-((4-carbamoyl-5-fluoro-2-methoxy-6-nitrophenyl)amino)but-2-en-1-yl)carbamate

Compound **65b** (5.2 g, 14.7 mmol) was dissolved in acetic anhydride (30 mL), and when it was cooled to 0 °C under an ice bath, nitric acid (1.7 g, 17.6 mmol, 65% purity) was added dropwise to the reaction solution. The reaction was maintained at 0°C for 2 h. The reaction solution was poured into water, extracted with ethyl acetate, washed with saturated brine. The organic phase was concentrated and purified by silica gel column (petroleum ether/ethyl acetate = 1/1) to obtain compound **65c** (2 g, yield 34%).
MS(ESI)m/z = 399[M+H]⁺

### Step 4: synthesis of tert-butyl (E)-(4-((4-carbamoyl-5-fluoro-2-methoxy-6-aminophenyl)amino)but-2-en-1-yl)carbamate

Under an ice bath, ammonium hydroxide (5 mL) was added to a solution of **65c** (2 g, 5 mmol) in methanol (20 mL). After 10 minutes, an aqueous solution of sodium dithionite (4.3 g, 25 mmol) was added and reacted for 2 h. Inorganic salts were removed by filtration, and ethyl acetate was added for extraction. The organic phase was washed with saturated brine, dried over anhydrous magnesium sulfate and spin-dried to obtain the crude product, which was purified on a silica gel column (eluent: dichloromethane/methanol=50/1-30/1) to obtain compound **65d** (1.1 g, yield 64%).
MS(ESI)m/z = 369[M+H]⁺

### Step 5: synthesis of tert-butyl (E)-(4-(5-carbamoyl-2-(1-ethyl-3-methyl-1H-pyrazole-5-formylamino)-4-fluoro-7-methoxy-1H-benzo[d]imidazol-1-yl)-2-en-1-yl)carbamate

Under an ice bath, to a solution of **65d** (1.1 g, 2.9 mmol) in DMF (20 mL), 1-ethyl-3-methyl-1H-pyrazole-5-carbonyl isothiocyanate (565 mg, 2.9 mmol) was added. After reacting for 0.5 h, DIPEA (748 mg, 5.8 mmol) and HATU (1.3 g, 3.48 mmol) were added, and then heated to room temperature to react for 12 h. The reaction solution was slowly poured into water, and a yellow solid precipitated out. The solid was filtered and dried to obtain **65e** (1.1 g, yield 75%).
MS(ESI)m/z = 530[M+H]⁺

### Step 6: synthesis of methyl (E)-1-(4-aminobut-2-en-1-yl)-2-(4-ethyl-2-methylthiazole-5-formylamino)-7-methoxy-1H-benzo[d]imidazole-5-carboxylate

Under an ice bath, TFA (5 mL) was added to 65e (1.1 g, 2.1 mmol) in DCM (10 mL), and then heated to room temperature for 2 h. The solvent was spin-dried under reduced pressure, ethyl acetate was added and then free TFA was removed by spin-drying under reduced pressure to obtain compound **65f** (1.5 g, containing trifluoroacetic acid).
MS(ESI)m/z =430[M+H]⁺

### Step 7: synthesis of (E)-1-(4-aminobut-2-en-1-yl)-2-(1-ethyl-3-methyl-1H-pyrazole-5-formylamino)-4-fluoro-7-methoxy-1H-benzo[d]imidazole-5-carboxamide

**65f** (1.5 g, 2.1 mmol, 57% purity) was dispersed in n-butanol (10 mL), and 4-chloro-3-(3-morpholinopropoxy)-5-nitrobenzamide (720 mg, 2.1 mmol) and DIPEA (1.35 g, 10.5 mmol) were added. The reaction solution was heated to 120°C and stirred for 18 h. The crude product obtained by distillation under reduced pressure was dissolved in ethyl acetate, washed with water and saturated brine respectively, dried over anhydrous sodium sulfate and spin-dried, and purified by silica gel column (eluent: dichloromethane/methanol=30/1) to obtain **65g** (770 mg, yield 49%).
MS(ESI)m/z = 737.[M+H]⁺

### Step 8: synthesis of (E)-1-(4-(((2-amino-4-carbamoyl-6-(3-morpholinopropoxy) phenyl)amino)but-2-ene-1-yl)-2-(1-ethyl-3-methyl-1H-pyrazole-5-formylamino)-4-fluoro-7-methoxy-1H-benzo[d]imidazole-5-formylamide

Under an ice bath, ammonium hydroxide (1 mL) was added to a solution of **65g** (500 mg, 0.68 mmol) in methanol (10 mL). 10 minutes later, an aqueous solution of sodium dithionite (591 mg, 3.4 mmol) was added, and the mixture was slowly heated to react at room temperature for 2 h. The inorganic salt was removed by filtration, the filtrate was concentrated to obtain the crude product, and then purified by reversed-phase column chromatography (eluent: acetonitrile/water=60/40) to obtain compound **65h** (200 mg, yield 41%).
MS(ESI)m/z = 707[M+H]⁺

### Step 9: synthesis of (E)-N-(5-carbamoyl-1-(4-(5-carbamoyl-2-(1-ethyl-3-methyl-1H-pyrazole-5-formylamino)yl)-4-fluoro-7-methoxy-1H)-benzo[d]imidazol-1-yl)but-2-en-1-yl)-7-(3-morpholinopropoxy)-1H-benzo[d]imidazol-2-yl)-4-ethyl-2-methylthiazole-5-formylamide

Under an ice bath, 4-ethyl-2-methylthiazole-5-carbonyl isothiocyanate (30 mg, 0.14 mmol) was added to a solution of **65h** (100 mg, 0.14 mmol) in DMF (3 mL). After 0.5 h of reaction, DIPEA (54 mg, 0.42 mmol) and HATU (53 mg, 0.14 mmol) were added, and then the mixture was heated to room temperature for 2 h. The reaction solution was purified by preparative HPLC to obtain **65** (20 mg, yield 16%).
MS(ESI)m/z = 885[M+H]⁺

The following experimental examples are used to illustrate the beneficial effects of the present invention:

### Experimental example 1: The binding affinity test of the compound of the present invention and Sting protein

### (1) Experimental method

The protein thermal transfer test (TSA) was used to determine the binding affinity of the compound to Sting protein. The 100ug/ml Sting protein was mixed with different concentrations of the compound and 5X SYPRO Orange dye in a buffer of 20mM Hepes, 150mM NaCl, 1mM MgCl₂, 1mM DTT, pH=7.5. The protein dissolution curve was measured on a qPCR instrument, the Tm value was fitted with Protein Thermal Shift Software 1.3 software, and the Tm difference of the protein was calculated when different concentrations of compound and no compound were added. The dissociation constant Kd was fitted and obtained according to the change of _{Δ}Tm with the concentration of the compound. The compound 3 reported in the article (Nature. 2018 Dec;564(7736):439-443) was used as a positive reference compound. The test results are shown in Table 5 below. The Kd value of each compound is classified according to the following instructions:
"+" means the Kd value is greater than 10 µM;
"++" means that the Kd value is less than 10 µM and greater than 1 µM;
"+++" means that the Kd value is less than 1 µM;
The lower the Kd, the stronger the binding affinity of the compound to the Sting protein.

### (2) Experimental results

**Table 6 binding affinity of the compound to Sting protein**

| Example | Kd |
|---|---|
| reference compound | ++ |
| 1 | ++ |
| 2 | ++ |
| 3 | ++ |
| 4 | ++ |
| 5 | ++ |
| 6 | ++ |
| 7 | ++ |
| 8 | ++ |
| 9 | ++ |
| 10 | ++ |
| 11 | ++ |
| 12 | ++ |
| 13 | ++ |
| 14 | ++ |
| 15 | ++ |
| 16 | ++ |
| 17 | ++ |
| 18 | ++ |
| 19 | ++ |
| 20 | ++ |
| 21 | ++ |
| 22 | ++ |
| 23 | ++ |
| 29 | ++ |
| 30 | ++ |
| 35 | ++ |
| 40 | ++ |
| 41 | ++ |
| 47 | ++ |
| 48 | ++ |
| 49 | ++ |
| 50 | ++ |
| 51 | ++ |
| 52 | ++ |
| 53 | ++ |
| 54 | ++ |

The above experimental results show that the compound of the present invention has a good ability to bind to the Sting protein, and has a similar *in vitro* affinity with the reference compound, so the compound of the present invention can be used as an effective STING protein regulator.

### Experimental example 2. The agonistic function test of the compound of the present invention on Sting protein

### (1) Experimental method

In this experiment, the function of sting agonist was evaluated by detecting the changes of IFN-β and CXCL10 (IP10) cytokines produced by human peripheral blood mononuclear cell line THP1 cells (Shanghai Cell Bank) stimulated by the compounds. On the first day of the experiment, the ELISA plate was coated according to the IFN-β (R&D, #DY814-05) and IP10 (BD, #550926) ELISA test kit instructions. The compound was dissolved into DMSO stock solution and diluted with culture medium to a 2X working concentration, added to a 96-well plate, 100 µL per well. The THP1 cells in the logarithmic growth phase were taken to count, and were diluted to a concentration of 2* 10⁶/mL, added to the above-mentioned 96-well plate containing the compounds, with 100µL per well, mixed well, and incubated in a 37°C, 5% CO₂ incubator for 18 hours. On the second day, the above cell culture supernatant was taken, 100µL per well, and tested according to IFN-β and IP10 ELISA test kits respectively. The OD450 value was read, and converted into IFN-β and IP10 concentration according to the standard curve, and fitted with GraphPad 5.0 to calculate the EC₅₀ value of the dose-efficiency curve. EC₅₀ is the half-maximal effect concentration (concentration for 50% of maximal effect, EC₅₀), which refers to the concentration of the drug that can cause 50% of the individual to be effective.

The compound 3 reported in the article (Nature. 2018 Dec;564(7736):439-443) was used as a positive reference compound.

### (2) Experimental results

The experimental results are shown in Table 6 below, and the EC₅₀ values of each compound are classified according to the following instructions:
"+" means that the EC₅₀ value is greater than 1 µM;
"++" means that the EC₅₀ value is less than 1 µM and greater than 100 nM;
"+++" means that the EC₅₀ value is less than 100 nM and greater than 10 nM;
"++++" means that the EC₅₀ value is less than 10 nM.

**Table 7 Effects of compounds on IFN-β and CXCL10 (IP10) cytokines**

| Example | IFN-β EC₅₀ | IP10 EC₅₀ |
|---|---|---|
| reference compound | ++ | +++ |
| 1 | ++ | +++ |
| 2 | +++ | +++ |
| 3 | ++ | +++ |
| 4 | +++ | ++++ |
| 5 | +++ | ++++ |
| 6 | ++ | ++ |
| 7 | ++ | ++ |
| 8 | ++ | +++ |
| 9 | ++ | +++ |
| 10 | ++ | +++ |
| 11 | ++ | +++ |
| 12 | ND | ++ |
| 13 | ++ | +++ |
| 14 | ++ | ++ |
| 15 | +++ | +++ |
| 16 | ND | ++ |
| 17 | ++ | +++ |
| 18 | ++ | +++ |
| 19 | ++++ | ++++ |
| 20 | ++ | +++ |
| 21 | ++ | +++ |
| 22 | ++ | +++ |
| 23 | +++ | +++ |
| 29 | ++ | +++ |
| 30 | ++ | +++ |
| 35 | +++ | +++ |
| 40 | +++ | ND |
| 41 | ++ | +++ |
| 47 | + | +++ |
| 48 | ++ | +++ |
| 49 | +++ | ++++ |
| 50 | +++ | +++ |
| 51 | +++ | ++++ |
| 52 | +++ | ++++ |
| 53 | +++ | ++++ |

In Table 7, "ND" means that the test has not been performed yet.

The above experimental data show that the compounds of the present invention have good stimulating activity of IFN-β and CXCL10 (IP10) cytokines produced by THP1 cells, and have a good STING protein agonistic function. Especially for compounds 2, 4, 5, 15, 19, 23, 35, 40, 49, 50, 51, 52, 53, etc., compared with the reference compound, the compounds of the present invention have comparable or even higher activity of stimulating the immune cells to produce cytokines.

### Experimental example 3: IFN-β induction experiment on mice in vivo

### (1) Experimental method

Balb/C mice were inoculated with 5×10⁵ CT26 cells subcutaneously on the back. After inoculation, tumor-bearing mice with tumor volume in the range of 200-300 mm³ were selected for the experiment. According to the random method of tumor size and segment, they were divided into 21 groups with 3 mice in each group. Different doses of drugs (compounds of the present invention) were given via tail vein injection, and the control group was given a control vehicle (5% DMSO, 40% PEG400 formulated in physiological saline). Three hours after the administration, blood was taken from the mouse orbital vein, and EDTA•2K was used as an anticoagulant. The collected mouse whole blood was centrifuged at 8000g for 5 minutes and then the upper plasma was taken for testing. Three hours after administration, mouse tumor tissues were taken, weighed and homogenized by adding 9 times volume of PBS. After centrifuging the homogenate at 10,000 rpm for 15 minutes, the supernatant was collected for testing. The detection was performed according to the IFN-β detection kit (R&D, #DY814-05), the OD450 value was read, which was converted to the IFN-β concentration according to the standard curve.

### (2) Experimental results

Experimental results show that the tail vein administration of mice can induce the release of IFN-β downstream of the STING pathway in blood and tumors. After administration of the compound of the present invention, the intratumor IFN-β level was more than 3 times that of the plasma IFN-β level, indicating that cytokines were relatively enriched in tumor tissues, and blood cytokines were low, suggesting a higher treatment window.

### Example 4. Tumor inhibitory effect of the compound of the present invention (CT26 tumor model)

### (1) Experimental method

After Balb/c mice rested for one week, CT26 cells were inoculated subcutaneously on the back, and each mouse was inoculated with 100 µL cell suspension containing 5×10⁵ cells. When the average tumor volume grew to about 120 mm³, they were randomly divided into groups according to the size of the tumor, with 5 in each group. The mice in each group were administered intravenously through the tail vein on Day 1, 4, and 8 after grouping, and the control group was given a control vehicle (5% DMSO, 40% PEG400 formulated in physiological saline). On the day of grouping, 3 times a week after the first administration, and before euthanasia, the length and short diameter of the tumor were measured and recorded with a vernier caliper, the tumor volume was calculated, and the tumor growth curve was drawn according to the tumor volume. The tumor volume was calculated according to the following formula: V=1/2×long diameter×short diameter×short diameter.

### (2) Experimental results

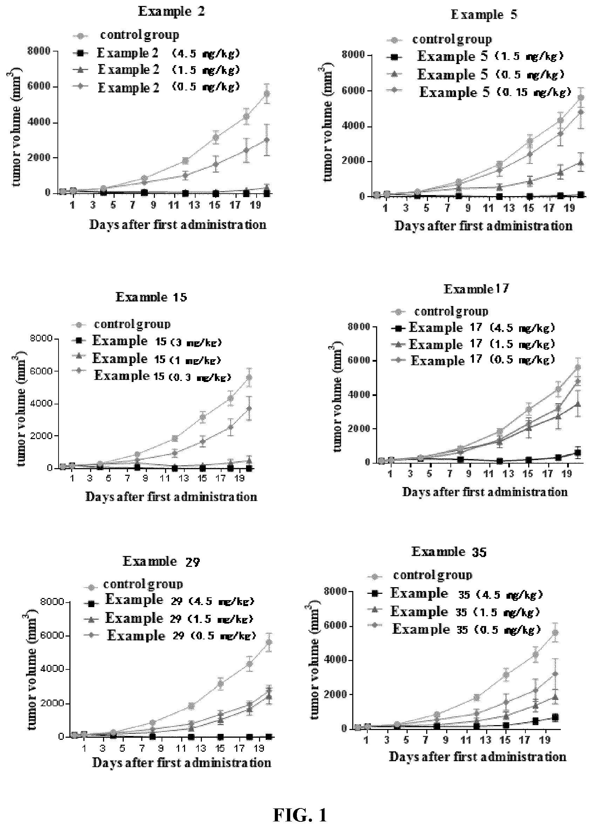

The experimental results are shown in Figure 1. After 20 days of administration, the compounds prepared in the examples of the present invention all effectively inhibited tumor growth and exhibited a dose-dependent tumor growth inhibitory effect.

### Example 5. Tumor inhibitory effect of the compounds of the present invention (H22 tumor model)

### (1) Experimental method

Kunming rats were inoculated with H22 cells subcutaneously on their backs after a week of resting. Each mouse was inoculated with 100 µL of cell suspension containing 5×10⁶ cells. When the average tumor volume grew to about 200 mm³, they were randomly grouped according to the tumor size, with 7 in each group. On the Day 1, 4 and 8 after grouping, the mice in the high, medium and low dose groups were administered intravenously via the tail vein (the compounds of the present invention), or on the Day 1, 8, and 15 the mice in the high dose group were administered intravenously via the tail vein. The control group was given a control vehicle (5% DMSO, 40% PEG400 formulated in physiological saline). On the day of grouping, 3 times a week after the first administration, and before euthanasia, the length and short diameter of the tumor were measured and recorded with a vernier caliper, the tumor volume was calculated, and the tumor growth curve was drawn according to the tumor volume. The tumor volume was calculated according to the following formula: V=1/2×long diameter×short diameter×short diameter.

### (2) Experimental results

After 30 days of administration, the compounds of the present invention all effectively inhibited tumor growth, and exhibited a dose-dependent tumor growth inhibitory effect.

### Example 6. Tumor inhibitory effect of the compounds of the present invention (tumor re-excitation model)

### (1) Experimental method

The tumor regression of each group of mice in the CT26 anti-tumor efficacy model of the above experimental example 4 was further observed. After 30 days of the last administration, CT26 cells were inoculated subcutaneously on the back. Each mouse was inoculated with 100 µL cell suspension containing 5×10⁵ cells; mice that have not been treated with drugs were used as a control group to inoculate CT26 cells in the same way. The tumor was observed and the growth of the tumor was recorded.

### (2) Experimental results

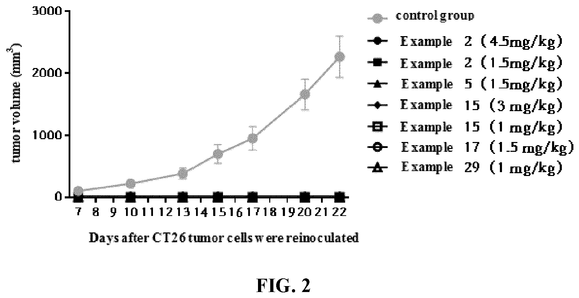

The experimental results are shown in Figure 2. The CT26 tumors in the control group can grow after inoculation, but the tumors in the mice whose tumors disappeared completely after treatment with the compounds of the present invention cannot grow again after inoculation, indicating that these compounds activated the immune memory mechanism of the mice, and render mice had immunity to tumor cells that were re-inoculated.

In summary, the present invention discloses a compound which is capable of effectively binding to STING, has a good STING protein agonistic function, has a good inhibitory effect on a variety of tumors, and can also activate immune memory mechanism in mice, to inhibit tumor restimulation. Therefore, the compounds of the present invention can be used as STING agonists and used to treat various related diseases. The compounds of the present invention have very good application prospects in the manufacture of a medicament for the treatment of diseases related to STING activity (especially a medicament for the treatment of inflammatory diseases, allergic diseases, autoimmune diseases, infectious diseases, cancer or precancerous syndromes) and in the manufacture of immune adjuvants, thus provides a new choice for clinical screening and/or manufacture of a medicament for diseases related to STING activity.

## Claims

1. A compound, or the stereoisomer, or the pharmaceutically acceptable salt thereof, wherein the compound is represented by Formula II, 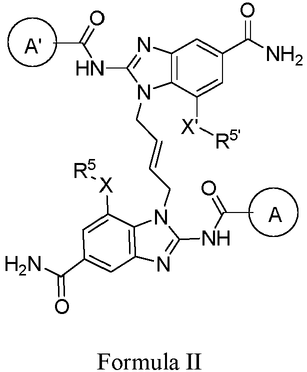 wherein X is selected from -O- and -S-, and X' is -O-;
A ring and A' ring are each independently selected from 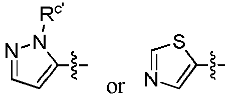 optionally substituted with 0-2 R^{c}; and when X and X' are both -O-, at least one of A ring and A' ring is selected from 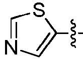 optionally substituted with 0-2 R^{c};
R⁵ is selected from C₁-C₃ alkyl;
R^{5'} is selected from 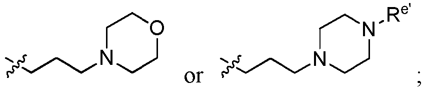
R^{e'} is selected from C₁-C₃ alkyl, 3-6 membered cycloalkyl, -C(O)R^{f};
R^{f} is selected from -OR^{a'}, C₁-C₃ alkyl, 3 to 4-membered cycloalkyl, and R^{a'} is selected from C₁-C₃ alkyl; and
wherein
the 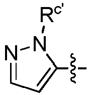 optionally substituted with 0 to 2 R^{c} is 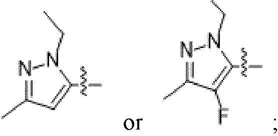 and
the 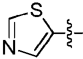 optionally substituted by 0-2 R^{c} is

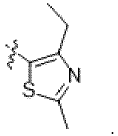

2. A compound, or the stereoisomer, or the pharmaceutically acceptable salt thereof, wherein the compound is:

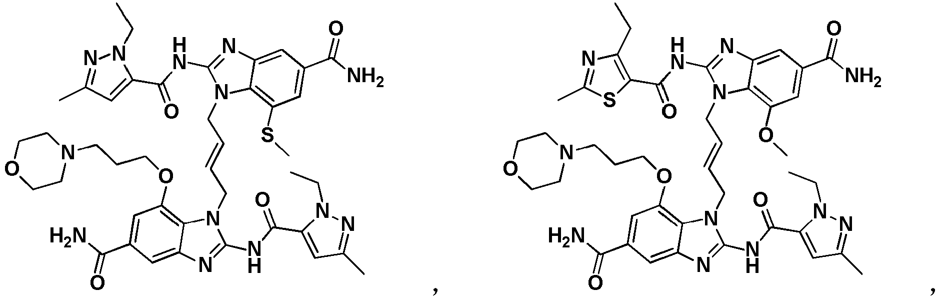

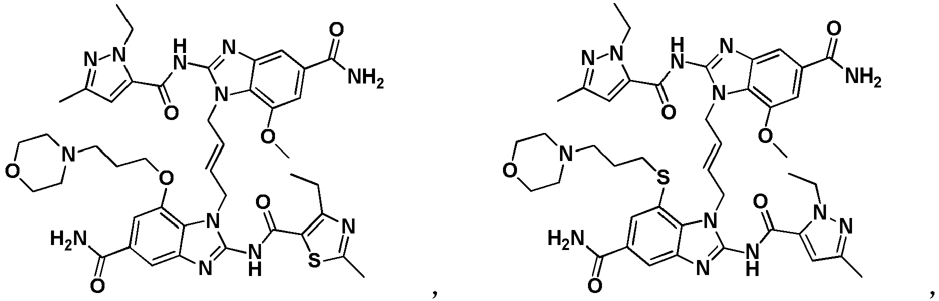

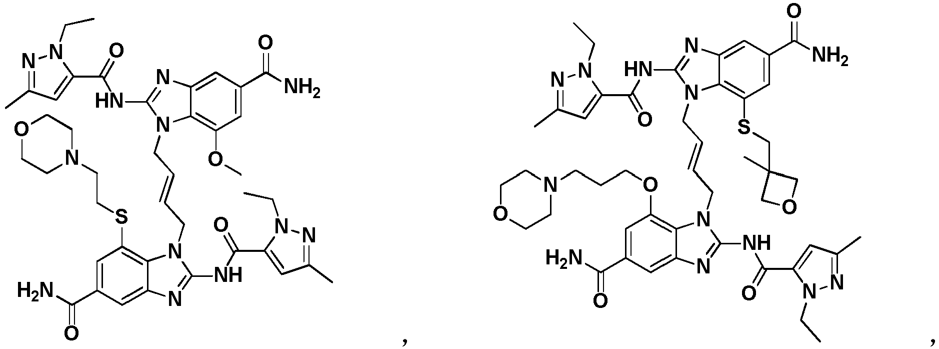

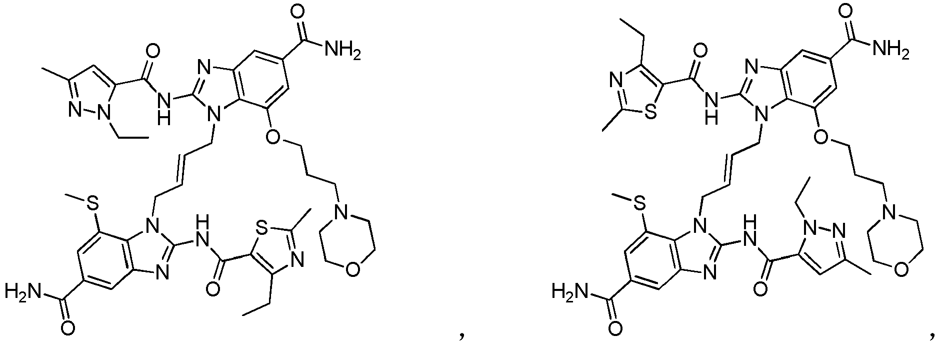

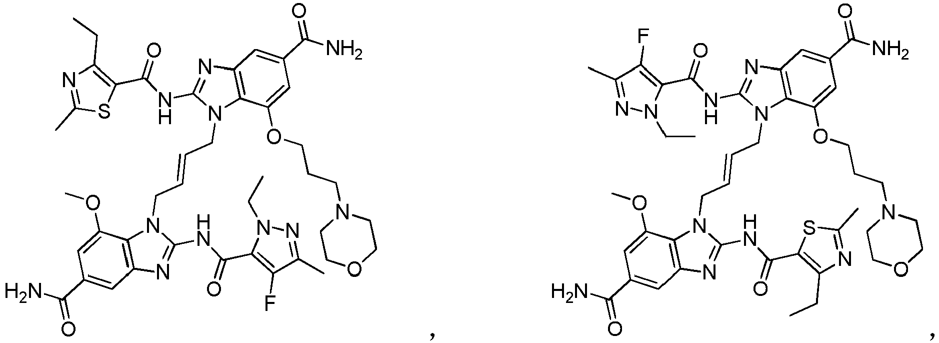

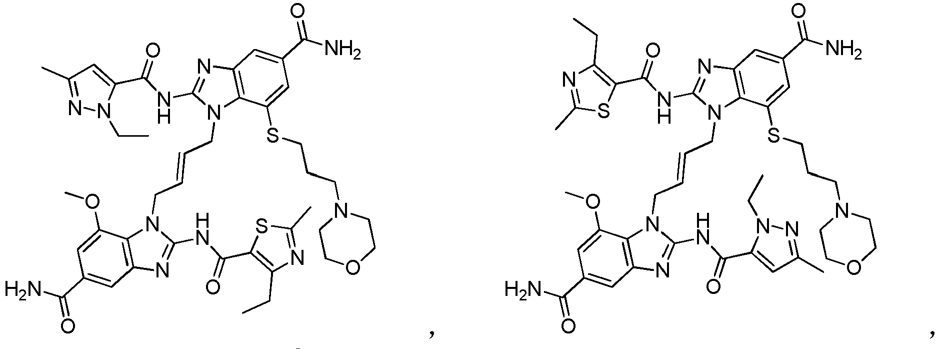

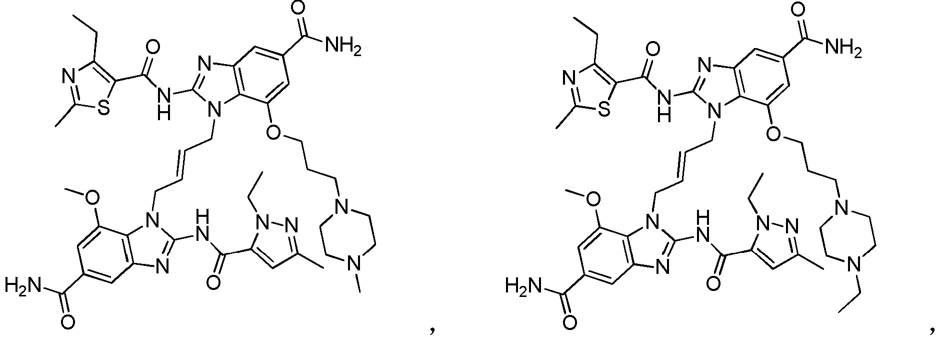

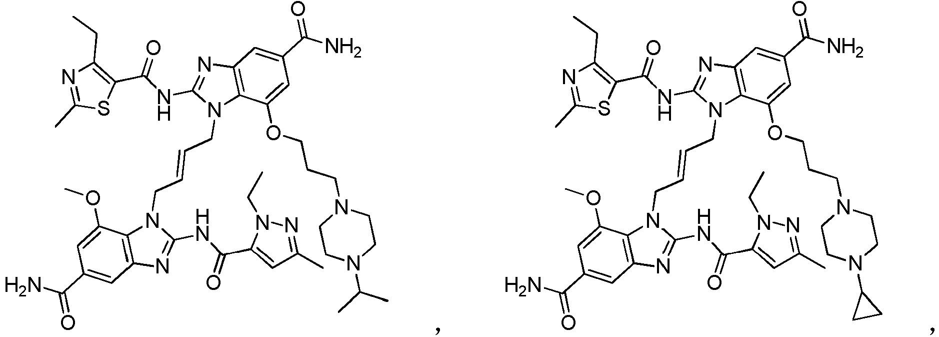

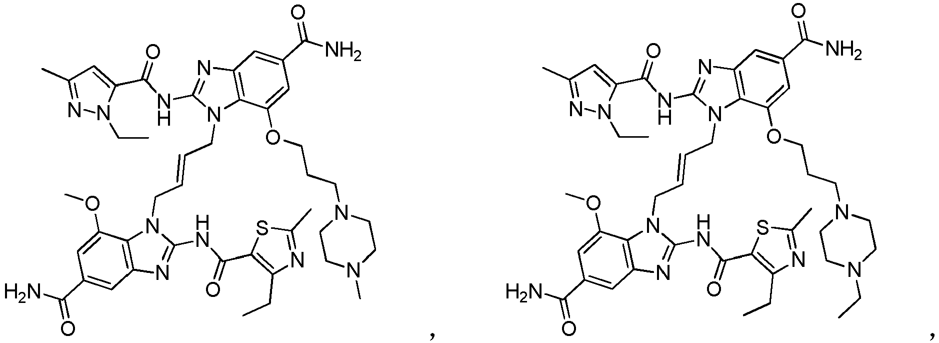

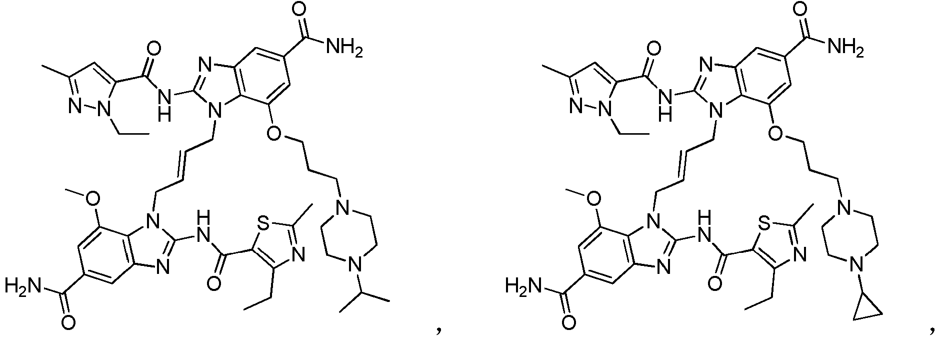

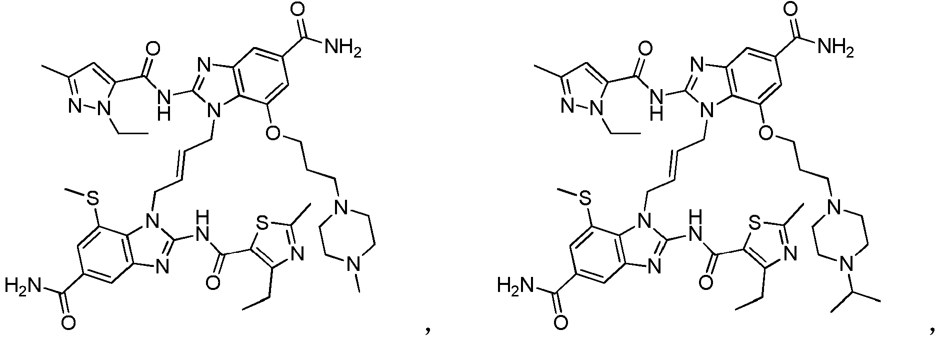

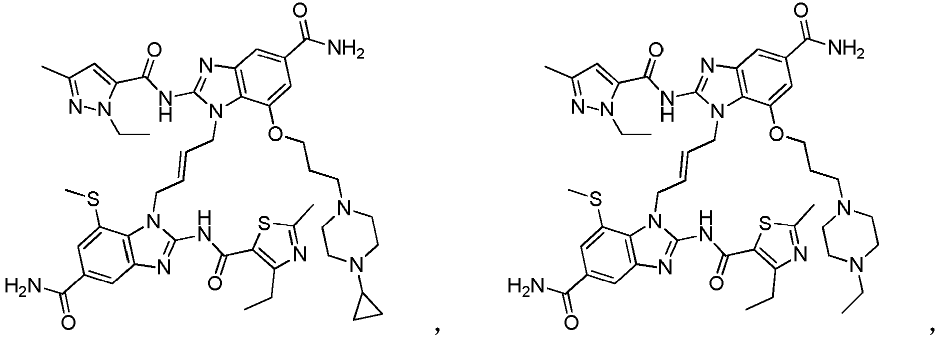

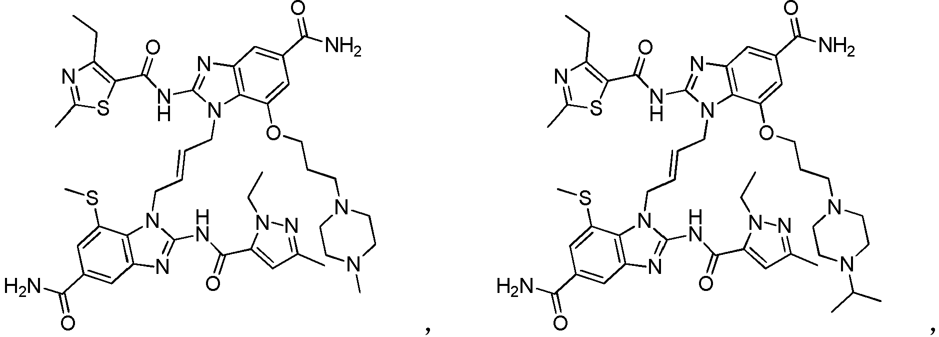

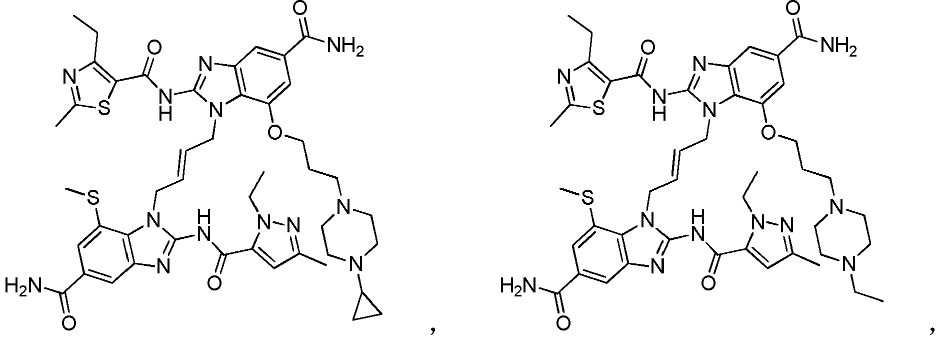

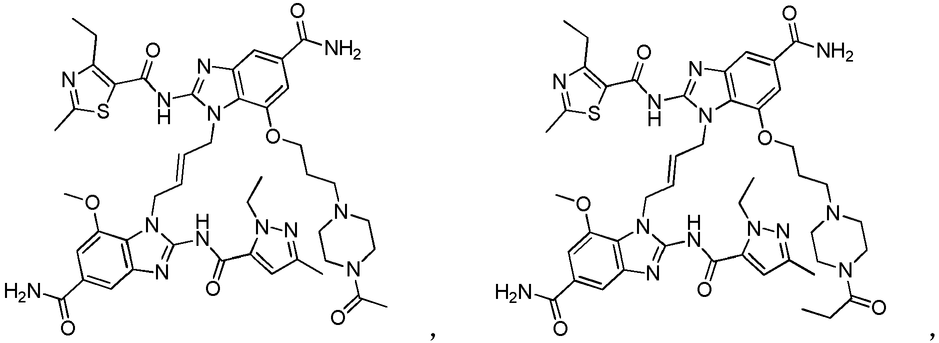

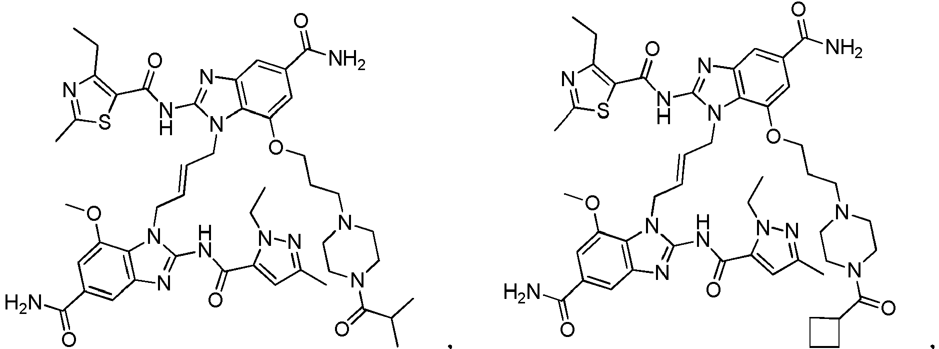

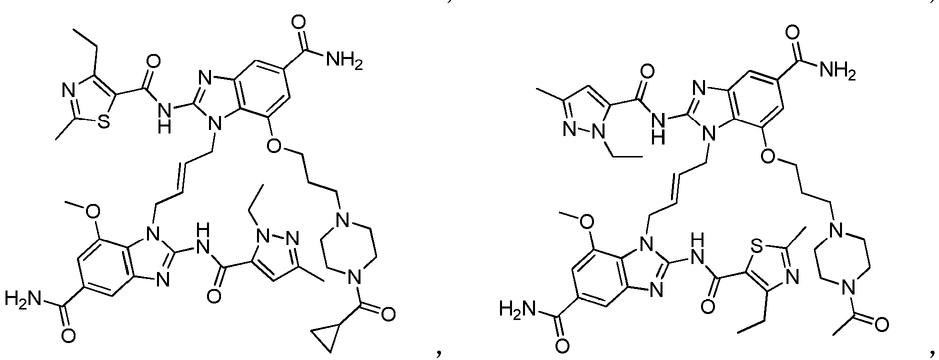

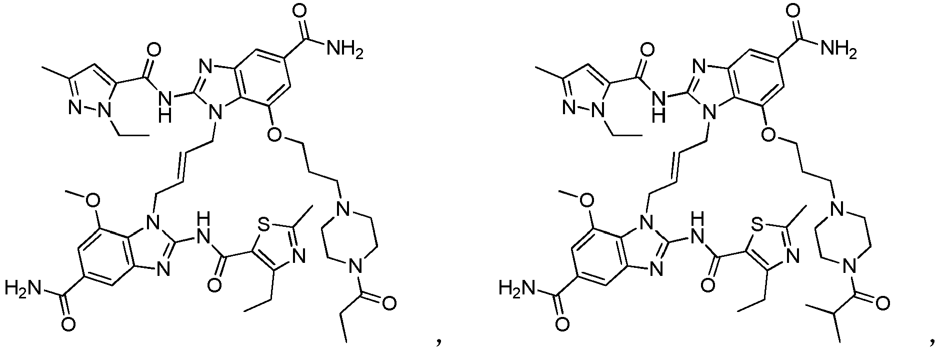

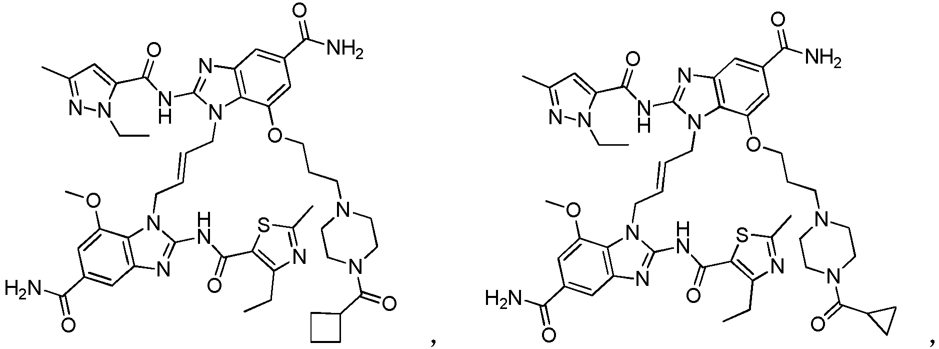

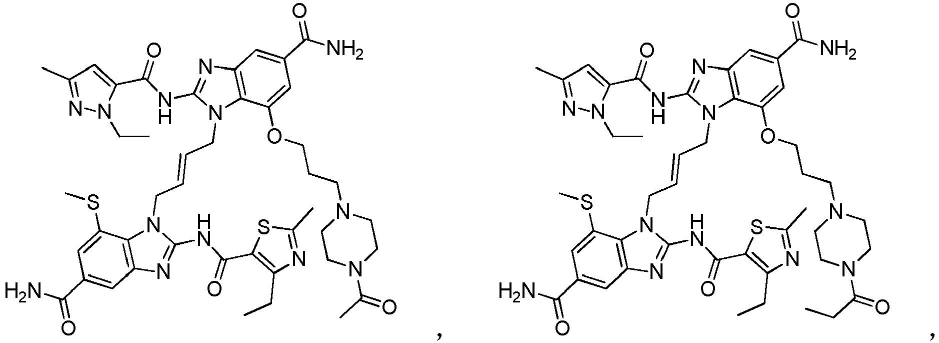

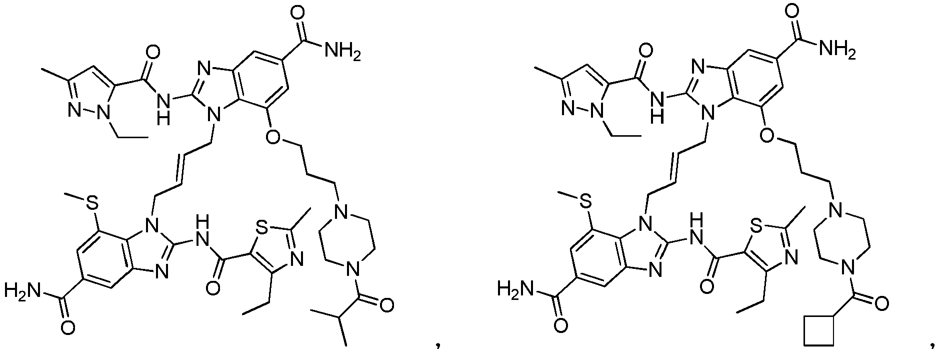

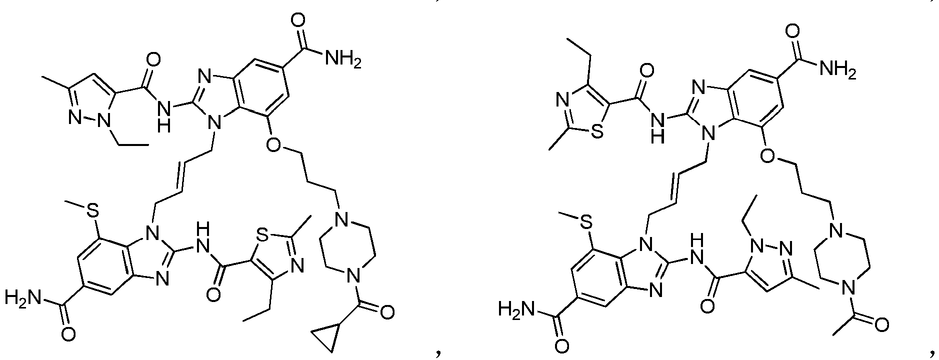

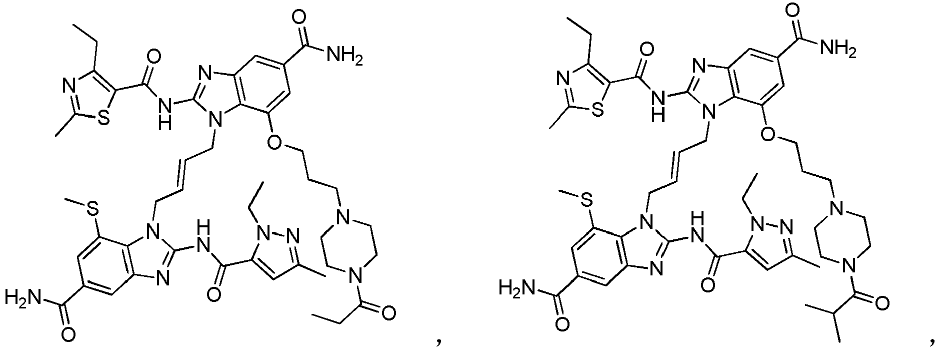

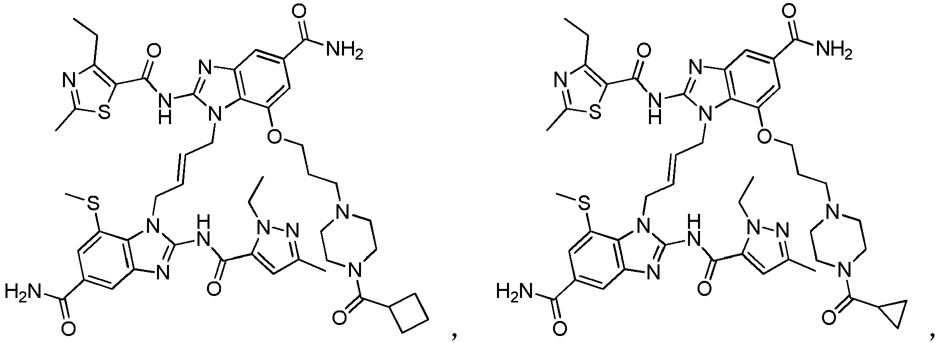

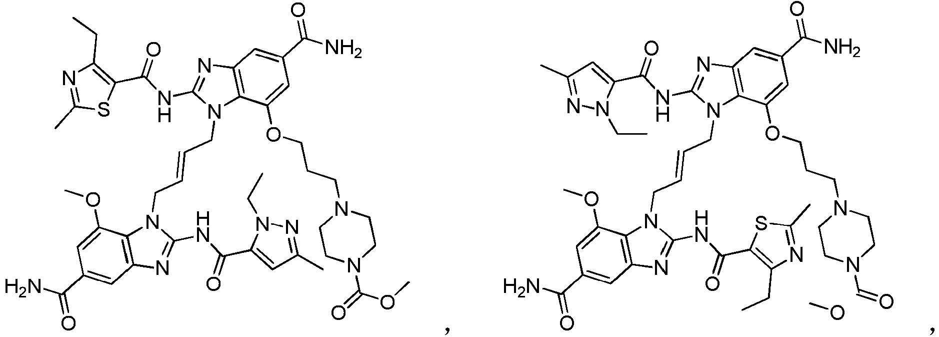

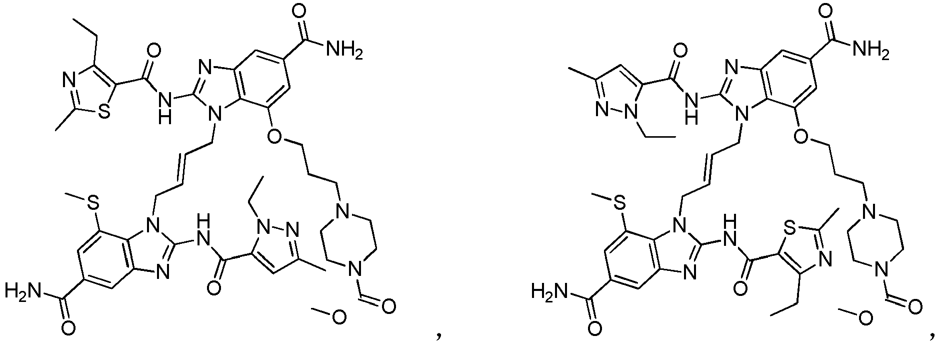

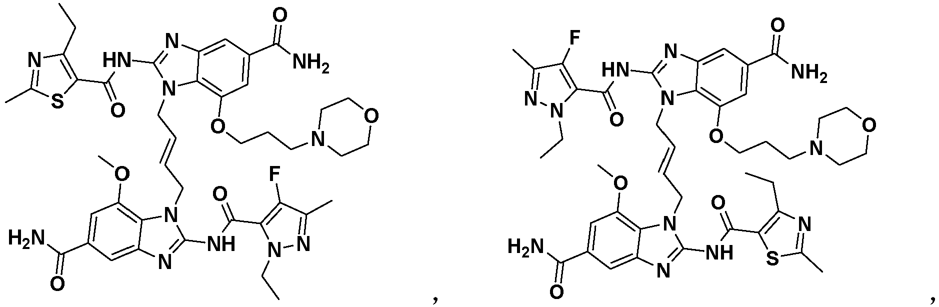

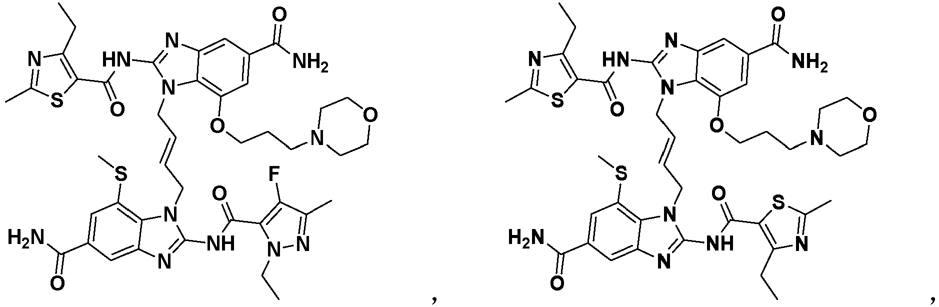

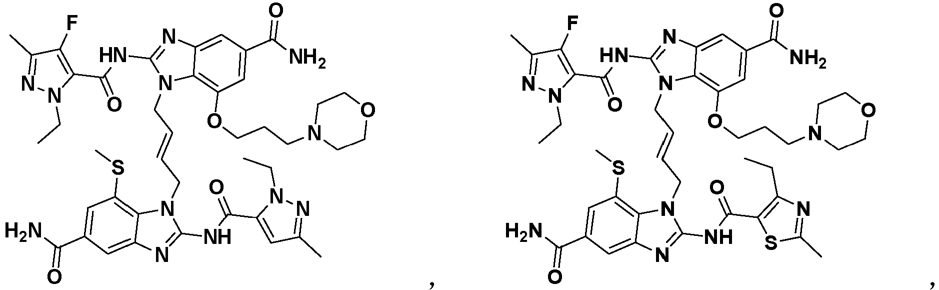

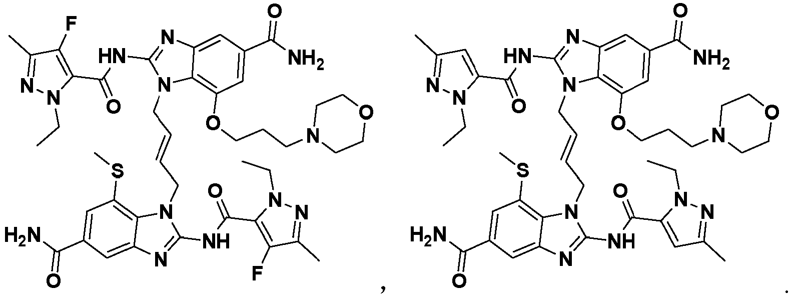

3. A compound, or the stereoisomer, or the pharmaceutically acceptable salt thereof, wherein the compound is represented by formula III: 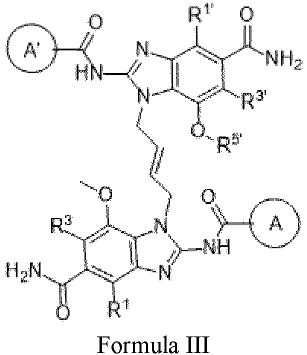 wherein R¹, R³, R^{1'}, and R^{3'} are hydrogen;
wherein A ring and A' ring are independently selected from 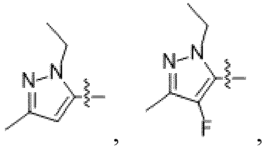 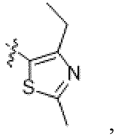 respectively, and at least one of A ring and A' ring is 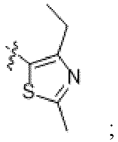
R^{5'} is selected from C₁-C₃ alkyl optionally substituted by 0-2 R^{d};
R^{d} is independently selected from

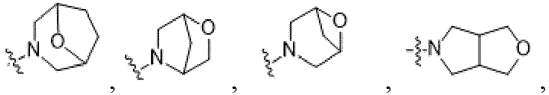

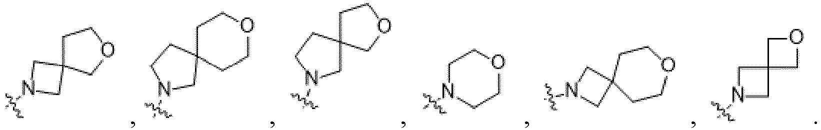

4. The compound, or the stereoisomer, or the pharmaceutically acceptable salt thereof, wherein the compound is:

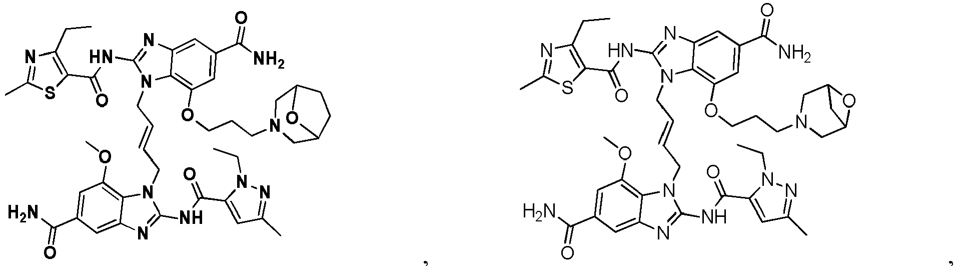

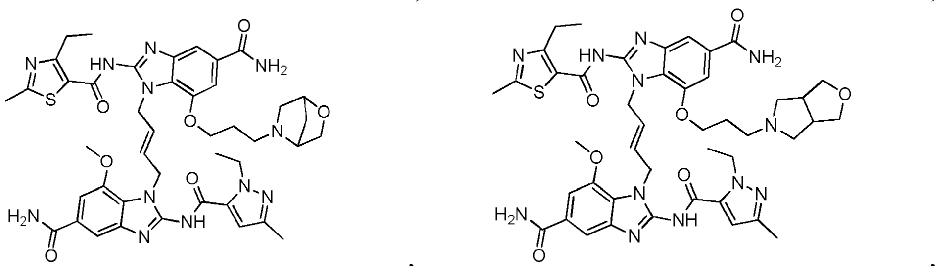

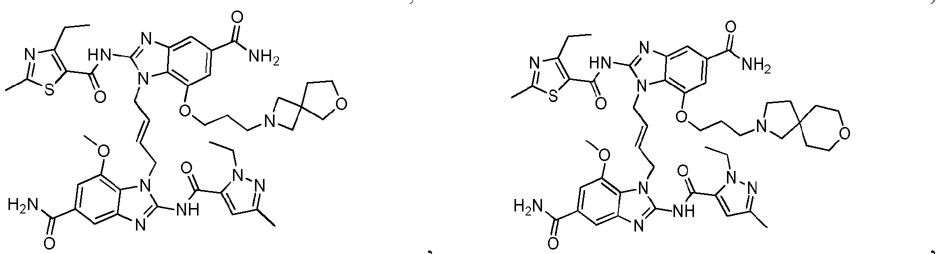

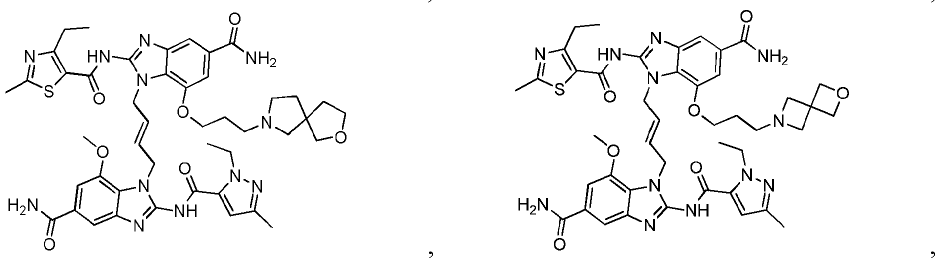

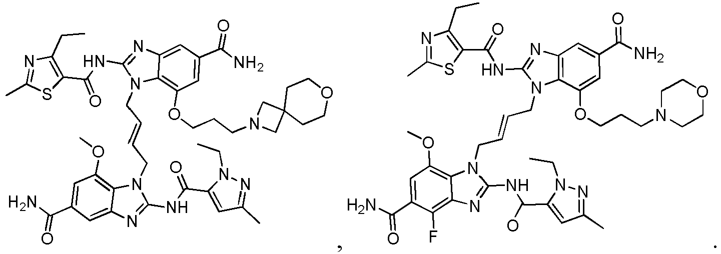

5. The compound, or the stereoisomer, or the pharmaceutically acceptable salt thereof according to any one of claims 1 to 4 for use in the treatment of a disease related to STING activity;
wherein the disease related to STING activity is any one of diseases related to inflammatory diseases, autoimmune diseases, infectious diseases, cancer, and precancerous syndrome or a combination thereof.

6. The compound, or the stereoisomer, or the pharmaceutically acceptable salt thereof according to any one of claims 1 to 4 for use as an immune adjuvant.

7. A medicine, which is a formulation prepared with the compound, or the stereoisomer, or the pharmaceutically acceptable salt thereof according to any one of claims 1 to 4 as an active ingredient, plus pharmaceutically acceptable excipients.

## Patentansprüche

1. Verbindung oder das Stereoisomer oder das pharmazeutisch verträgliche Salz davon, wobei die Verbindung durch die Formel II dargestellt ist, 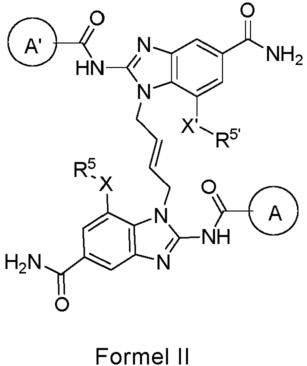 wobei X aus -O- und -S- ausgewählt ist und X' -O- ist;
Ring A und Ring A' jeweils unabhängig voneinander ausgewählt sind aus 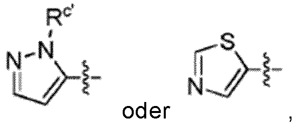 optional substituiert durch 0-2 R^{c}; und wobei, wenn X und X' beide -O- sind, mindestens einer von Ring A und Ring A' ausgewählt ist aus 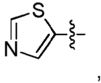 optional substituiert durch 0-2 R^{c};
R⁵ ausgewählt ist aus C₁-C₃-Alkyl;
R^{5'} ausgewählt ist aus 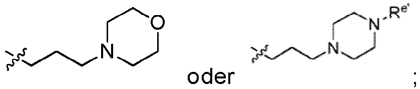
R^{e'} ausgewählt ist aus C₁-C₃-Alkyl, 3-6-gliedrigem Cycloalkyl, -C(O)R^{f};
R^{f} ausgewählt ist aus -OR^{a'}, C₁-C₃-Alkyl, 3- bis 4-gliedrigem Cycloalkyl, und R^{a'} ausgewählt ist aus C₁-C₃-Alkyl; und
wobei
das 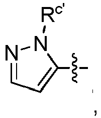 optional substituiert durch 0 bis 2 R^{c}, 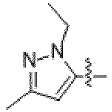 oder 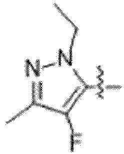 ist; und
das 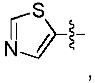 optional substituiert durch 0-2 R^{c}, 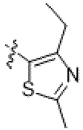 ist.

2. Verbindung oder das Stereoisomer oder das pharmazeutisch verträgliche Salz davon, wobei die Verbindung Folgendes ist:

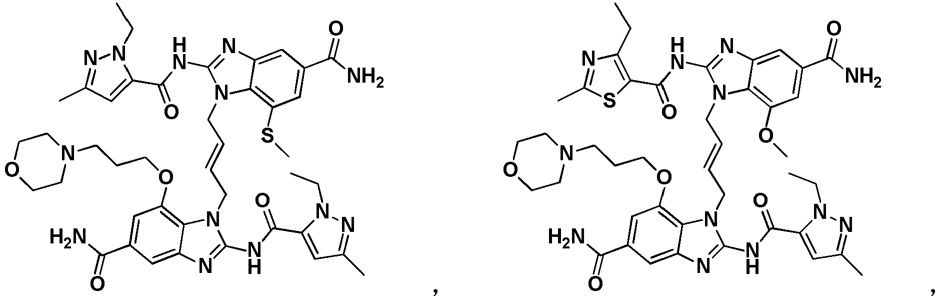

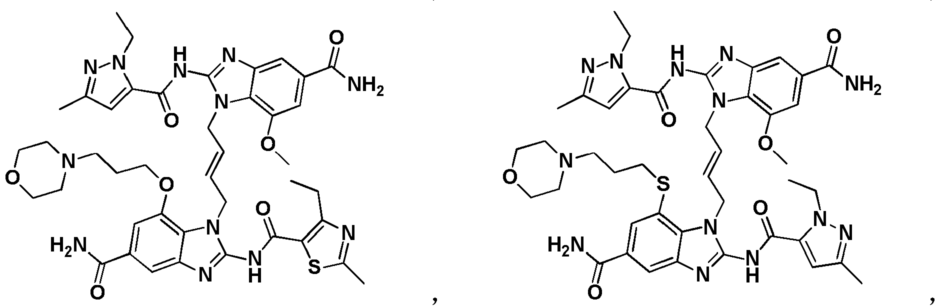

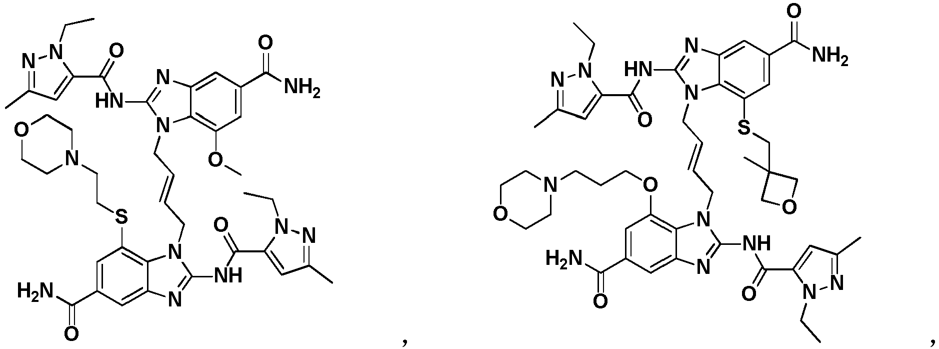

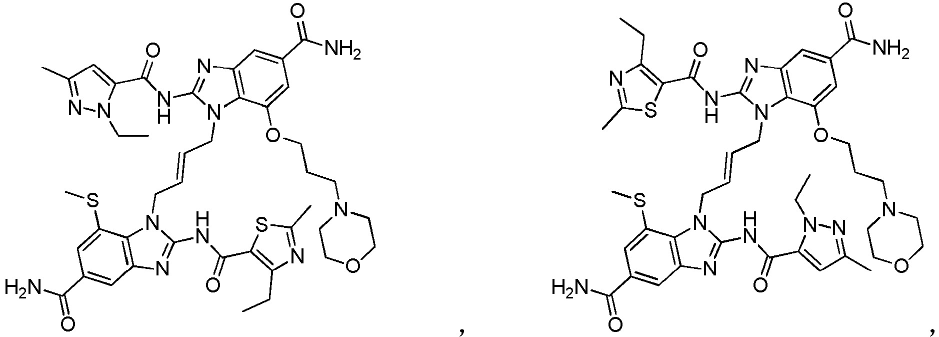

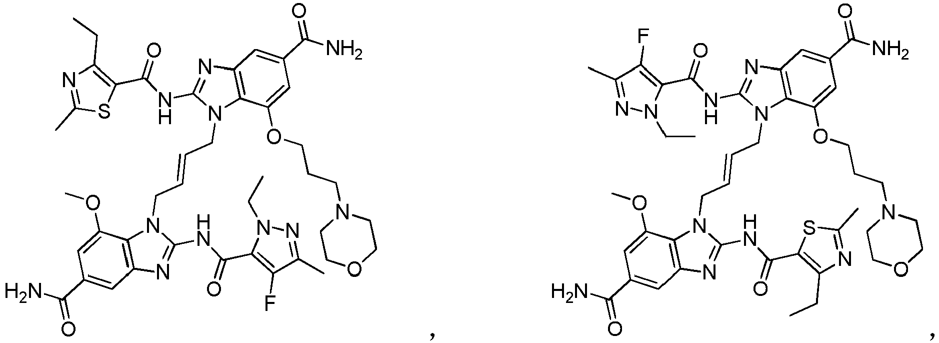

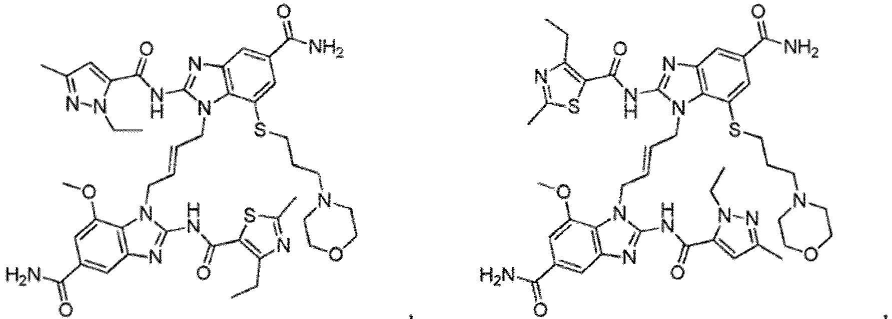

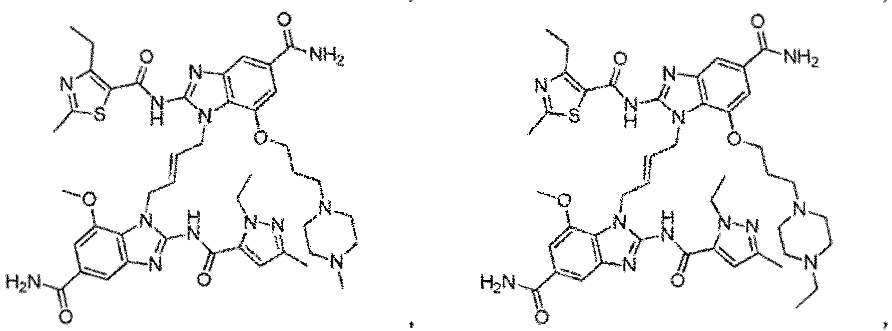

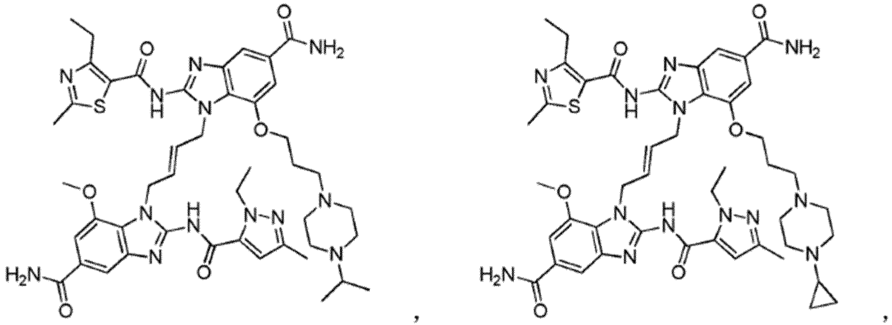

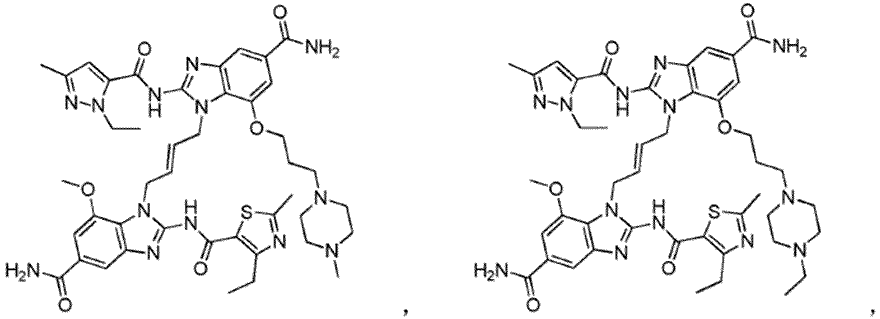

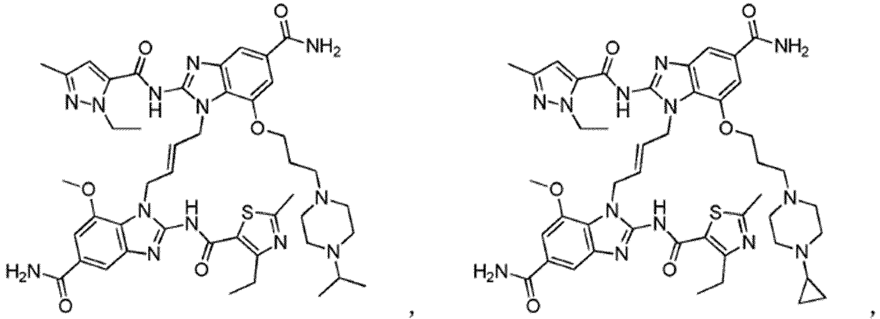

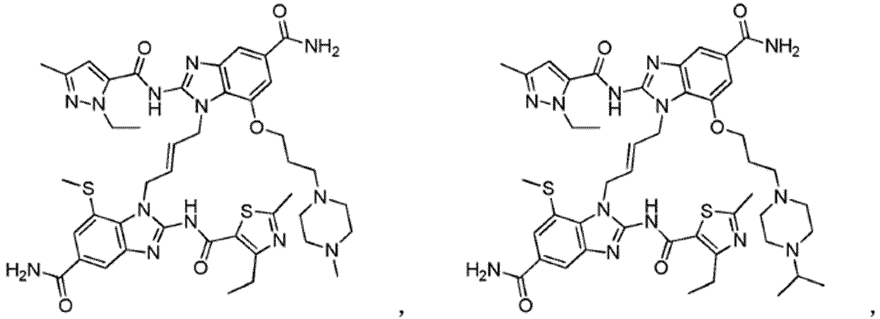

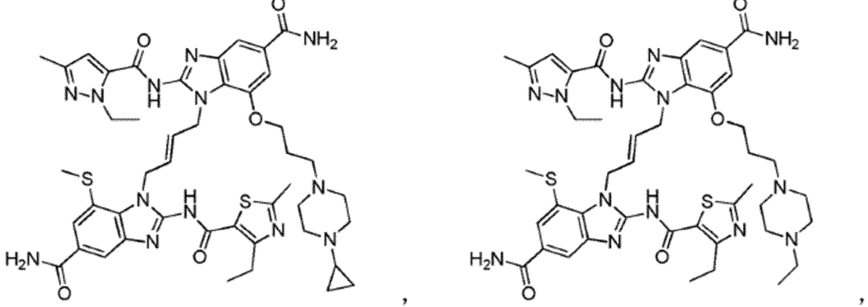

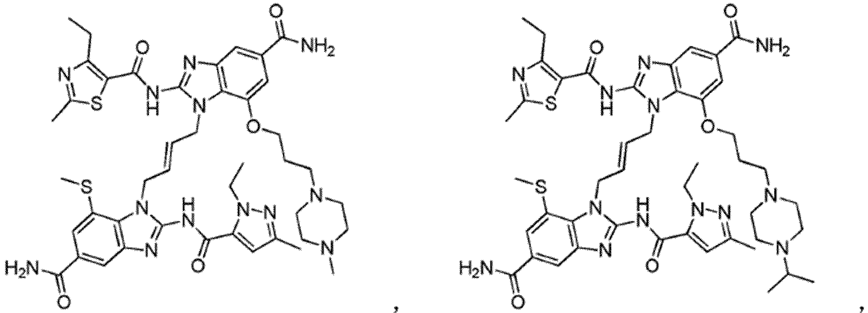

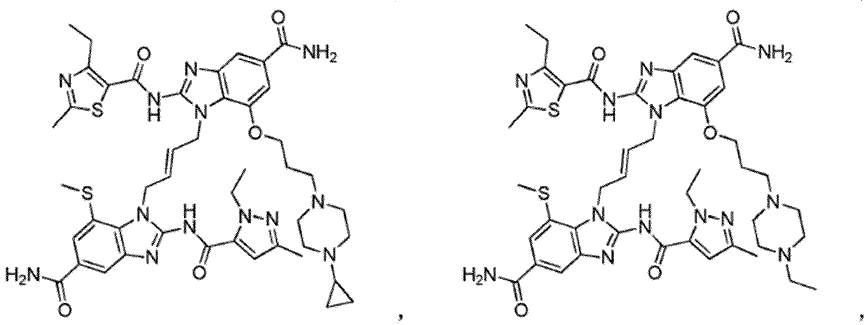

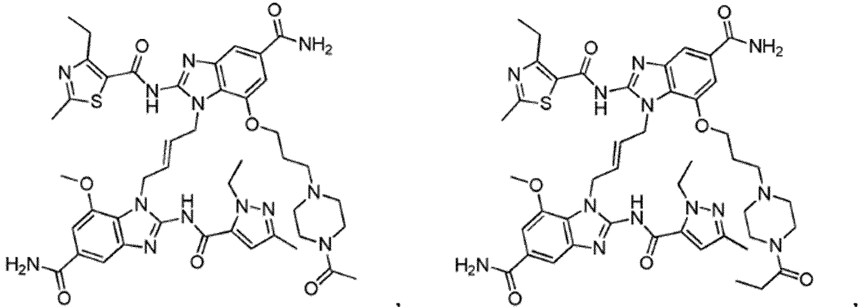

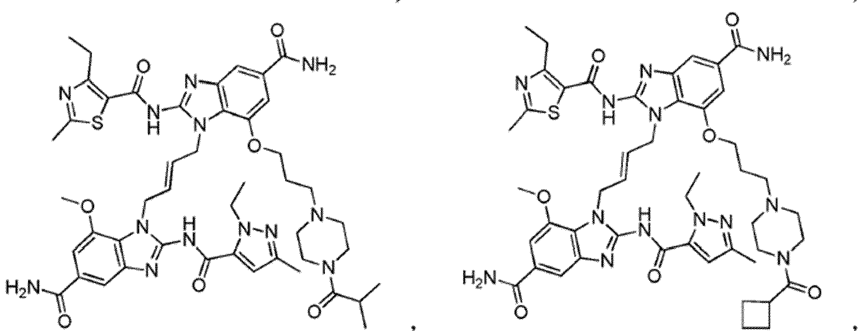

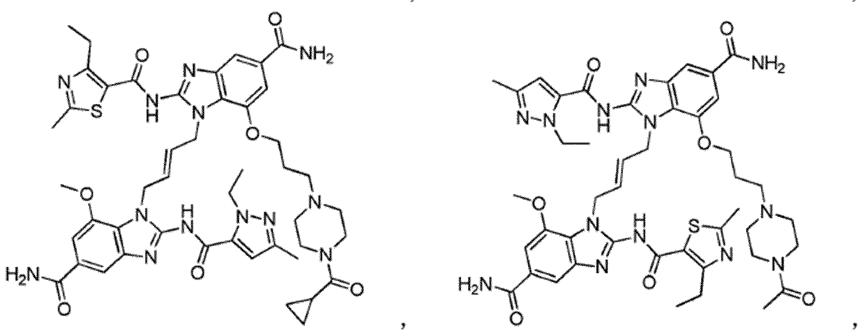

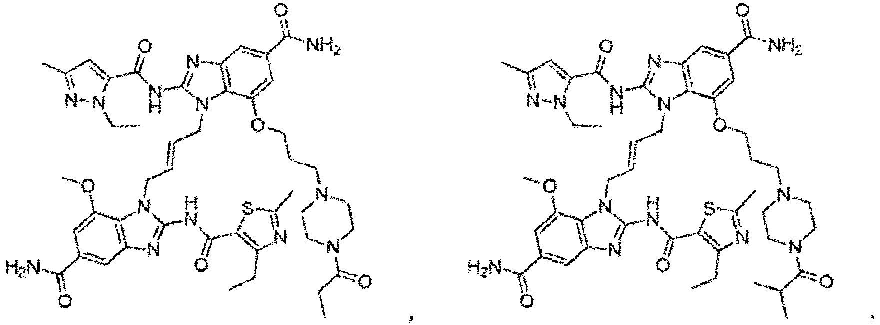

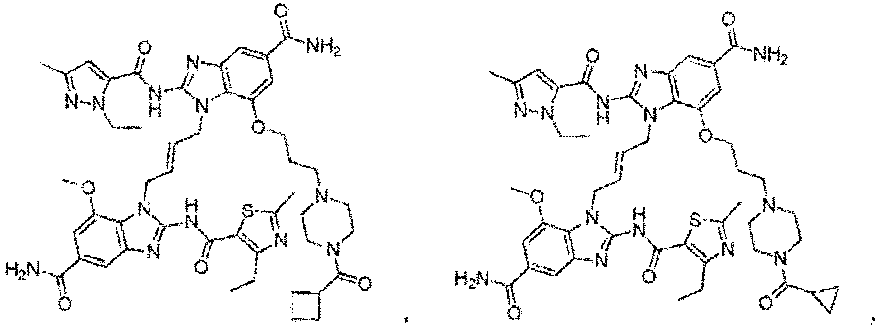

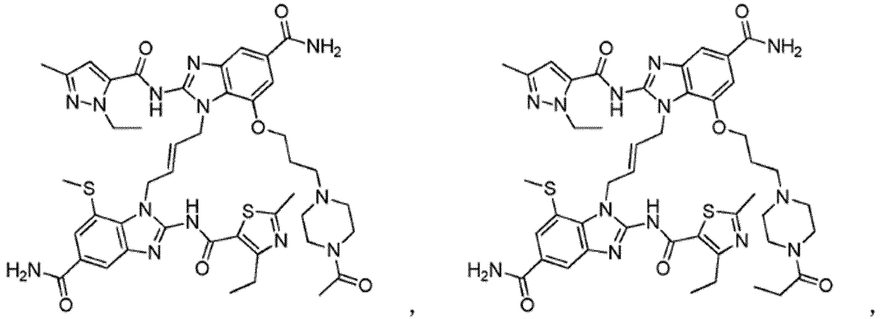

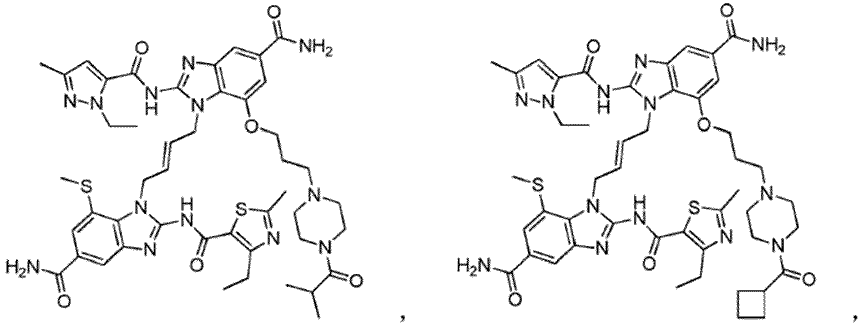

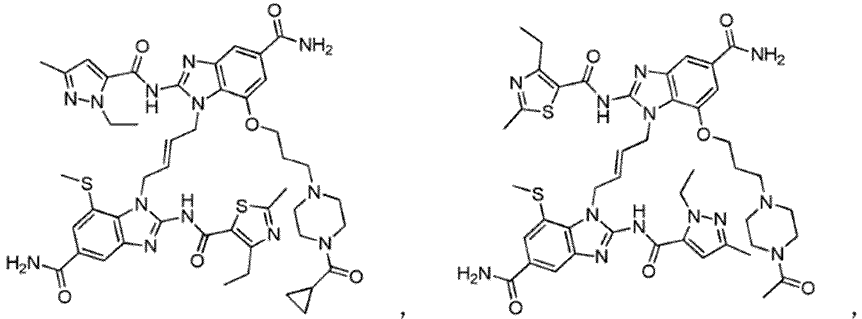

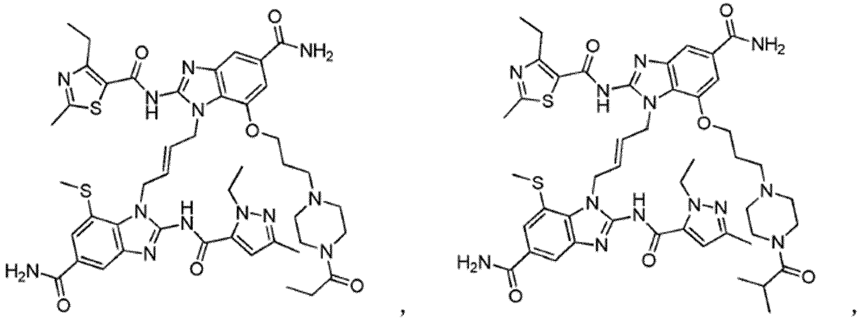

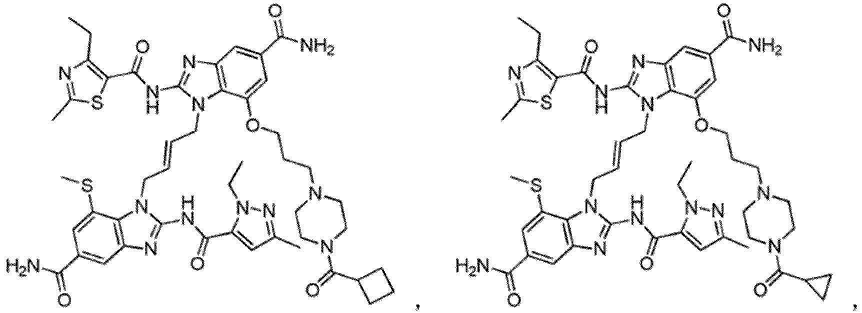

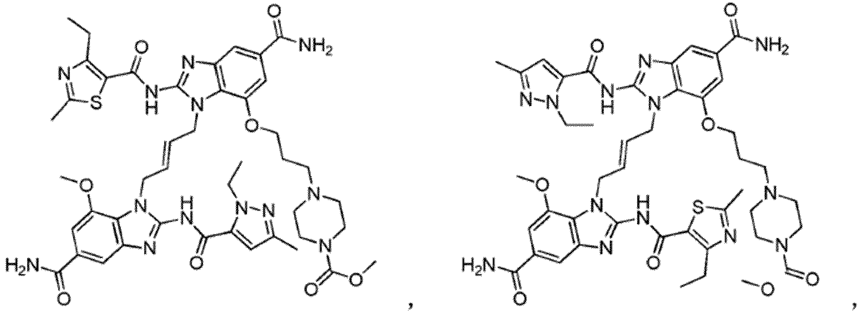

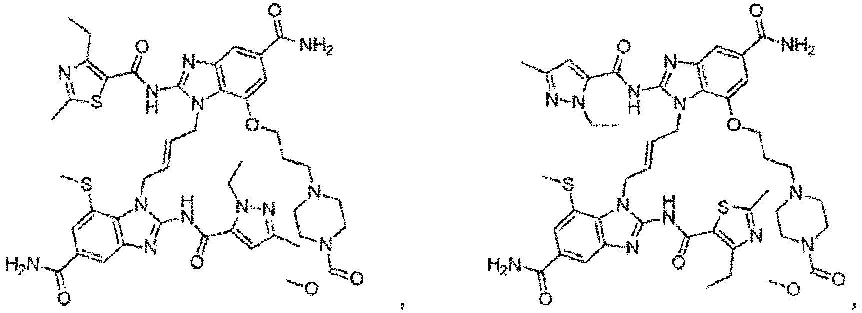

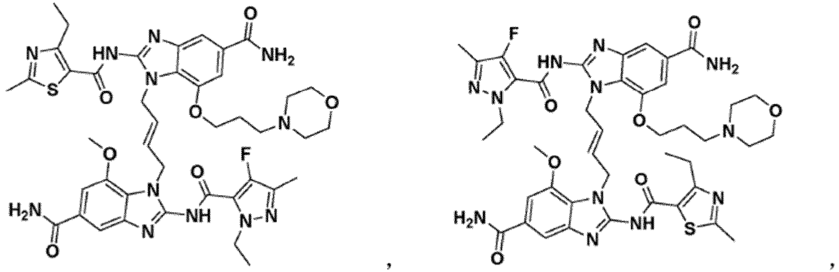

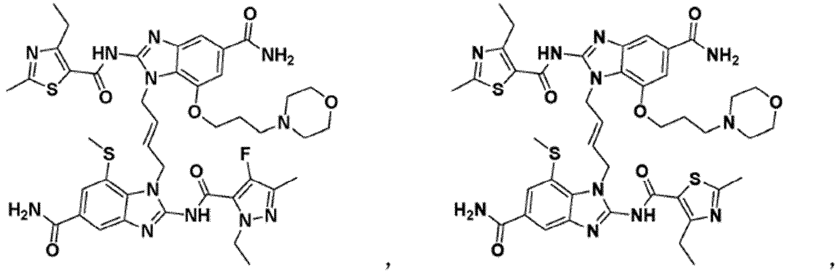

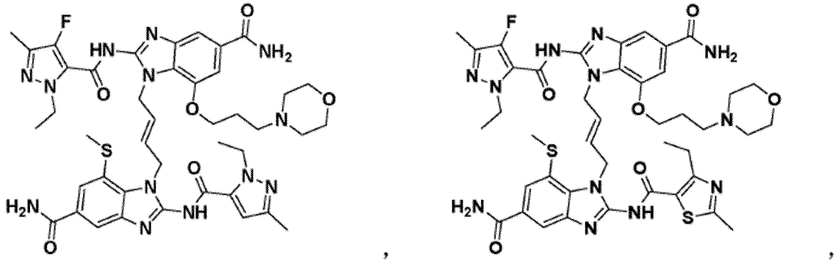

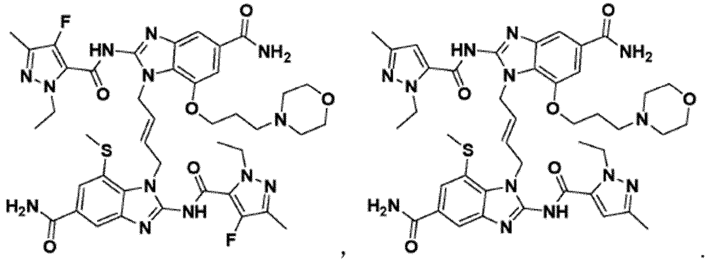

3. Verbindung oder das Stereoisomer oder das pharmazeutisch verträgliche Salz davon, wobei die Verbindung durch die Formel III dargestellt ist: 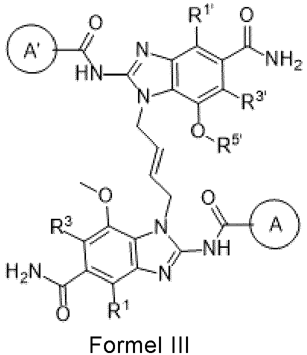 wobei R¹, R³, R^{1'} und R^{3'} Wasserstoff sind;
wobei Ring A und Ring A' jeweils unabhängig voneinander ausgewählt sind aus 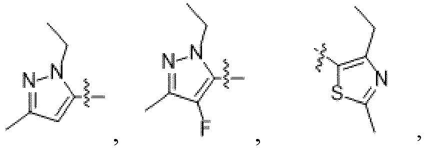 und mindestens einer von Ring A und Ring A' 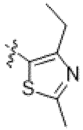 ist;
R^{5'} ausgewählt ist aus C₁-C₃-Alkyl, optional substituiert durch 0-2 R^{d};
Rd unabhängig ausgewählt ist aus

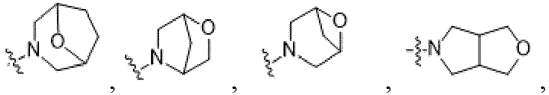

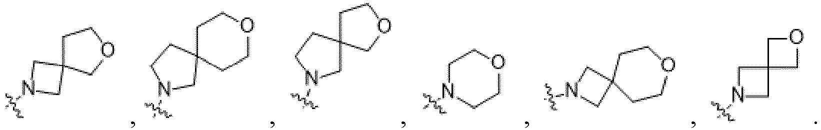

4. Verbindung oder das Stereoisomer oder das pharmazeutisch verträgliche Salz davon, wobei die Verbindung Folgendes ist:

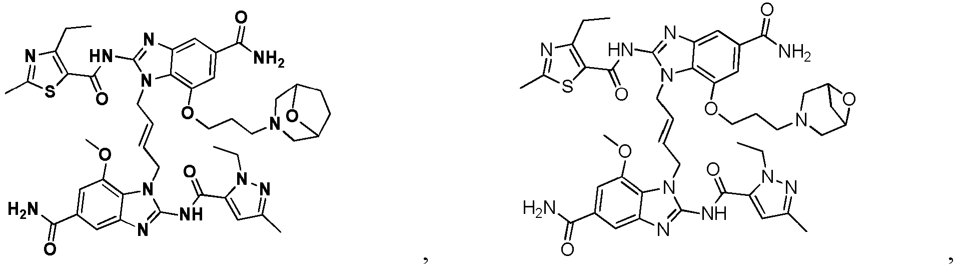

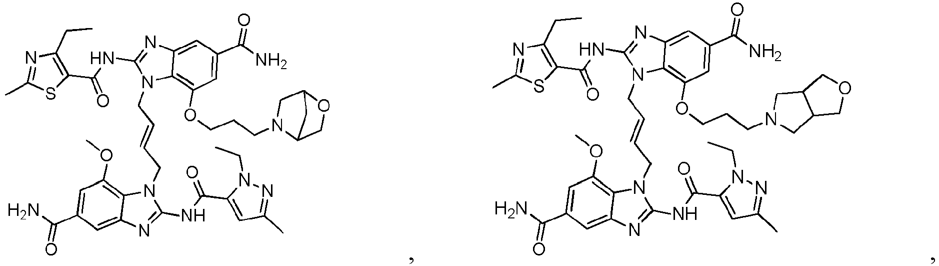

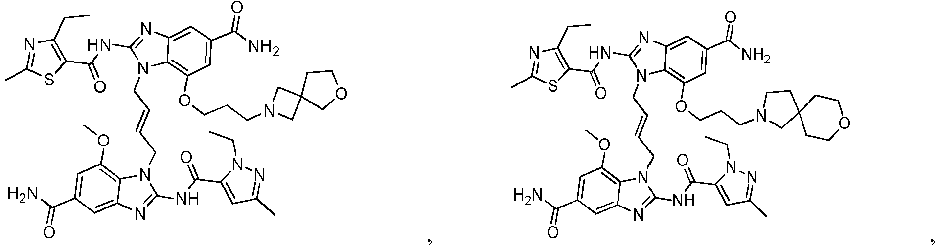

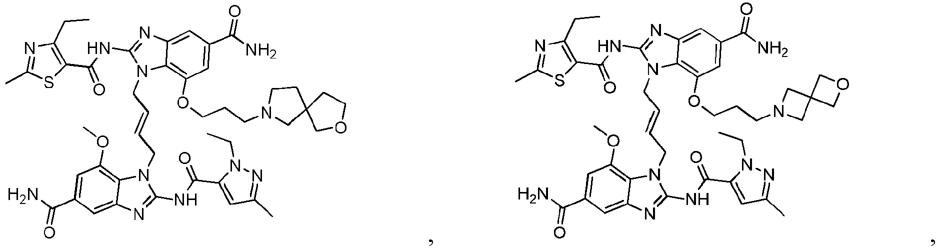

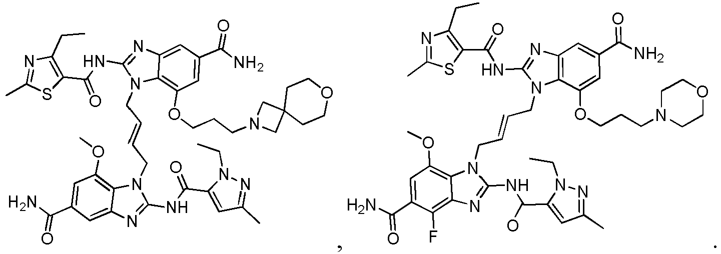

5. Verbindung oder das Stereoisomer oder das pharmazeutisch verträgliche Salz davon nach einem der Ansprüche 1 bis 4 zur Verwendung bei der Behandlung einer Krankheit, die mit der STING-Aktivität in Zusammenhang steht;
wobei die mit der STING-Aktivität in Zusammenhang stehende Krankheit eine der Krankheiten ist, die mit Entzündungskrankheiten, Autoimmunerkrankungen, Infektionskrankheiten, Krebs und präkanzerösen Syndromen oder einer Kombination davon in Zusammenhang stehen.

6. Verbindung oder das Stereoisomer oder das pharmazeutisch verträgliche Salz davon nach einem der Ansprüche 1 bis 4 zur Verwendung als Immunadjuvans.

7. Arzneimittel, das eine Formulierung ist, die mit der Verbindung oder dem Stereoisomer oder dem pharmazeutisch verträglichen Salz davon nach einem der Ansprüche 1 bis 4 als Wirkstoff sowie pharmazeutisch verträglichen Hilfsstoffen hergestellt ist.

## Revendications

1. Composé, ou stéréoisomère de celui-ci, ou sel pharmaceutiquement acceptable de celui-ci, dans lequel le composé est représenté par la Formule II, 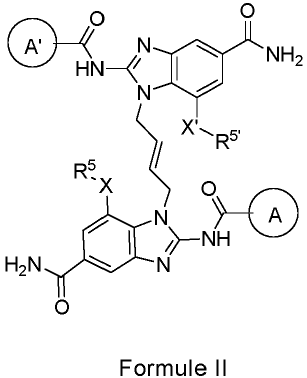 dans lequel X est choisi parmi -O- et -S-, et X' est -O- ;
le cycle A et le cycle A' sont chacun indépendamment choisis parmi 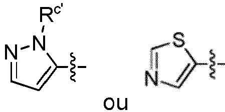 facultativement substitué par 0-2 R^{c} ; et lorsque X et X' sont tous deux -O-, au moins un du cycle A et du cycle A' est choisi parmi 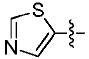 facultativement substitué par 0-2 R^{c} ;
R⁵ est choisi parmi un alkyle en C₁-C₃ ;
R^{5'} est choisi parmi 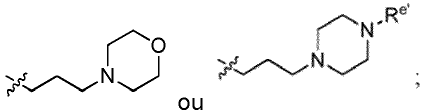
R^{e'} est choisi parmi un alkyle en C₁-C₃, un cycloalkyle à 3-6 éléments, -C(O)R^{f} ;
R^{f} est choisi parmi -OR^{a'}, un alkyle en C₁-C₃, un cycloalkyle à 3 à 4 éléments, et R^{a'} est choisi parmi un alkyle en C₁-C₃ ; et
dans lequel
le 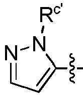 facultativement substitué par 0-2 R^{c} est 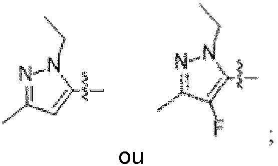
et le 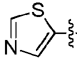 facultativement substitué par 0-2 R^{c} est

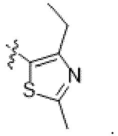

2. Composé, ou stéréoisomère de celui-ci, ou sel pharmaceutiquement acceptable de celui-ci, dans lequel le composé est :

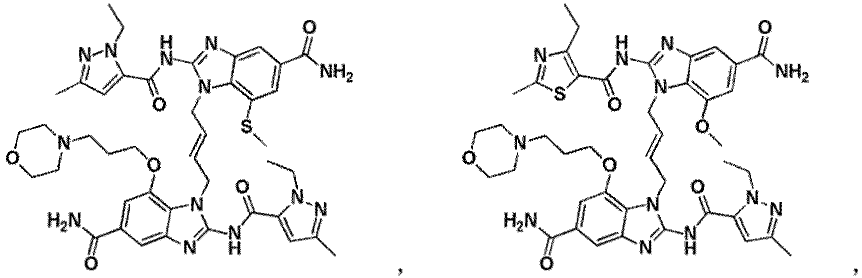

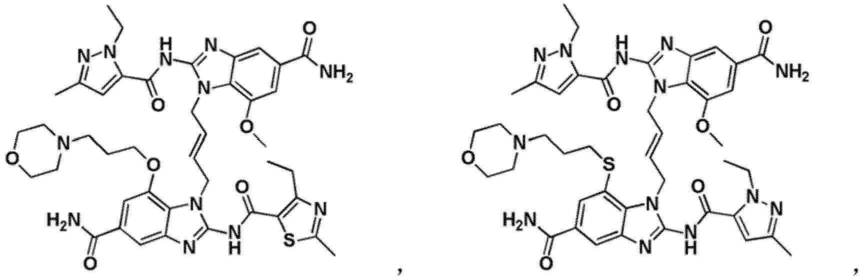

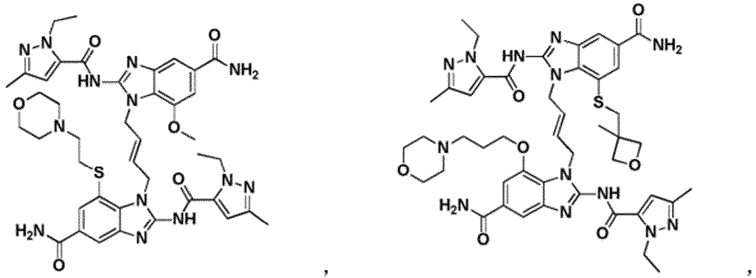

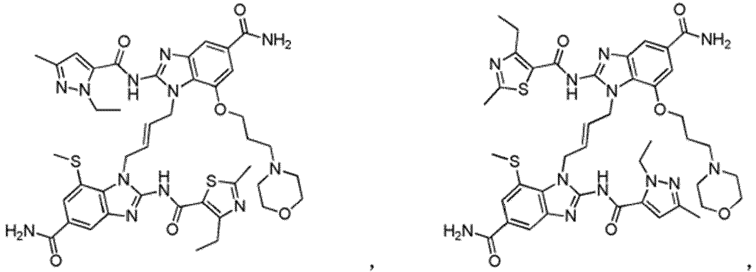

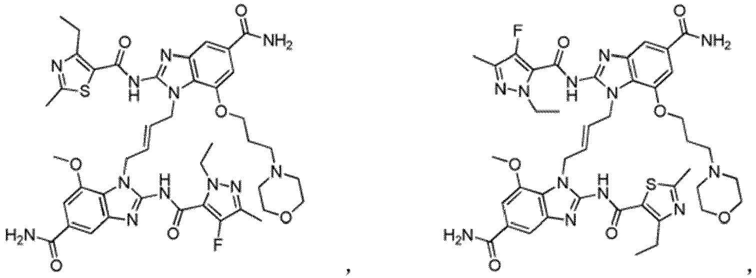

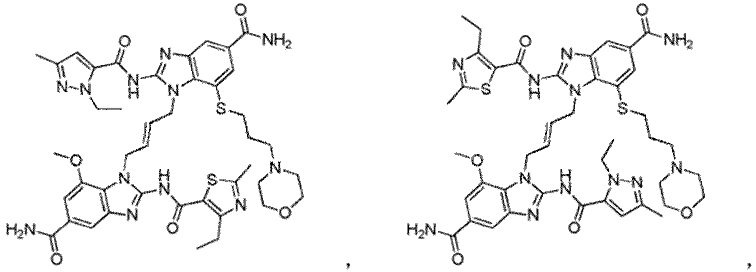

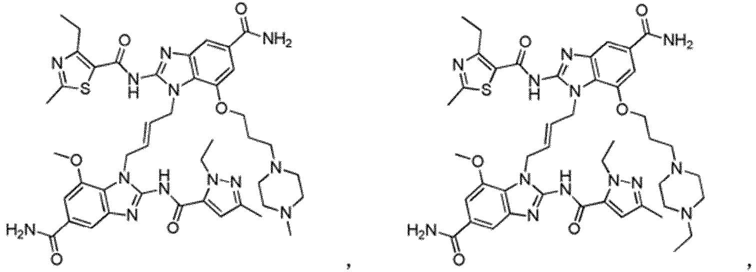

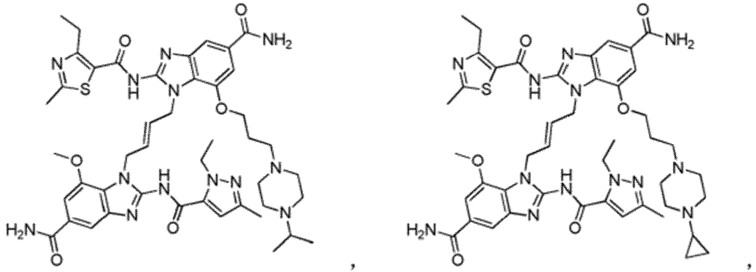

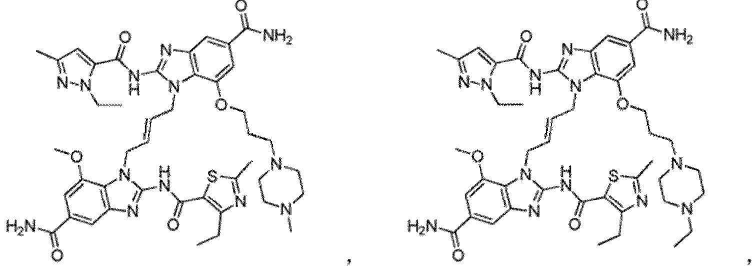

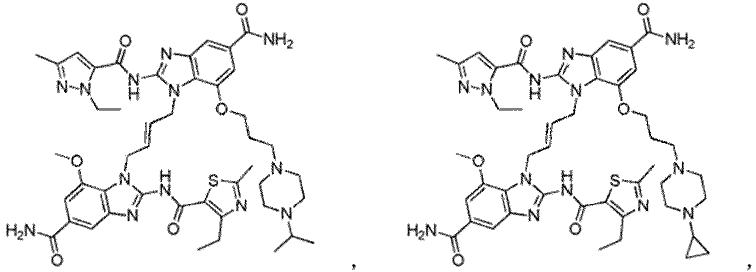

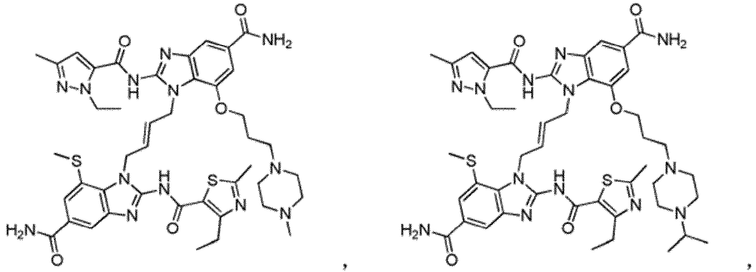

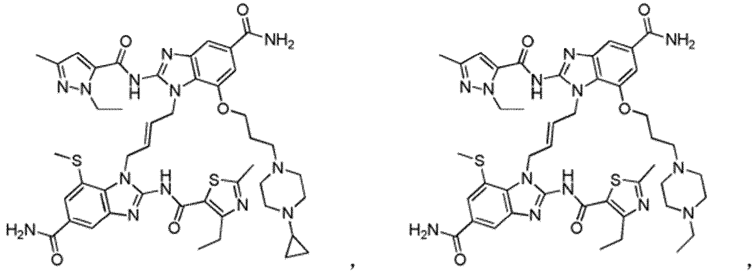

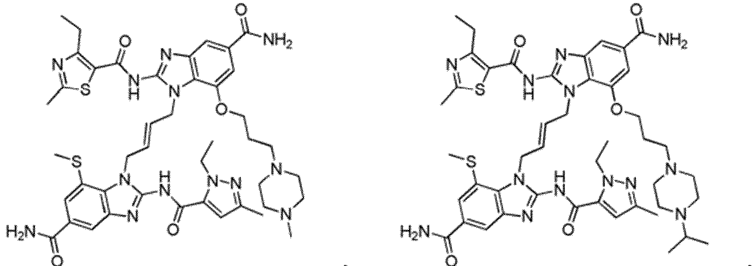

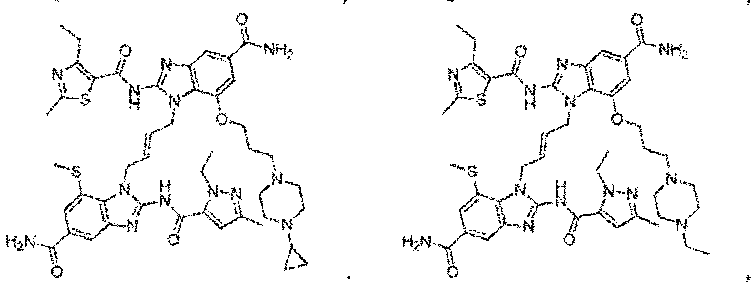

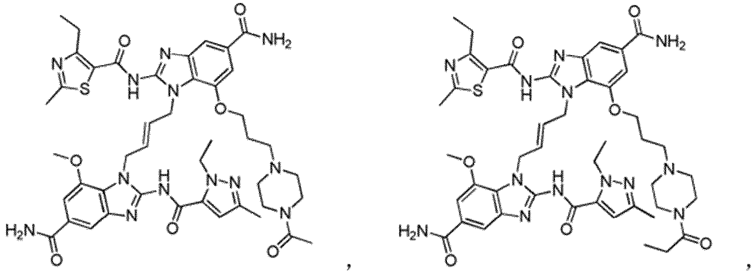

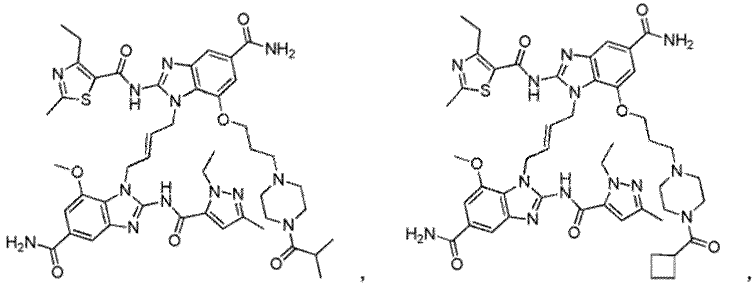

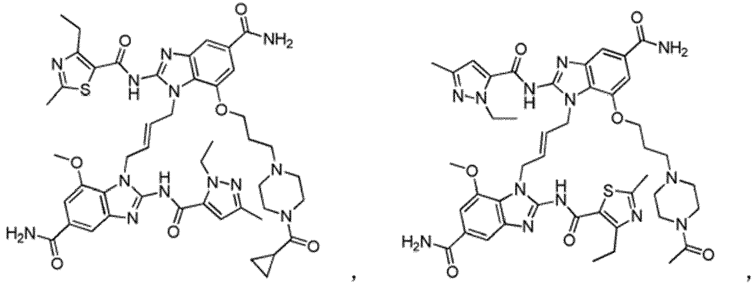

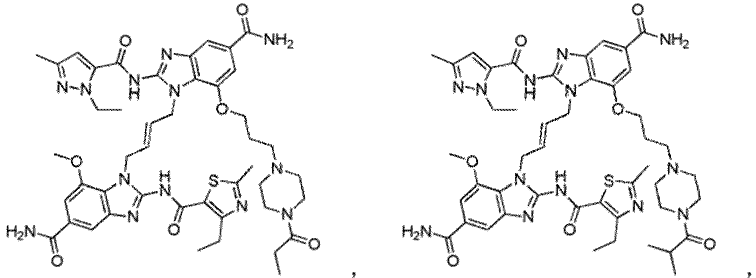

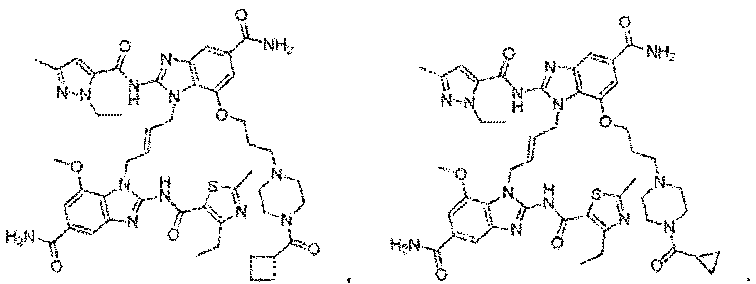

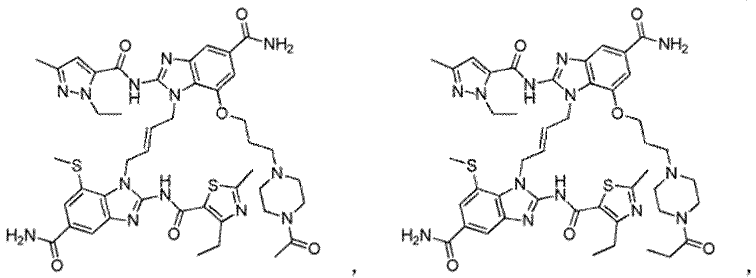

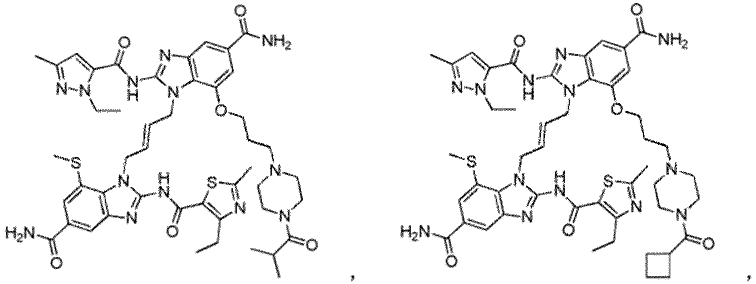

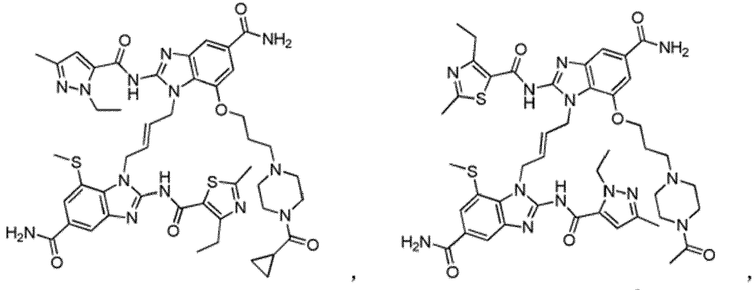

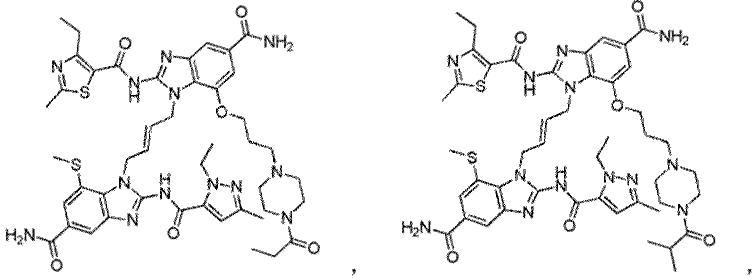

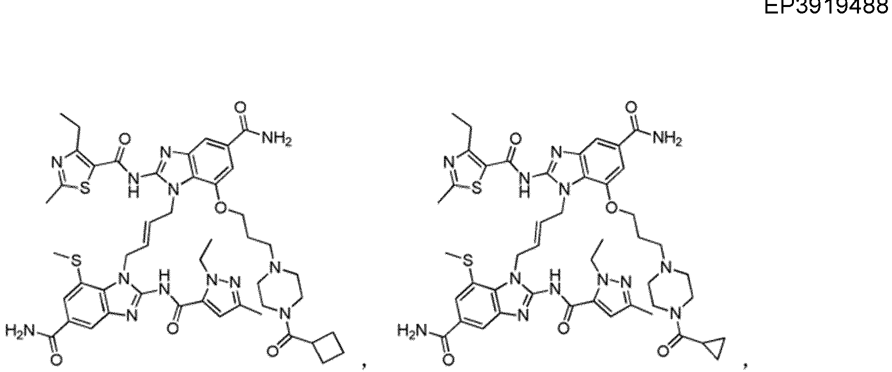

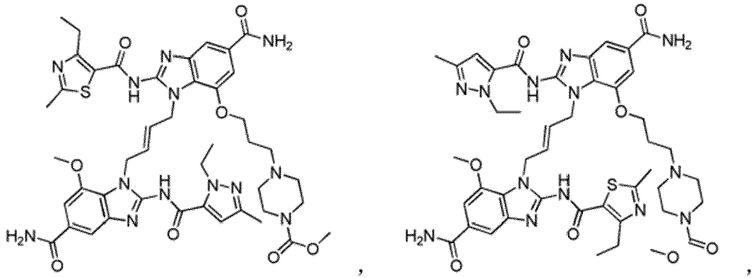

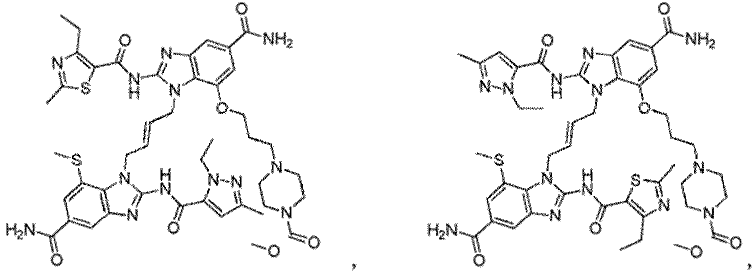

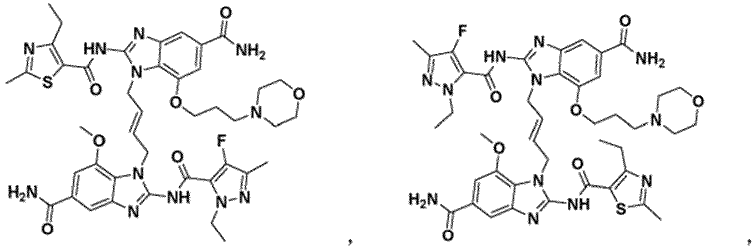

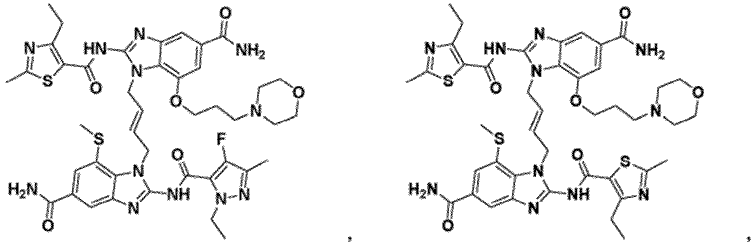

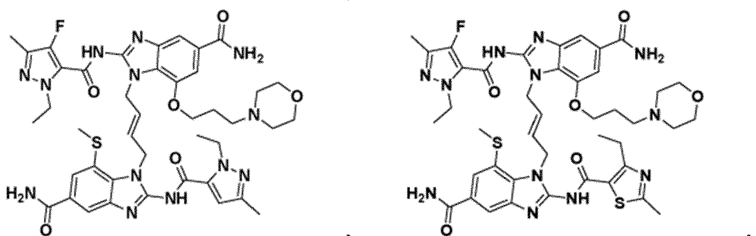

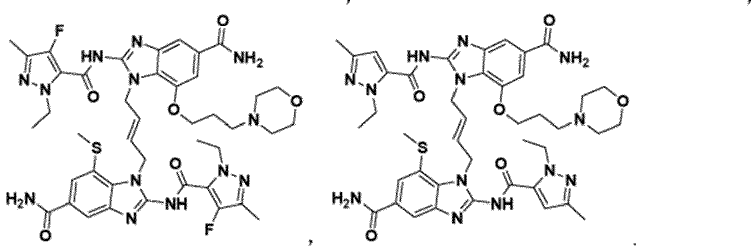

3. Composé, ou stéréoisomère de celui-ci, ou sel pharmaceutiquement acceptable de celui-ci, dans lequel le composé est représenté par la Formule III : 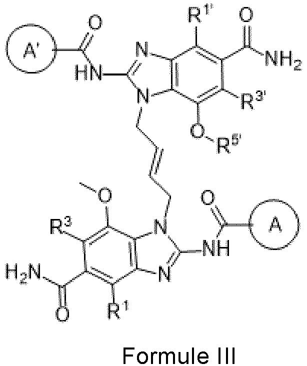 dans lequel R¹, R³, R^{1'}, et R^{3'} sont de l'hydrogène ;
dans lequel le cycle A et le cycle A' sont indépendamment choisis parmi 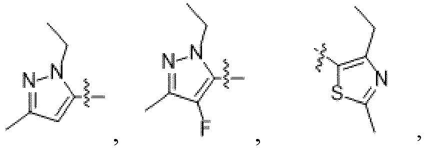 respectivement, et au moins un du cycle A et du cycle A' est 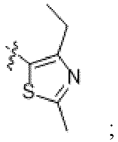
R^{5'} est choisi parmi un alkyle en C₁-C₃ facultativement substitué par 0-2 R^{d} ;
Rd est indépendamment choisi parmi

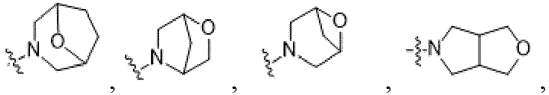

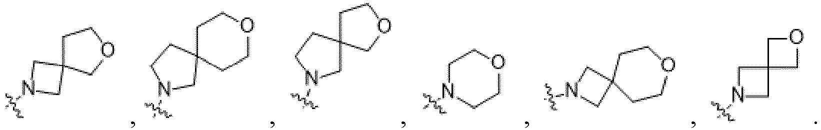

4. Composé, ou stéréoisomère de celui-ci, ou sel pharmaceutiquement acceptable de celui-ci, dans lequel le composé est :

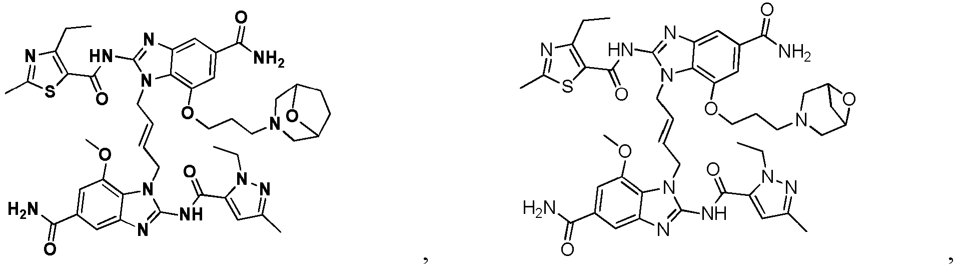

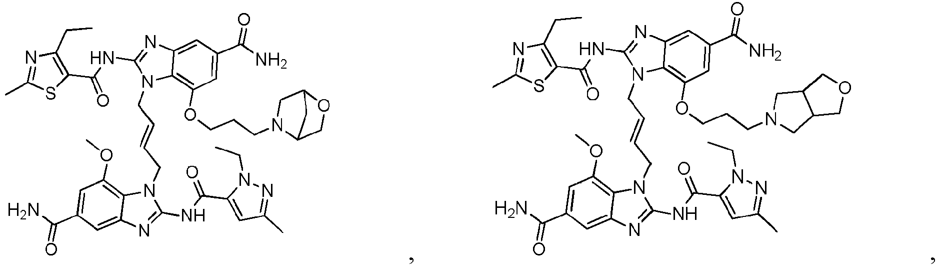

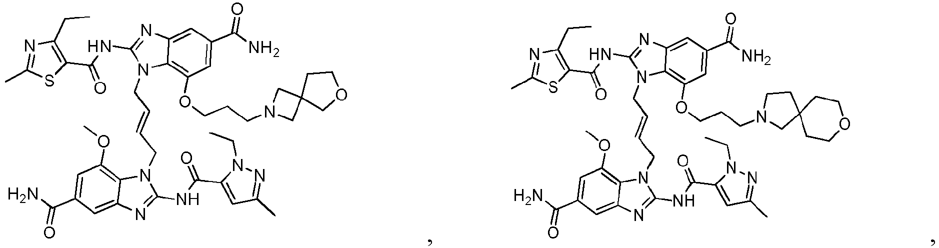

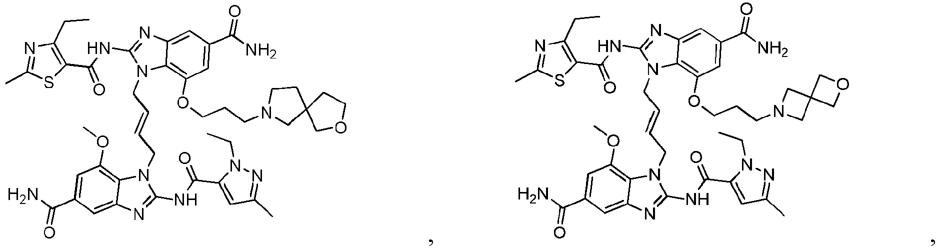

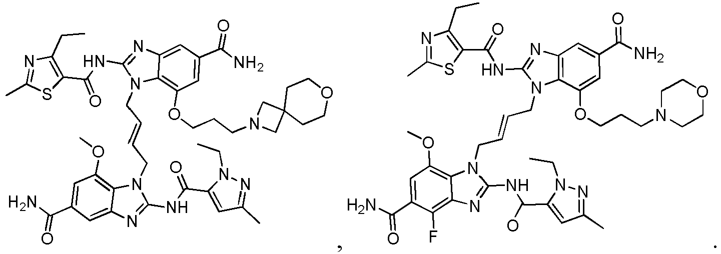

5. Composé, ou stéréoisomère de celui-ci, ou sel pharmaceutiquement acceptable de celui-ci selon l'une quelconque des revendications 1 à 4 destiné à être utilisé dans le traitement d'une maladie liée à l'activité de STING ;
dans lequel la maladie liée à l'activité de STING est l'une quelconque des maladies liées aux maladies inflammatoires, aux maladies auto-immunes, aux maladies infectieuses, au cancer et au syndrome précancéreux ou une combinaison de celles-ci.

6. Composé, ou stéréoisomère de celui-ci, ou sel pharmaceutiquement acceptable de celui-ci selon l'une quelconque des revendications 1 à 4 destiné à être utilisé en tant qu'adjuvant immunitaire.

7. Médicament, qui est une formulation préparée avec le composé, ou le stéréoisomère de celui-ci, ou le sel pharmaceutiquement acceptable de celui-ci selon l'une quelconque des revendications 1 à 4 en tant que principe actif, plus des excipients pharmaceutiquement acceptables.
